# EUROPEAN PATENT SPECIFICATION

(11) **EP 2 948 565 B1**
(45) Date of publication and mention of the grant of the patent: **10.10.2018**
(21) Application number: 14743573.9
(22) Date of filing: 24.01.2014
(51) Int. Cl.: C12Q 1/68, G06F 19/22

(54) **AN ASSAY FOR QUANTITATING THE EXTENT OF METHYLATION OF A TARGET SITE**
ASSAY ZUR QUANTIFIZIERUNG DES AUSMASSES DER METHYLIERUNG EINES ZIELORTES
DOSAGE POUR QUANTIFIER LE DEGRÉ DE MÉTHYLATION D'UN SITE CIBLE

(30) Priority: 25.01.2013 AU 2013900227
(43) Date of publication of application: 02.12.2015
(73) Proprietor: Murdoch Childrens Research Institute, Parkville, VIC 3052 (AU)
(72) Inventor: GODLER, David, Hughesdale 3166 (AU); INABA, Yoshimi, Parkville Victoria 3052 (AU)
(74) Representative: Schnappauf, Georg
(86) International application number: PCT/AU2014/000044
(87) International publication number: WO 2014/113843

(56) References cited:
- WO-A1-2010/094061
- WO-A1-2012/019235
- WO-A2-02/34942
- CN-A- 102 021 233
- US-A1- 2013 017 544
- T. K. WOJDACZ ET AL: "Methylation-sensitive high resolution melting (MS-HRM): a new approach for sensitive and high-throughput assessment of methylation", NUCLEIC ACIDS RESEARCH, vol. 35, no. 6, 1 March 2007 (2007-03-01), pages e41-e41, XP055083325, ISSN: 0305-1048, DOI: 10.1093/nar/gkm013
- CHIA-CHENG HUNG ET AL: "Quantitative and Qualitative Analyses of the SNRPN Gene Using Real-Time PCR with Melting Curve Analysis", THE JOURNAL OF MOLECULAR DIAGNOSTICS, vol. 13, no. 6, 1 November 2011 (2011-11-01), pages 609-613, XP055189180, ISSN: 1525-1578, DOI: 10.1016/j.jmoldx.2011.06.005
- C. DAHL ET AL: "A Homogeneous Assay for Analysis of FMR1 Promoter Methylation in Patients with Fragile X Syndrome", CLINICAL CHEMISTRY, vol. 53, no. 4, 1 April 2007 (2007-04-01), pages 790-793, XP055029491, ISSN: 0009-9147, DOI: 10.1373/clinchem.2006.080762
- TOBLER, AR ET AL.: 'Methylation Analysis Using Methylation-Sensitive HRM and DNA Sequencing' APPLIED BIOSYSLEMS APPLICATION NOTE 2010, XP055289628 Retrieved from the Internet: <URL:https://www3.appliedbiosystems.com/cms /groups/mcb_marketing/documents/generaldocu ments/cms_070933.pdf> [retrieved on 2014-04-07]
- WOJDACZ, TK ET AL.: 'Methylation-sensitive high resolution melting (MS-FIR.VI): a new approach for sensitive and high-throughput assessment of methylation' NUCLEIC ACIDS RESEARCH vol. 35, no. 6, 2007, page E41, XP055083325 Retrieved from the Internet: <URL:http://nar.oxfordjournals.org/content/ 35/6/041.full.pdf+html> [retrieved on 2014-02-27]
- WOJDACZ, TK ET AL.: 'Rapid Detection of Methytation Change at H19 in Human Imprinting Disorders Using Methylation-Sensitive High-Resofution Molting' HUMAN MUTATION vol. 29, no. 10, 2008, pages 1255 - 1260, XP055289624
- WOJDACZ, TK ET AL.: 'Methytation-sensitive high-resolution melting' NATURE PROTOCOLS vol. 3, no. 12, 2008, pages 1903 - 1908, XP055083325
- MALENTACCHL, F ET AL.: 'Quantitative evaluation of DNA methylation by optimization of a differential-high resolution melt analysis protocol' NUCLEIC ACIDS RESEARCH vol. 37, no. 12, 2009, page E86, XP055289626 Retrieved from the Internet: <URL:http://nar.oxfordjournals.org/contents /37/12/c86.full.pdf+html> [retrieved on 2014-02-27]
- GODLER, DE ET AL.: 'Mcthylation of novel markers of fragile X alleles is inversely correlated with FMRP expression and FMRI activation ratio' HUMAN MOLECULAR GENETICS vol. 19, no. 8, 2010, pages 1618 - 1632, XP055029010
- ZESCHN1GK, M ET AL.: 'A single-tube PCR test for the diagnosis of Angelman snd Prdcler-Willi syndrome based on allelic methylation differences at the SNRPN locus' EUROPEAN JOURNAL OF HUMAN GENETICS vol. 5, no. 2, 1997, pages 94 - 98, XP009011533

### SEQUENCE LISTING

<110> Murdoch Childrens Research Institute
<120> An Assay
<130> 35212037/EJH
<150> AU 2013900227
   <151> 2013-01-25
<160> 20
<170> PatentIn version 3.5
<210> 1
   <211> 242
   <212> DNA
   <213> artificial
<220>
   <223> Nucleotide sequence of FREE2 (A)
<400> 1 ct 242
<210> 2
   <211> 454
   <212> DNA
   <213> artificial
<220>
   <223> Nucleotide sequence of FREE2 (B)
<400> 2
   acttggggcc tgttggaagc ccctctccga ctccgagagg ccctagcgcc tatcgaaatg      60
   agagaccagc gaggagaggg ttctctttcg gcgccgagcc ccgccggggt gagctgggga     120
   tgggcgaggg ccggcggcag gtactagagc cgggcgggaa gggccgaaat cggcgctaag     180
   tgacggcgat ggcttattcc ccctttccta aacatcatct cccagcggga tccgggcctg     240
   tcgtgtgggt agttgtggag gagcgggggg cgcttcagcc gggccgcctc ctgcagcgcc     300
   aagagggctt caggtctcct ttggcttctc ttttccggtc tagcattggg acttcggaga     360
   gctccactgt tctgggcgag ggctgtgaag aaagagtagt aagaagcggt agtcggcacc     420
   aaatcacaat ggcaactgat ttttagtggc ttct                                 454
<210> 3
   <211> 302
   <212> DNA
   <213> artificial
<220>
   <223> Nucleotide sequence of FREE2 (C)
<400> 3
   caagagggct tcaggtctcc tttggcttct cttttccggt ctagcattgg gacttcggag      60
   agctccactg ttctgggcga gggctgtgaa gaaagagtag taagaagcgg tagtcggcac     120
   caaatcacaa tggcaactga tttttagtgg cttctctttg tggatttcgg aggagatttt     180
   agatccaaaa gtttcaggaa gaccctaaca tggcccagca gtgcattgaa gaagttgatc     240
   atcgtgaata ttcgcgtccc cctttttgtt aaacggggta aattcaggaa tgcacatgct     300
   tc                                                                    302
<210> 4
   <211> 373
   <212> DNA
   <213> artificial
<220>
   <223> Nucleotide sequence of FREE2 (D)
<400> 4
   aaaagtttca ggaagaccta acatggccca gcagtgcatt gaagaagttg atcatcgtga    60
   atattcgcgt cccccttttt gttaaacggg gtaaattcag gaatgcacat gcttcagcgt   120
   ctaaaaccat tagcagcgct gctacttaaa aattgtgtgt gtgtgtttaa gtttccaaag   180
   acctaaatat atgccatgaa acttcaggta attaactgag agtatattat tactagggca   240
   tttttttttt aactgagcga aaatattttt gtgcccctaa gaacttgacc acatttcctt   300
   tgaatttgtg gtgttgcagt ggactgaatt gttgaggctt tatataggca ttcatgggtt   360
   tactgtgctt ttt                                                      373
<210> 5
   <211> 360
   <212> DNA
   <213> artificial
<220>
   <223> Nucleotide sequence of FREE2 (E)
<400> 5
   gaatggcttg aatgtttcag acaggacacg tttggctata ggaaaataaa caattgactt    60
   tattctgtgt ttaccaattt tatgaagaca tttggagatc agtatatttc ataaatgagt   120
   aaagtatgta aactgttcca tactttgagc acaaagataa agccttttgc tgtaaaagga   180
   ggcaaaaggt aaccccgcgt ttatgttctt aacagtctca tgaatatgaa attgtttcag   240
   ttgactctgc agtcaaaatt ttaatttcat tgattttatt gatccataat ttcttctggt   300
   gagtttgcgt agaatcgttc acggtcctag attagtggtt ttggtcacta gatttctggc   360
<210> 6
   <211> 320
   <212> DNA
   <213> artificial
<220>
   <223> Nucleotide sequence of FREE3
<400> 6
   tttttcttac acaggcattt gtaaaggatg ttcatgaaga ttcaataaca gttgcatttg    60
   aaaacaagta agtgtctcgt tatataattt taatgatgag gttctttaat attttatgct   120
   aattctattc ttcatttttt aaaaattcaa gtccagtttg agtgcttttc aggaatggat   180
   cttcatgtta ctgactgaga agtttctgaa caactcagta ttaaactaat ggaatgactg   240
   tttctgctaa tgtcctggag gtcccttatt gtatggtatt gatccttacg tcttaattcc   300
   cttgaatgtg aagaaagaaa                                               320
<210> 7
   <211> 27
   <212> DNA
   <213> artificial
<220>
   <223> Nucleotide sequence of forward FREE2 (A) primer for MS-QMA
<400> 7
   aaggtatttg gttttagggt aggtttt 27
<210> 8
   <211> 21
   <212> DNA
   <213> artificial
<220>
   <223> Nucleotide sequence of reverse FREE2 (A) primer for MS-QMA
<400> 8
   aaatccaatc cttccctccc a 21
<210> 9
   <211> 35
   <212> DNA
   <213> artificial
<220>
   <223> Nucleotide sequence of forward FREE2 (B) primer for MS-QMA
<400> 9
   aggaagagag ggtttttttg aaatttttgg attta 35
<210> 10
   <211> 56
   <212> DNA
   <213> artificial
<220>
   <223> Nucleotide sequence of reverse FREE2 (B) primer for MS-QMA
<400> 10
   cagtaatacg actcactata gggagaaggc ttaaaaccta ttaaaaaccc ctctcc 56
<210> 11
   <211> 35
   <212> DNA
   <213> artificial
<220>
   <223> Nucleotide sequence of forward FREE2 (C) primer for MS-QMA
<400> 11
   aggaagagag taagagggtt ttaggttttt tttgg 35
<210> 12
   <211> 59
   <212> DNA
   <213> artificial
<220>
   <223> Nucleotide sequence of reverse FREE2 (C) primer for MS-QMA
<400> 12
   cagtaatacg actcactata gggagaaggc taaaacatat acattcctaa atttacccc 59
<210> 13
   <211> 40
   <212> DNA
   <213> artificial
<220>
   <223> Nucleotide sequence of forward FREE3 primer for MS-QMA
<400> 13
   aggaagagag ttttttttat ataggtattt gtaaaggatg 40
<210> 14
   <211> 59
   <212> DNA
   <213> artificial
<220>
   <223> Nucleotide sequence of reverse FREE3 primer for MS-QMA
<400> 14
   cagtaatacg actcactata gggagaaggc ttctctaatt tctttcttca cattcaaaa 59
<210> 15
   <211> 23
   <212> DNA
   <213> artificial
<220>
   <223> Nucleotide sequence of SNRPN-M forward primer for PWS/AS PCR
<400> 15
   taaataagta cgtttgcgcg gtc 23
<210> 16
   <211> 21
   <212> DNA
   <213> artificial
<220>
   <223> Nucleotide sequence of SNRPN-M reverse primer for PWS/AS PCR
<400> 16
   aaccttaccc gctccatcgc g 21
<210> 17
   <211> 24
   <212> DNA
   <213> artificial
<220>
   <223> Nucleotide sequence of SNRPN-P forward primer for PWS/AS PCR
<400> 17
   gtaggttggt gtgtatgttt aggt 24
<210> 18
   <211> 23
   <212> DNA
   <213> artificial
<220>
   <223> Nucleotide sequence of SNRPN-P reverse primer for PWS/AS PCR
<400> 18
   acatcaaaca tctccaacaa cca 23
<210> 19
   <211> 174
   <212> DNA
   <213> artificial
<220>
   <223> Nucleotide sequence of SNRPN-M target sequence to detect AS
<400> 19
   caaacaagca cgcctgcgcg gccgcagagg caggctggcg cgcatgctca ggcggggatg       60
   tgtgcgaagc ctgccgctgc tgcagcgagt ctggcgcaga gtggagcggc cgccggagat      120
   gcctgacgca tctgtctgag gagcggtcag tgacgcgatg gagcgggcaa ggtc            174
<210> 20
   <211> 100
   <212> DNA
   <213> artificial
<220>
   <223> Nucleotide sequence of SNRPN-P target sequence to detect PWS
<400> 20
   gcaggctggc gcgcatgctc aggcggggat gtgtgcgaag cctgccgctg ctgcagcgag       60
   tctggcgcag agtggagcgg ccgccggaga tgcctgacgc                            100

## Description

### BACKGROUND

### FIELD

The present specification relates to an assay to quantitate extent of methylation in a DNA sample. Kits and clinical diagnostic assays are also disclosed herein including assays to determine clinical phenotypes based on extent of methylation of a DNA target site.

### DESCRIPTION OF RELATED ART

The fragile X mental retardation genetic locus ("FMR genetic locus") includes the FMR1 gene which is composed of 17 exons, spanning 38Kb, and encodes fragile X mental retardation protein (FMRP), essential for normal neurodevelopment (Verkerk et al. (1991) Cell 65(5):905-914; Terracciano et al. (2005) Am J Med Genet C Semin Med Genet 137C(1):32-37). A CGG repeat segment is located within the 5' untranslated region (UTR) of the gene. Its normal range is <40 repeats. When expanded, these repeats have been implicated in a number of pathologies, including the fragile X syndrome (FXS), fragile X-associated tremor ataxia syndrome (FXTAS) and fragile X-associated primary ovarian insufficiency (FXPOI; formerly referred to as premature ovarian failure [POF]). FXS is neurodevelopmental in nature with a frequency of 1/2000 males and 1/4000 females, associated with a fragile site at the Xq27.3 locus (Jin and Warren (2000) Hum. Mol. Genet 9(6):901-908). Furthermore, up to 60% of FXS males have a more severe form of autism termed autism disorder (AD). FXS is the single most common known genetic cause of intellectual impairment and co-morbid autism.

This syndrome is usually associated with a CGG expansion to "full mutation" (FM) which comprises >200 repeats, leading to a gross deficit of FMRP and subsequent synaptic abnormalities (Pieretti et al. (1991) Cell 66(4):817-822; Irwin et al. (2000) Cereb Cortex 10(10):1038-1044). The FXS clinical phenotype ranges from learning disabilities to severe mental retardation and can be accompanied by a variety of physical and behavioral characteristics. FXTAS is prevalent in ∼30% of premutation individuals (PM), comprising from about 55 to 199 repeats (Nolin et al. (2003) Am J. Hum Genet 72(2):454-464) and is a progressive neurodegenerative late-onset disorder with a frequency of 1:200 to 1:600 in the general population, manifesting as tremor, imbalance and distinct MRI and histological changes (Hagerman et al. (2001) Neurology 57(1):127-130; Jacquemont et al. (2005) J Med Genet 42(2):e14; Loesch et al. (2005) Clin Genet 67(5):412-417). It is often associated with 'toxicity' of elevated FMR1 mRNA, which has been linked to the intranuclear inclusions and cell death observed during neurodegeneration (Jin et al. (2003) Neuron 39(5):739-747*).*

Studies in Australia and the USA indicate that approximately 1 in 100 children have autism spectrum disorder (ASD); ASD is associated with FXS up to 10% of these children. At present, there is no laboratory test available to diagnose ASD. The use of intervention programs at the earliest stages has been shown to result in better outcomes for these children and early diagnosis is a clear unmet need.

It is apparent that DNA methylation and other epigenetic modifications play a role in the regulation of gene expression in higher organisms. The importance of epigenetic modification has been highlighted by its involvement in several human diseases. Methylation, for example, of cytosine at the 5' position is the only known methylation modification of mammalian genomic DNA. In particular, methylation of CpG islands within regulatory regions of the genome appears to be highly tissue specific. It is now apparent that methylation of cytosines distal to the islands is also important. These regions are called "shores" or "island shores" (Irizarry et al, Nature Genetics 41(2):178-186, 2009).

Despite the availability of a range of methylation assays (see, for example, Rein et al. (1998) Nucleic Acids Res. 26:2255), accurate quantitation of the extent of methylation is an important aspect of determining an epigenetic profile characteristic of a disease condition.

The current gold standard assay used for molecular diagnosis of FXS is methylation sensitive southern blot. Whilst this approach is time consuming and low throughput, it provides information on the CGG size up to FM and methylation status of several methylation sensitive restriction sites within the FMR1 CpG island. One major limitation associated with this and other alternative approaches currently used for the molecular diagnosis of FXS is that the test results cannot be used to provide accurate prognostic information in both male and female carriers of the expanded alleles on the type and severity of the disease. Alternative PCR based diagnostic assays targeting only the CGG expansion size have not been prognostically informative.

Another form of an assay is a methylation sensitive PCR (MS-PCR) which has been used for the diagnosis of FXS in males and Prader-Willi syndrome (PWS)/Angelman syndrome (AS) and is based on the difference in methylation status of a few CpG sites of the targeted region. A complicating factor is methylation mosaicism which is rare in PWS (Wey et al. (2005) Eur J Hum Genet 13(3):273-277) but is much more common (approximately 27%) in AS (Buiting et al. (2003) Am J Hum Genet 72(3):571-577) and in FXS (approximately 40%) [Nolin et al. (1994) Am J Med Genet. 51(4):509-512]. The level of mosaicism representing the proportion of "diseased" cells, has also been related to the disease severity in X-linked disorders involving imprinting genes. Since MS-PCR is not highly sensitive or quantitative, a certain proportion of the methylation mosaic individuals, AS, PWS, FXS and modified X-chromosome disorders are not detected or accurate level of mosaicism is not determined using current techniques for the molecular diagnosis of these disorders and thus disease severity cannot be accurately predicted.

High resolution melt curve analysis has been used for PWS and AS (Wang et al. (2009) J. Mol. Diagn. 11(5):446-449; White et al. (2007) Clin Chem 53(11):1960-1962) and FXS (Dahl et al. (2007) Clin Chem. 53(4):790-793). However, this method does not accurately quantitate the extent of methylation.

Coffee et al. (2009) Am. J. Hum. Genet. 85:503-514 proposed a methylation assay of the FMR1 locus to assess the incidence of FXS. The assay employed methylation-sensitive PCR targeting the FMR1 CpG island. Whilst the assay provided some information, the usefulness of assay is limited to males. CpG island methylation data do not correlate with the type or severity of any of the FXS clinical features.

Elias et al. (2011) Genet. Testing and Mol. Biomarkers 15(56):387-393 also proposed a gene methylation assay based on multiplex-specific real-time PCR. However, insofar as it was applied to the FMR1 gene, it was only suitable for molecular diagnosis in males and did not accurately quantitate the extent of methylation.

A poster presented by Hamilton et al. (2012) European Human Genetics Conference, June 23-26, Nümberg, Germany evaluated a melt assay to determine the methylation status of the FMR1 promoter. The assay achieved qualitative results in line with other assays and, in FM females, the assay did not provide a quantitative threshold that could separate affected from non-affected subjects; and the qualitative results obtained using this method were not quantitatively correlated with any parameter of disease type and severity, which is a major limitation for use of these tests in female expansion carriers.

An accurate quantitative assay has been successfully used to determine methylation of a region of the FMR1 genetic locus in males and females (Godler et al. (2010) Hum Mole Genet 19:1618-1632; Godler et al. (2011) J Mol Diag 13:528-536; Godler et al. (2012) Clin Chem 58:590-598; International Patent Application No. PCT/AU2010/00169 [WO 2010/094061]). However, this assay requires the use of MALDI-TOF MS which requires expensive equipment and a level of expertise outside the resources of many diagnostic laboratories. There is a pressing need to develop a low cost, high throughput, quantitative methylation test that does not require specialized equipment and training, and can be used by most diagnostic laboratories with existing technical platforms to provide at least comparable results to the MALDI-TOF MS reference method.

Chinese Patent Application Publication No. 102 021 233 A discloses a method for assessing the methylation status of AKAP12 gene promoter region using a standard curve established by assessing the melt profile of samples ranging from 0 to 100% methylation.

Wojdacz et al. (2007) Nucleic Acids Res 35:e41 disclose that high resolution melting is a sensitive and specific method for the detection of methylation.

International Patent Application Publication No. WO 02/34942 A2 discloses methods for determining the methylation profile of a nucleic acid sequence and for determining one or more base changes in a target nucleic acid sequence as compared to a corresponding control sequence.

U.S. Patent Application Publication No. 2013/0017544 A1 discloses an integrated droplet actuator device and methods for performing PCR amplification and high-resolution melting analysis on a single droplet actuator.

Hung et al. (2011) J Mol Diagn 13:609-613 disclose the utility of combined real-time PCR and melting curve analysis for rapid genotyping of *SNRPN* gene methylation status.

### SUMMARY

The present invention is directed to the subject-matter set forth in the appended claims.

A present disclosure teaches an assay to quantitate the extent of methylation at a target site within a DNA sample. The assay enables high throughput screening of DNA samples. The assay enables the detection of methylation changes which can be used to correlate with clinical phenotypes and, hence, has significant diagnostic and prognostic value. This is useful especially when screening children too young to undergo certain procedures such as, in the case of neurological disorders, neuropsychological testing. An algorithm can be used to interface output data from the assay with percentage of methylation and ultimately with clinical phenotype.

The present disclosure is, therefore, instructional for a method referred to herein as "methylation specific-quantitative melt analysis" (MS-QMA) for quantitating the extent of methylation of a target region comprising one or more CpG sites within a DNA sample. The method comprises subjecting the DNA sample to bisulfite treatment to convert non-methylated cytosine nucleotides in the DNA to uracil nucleotides followed by amplifying and melting a portion of the DNA sample comprising the target region. The amplification uses selected forwarded and reverse primers to amplify and incorporate a label in real-time which is capable of providing an identifiable signal in double stranded DNA as determined by units of signal strength. The melting reaction of the amplified DNA releases the label in real-time. The assay is conducted in the presence of a first control DNA sample set of known DNA concentration and a second control DNA sample set of known percentage methylated and non-methylated DNA. The first control DNA sample set is used to generate a standard curve of DNA concentration *versus* cycle threshold (Ct) to determine the dynamic linear range of the amplification and the stable amplified product melting range where the signal strength is independent of the DNA dilution which indicates that the product has reached the maximum PCR amplification. The second control DNA is used to generate a standard curve of signal strength *versus* percentage of methylation. DNA samples to be tested which fall within the dynamic linear range of amplification and the stable amplified product melting range are used to determine the percentage of methylation based on the standard curve generated by the second control DNA sample set. The melting temperature for the second control DNA sample set is selected at the signal providing a measurable difference between the non-methylated and methylated control samples. In an embodiment, this is the temperature giving a maximum difference between non-methylation and methylated DNA.

The percentage of methylation can, therefore, be determined for a given DNA sample. It will be understood that the terms "percentage of methylation" and "methylation ratio" or "MR" are used herein interchangeably. The percentage methylation is calculated by multiplying the methylation ratio by 100, i.e. MR x 100 = % methylation.

In an embodiment, the DNA sample is serially diluted after bisulfite conversion and if multiple dilutions of the same DNA sample are selected, the mean of the determined percentage methylation is calculated. In an embodiment, the DNA sample is serially diluted four times after bisulfite treatment. Conveniently, the amplification reaction is a real-time polymerase chain reaction (RT-PCT) and the label incorporated into the amplified DNA is an intercalating fluorescent dye which binds double stranded DNA and fluorescences at a signal strength measured in aligned fluorescence units (AFU) and which does not provide a fluorescence signal in single stranded DNA.

Generally, the melt reaction is high resolution melt (HRM) conducted at a defined temperature which, in an embodiment, is the temperature providing the greatest difference between the non-methylated and methylated controls. This can be regarded as the lowest temperature where all double stranded DNA from non-methylated DNA in the second control DNA set is melted.

Conveniently, the DNA sample to be tested is one of a multiplicity of DNA samples and the multiplicity of DNA samples and the first and second control samples are located in a multi-compartmental container, such as a microtiter tray. The DNA sample may be derived from any source and includes cells contained in body fluid or a dried body fluid sample from a human subject. In an embodiment, the DNA sample is derived from cells in venous blood or a dried blood spot. In another embodiment, the DNA sample is derived from cells from sputum or other respiratory fluid, blood, saliva tissue fluid, a tissue sample, lymph fluid, semen, urine, fecal material or skin material. Reference to "blood" includes whole blood or a fraction thereof, a dried blood spot, and venous or arterial blood.

Taught herein is the correlation between extent of methylation of an artificially treated target site and a clinical phenotype including presence or absence of an adverse clinical condition, the stage of progression of the clinical condition, the severity of the clinical condition and the amelioration of the clinical condition after or during treatment. By "artificially treated" includes bisulfite treatment to convert non-methylated cytosines to uracils. The clinical phenotype further extends to rejection of transplanted tissue in a subject and monitoring for fetal cells in a maternal subject.

The DNA target site may be located in a genetic locus associated with neurological development, cognitive impairment, tumor suppression, tumor growth, sex determination, aneuploidy, immune response progression, trinucleotide disorders, imprinting disorders, X-linked disorders, modified X-chromosome disorders and X-chromosome inactivation skewing related disorders. In an embodiment, the assay is capable of quantitating skewed X-chromosome inactivation. The DNA target site may also be selected to monitor transplant tissues and organs and in prenatal diagnosis. The DNA target site may also be used to monitor treatment.

Included herein is a DNA target site selected from a site defined by an intron, exon, intron-exon boundary, promoter region or a region 5' of the promoter region, a CpG island or group of CpG islands and a site downstream of the 3' end region of a genetic locus, differentially methylated loci and CpG island shores. In an embodiment, the DNA target site is within the FMR1 genetic locus.

In an embodiment, the target DNA is fragile X-related epigenetic element 2 (FREE2). Reference to "FREE2" includes FREE2 (A) [SEQ ID NO:1], FREE2 (B) [SEQ ID NO:2], FREE2 (C) [SEQ ID NO:3], FREE2 (D) [SEQ ID NO:4] and FREE2 (E) [SEQ ID NO:5]. In another embodiment the target DNA is fragile X-related epigenetic element 3 (FREE3) [SEQ ID NO:6]. In any embodiment, the defined temperature for providing the greatest difference between methylated and non-methylated DNA is 78°C ± 10°C.

In relation to the targeting FREE2 (A), forward and reverse primers are those defined by SEQ ID NOs:7 and 8, respectively. FREE2 (B) can be targeted by forward and reverse primers defined by SEQ ID NOs:9 and 10, respectively. FREE2 (C) can be targeted by forward and reverse primers defined by SEQ ID NOs:11 and 12, respectively. FREE3 can be targeted by forward and reverse primers defined by SEQ ID NOs:13 and 14, respectively.

In another embodiment, the DNA target site is the SNRPN gene promoter. Reference to "SNRPN" includes SNRPN-M [SEQ ID NO:19] and SNRPN-P [SEQ ID NO:20]. In one embodiment, the defined temperature for providing the greatest difference between methylated and non-methylated DNA is 80.08°C + 10°C.

In relation to the targeting SNRPN-M, forward and reverse primers are those defined by SEQ ID NOs:15 and 16, respectively. SNRPN-P can be targeted by forward and reverse primers defined by SEQ ID NOs:17 and 18, respectively.

The assay enabled herein includes using the DNA target site to distinguish one cell type from another cell type based on extent of methylation in a target site in the genome of one cell type which differs from another cell type. Furthermore, the assay can be used to identify an epigenetic-based disorder in a subject such as a developmental or neurological disorder, Prader-Willi syndrome/Angelman Syndrome, Alzheimer's disease, autism, bipolar disorder, diabetes, male sexual orientation, obesity, schizophrenia and a cancer selected from bladder, breast, cervical, colorectal, esophageal, hepatocellular, lung, mesothelioma, ovarian, prostate and testicular cancer and leukemia.

Conditions contemplated herein include pathoneurological conditions such as pathoneurodevelopmental and pathoneurodegenerative conditions as well as non-neurological conditions. Conditions and disorders contemplated herein include polyglutamine (polyQ) diseases such as Huntington's disease (HD), dentatorubropallidoluysiantrophy (DRPLA), spinobulbar muscular atrophy or Kennedy disease (SBMA), spinocerebella ataxia Type 1 (SCA1), spinocerebella ataxia Type 2 (SCA2), spinocerebella ataxia Type 3 or Machado-Joseph disease (SCA3), spinocerebella ataxia Type 6 (SCA6), spinocerebella ataxia Type 7 (SCA7), spinocerebella ataxia Type 17 (SCA17) and non-polyQ diseases such as Fragile X syndrome (FXS), Fragile X-associated tremor or ataxia (FXTAS), Fragile XE mental retardation (FRAXE), myotonic dystrophy (DM), spinocerebella ataxia (SCA8) and spinocerebella ataxias Type 12 (SCA12). Other conditions contemplated herein include trinucleotide expansion related disorders including but not limited to Fragile X-associated primary ovary insufficiency (FXPOI), Friedrich's ataxia (FRDA). Fragile type, folic acid type, rare 12 (FRA12A), autism (including co-morbid autism), mental retardation (MR), Klinefelter's syndrome, RNA toxicity disease, Turner's syndrome, a modified X-chromosome and cognitive impairment are also contemplated herein. Further contemplated herein are learning and behavioral problems. Other conditions include skewed X-chromosome inactivation disorders.

The assay is also useful for diagnosing the epigenetic cause of cognitive impairment and autism-related disorders (including ASD and AD), especially in young children.

The assay is quantitative and, hence, the extent of methylation can be correlated to a disease condition or clinical phenotype, its stage or level of progression or severity and the effectiveness or otherwise of treatment. The instant disclosure further enables an algorithm to transform raw output data into a percentage of methylation or methylation ratio as well as the probability that a particular subject has or does not have a clinical condition. It will be understood that the terms "percentage of methylation" and "methylation ratio" or "MR" are used herein interchangeably. The percentage methylation is calculated by multiplying the methylation ratio by 100, i.e. MR x 100 = % methylation.

In an embodiment, contemplated herein is the use of an algorithm to resolve data from a real-time standard curve and a melt standard curve in the manufacture of a diagnostic assay to quantitate the extent of methylation at a target site within a DNA sample. In an embodiment, an algorithm is used to determine DNA concentration and quality post conversion of DNA from the DNA samples to be tested from the real-time amplification standard curve. In a further embodiment, the algorithm assists in correlating the extent of methylation with a clinical phenotype.

Kits for conducting the assay are also enabled by the instant disclosure.

Nucleotide sequences are referred to by a sequence identifier number (SEQ ID NO). The SEQ ID NOs correspond numerically to the sequence identifiers <400>1 (SEQ ID NO:1), <400>2 (SEQ ID NO:2), etc. A summary of the sequence identifiers is provided in Table 1. A sequence listing is provided after the claims.

**TABLE 1**

| ***Summary of sequence identifiers*** | |
|---|---|
| **SEQUENCE ID NO:** | **DESCRIPTION** |
| 1 | Nucleotide sequence of FREE2 (A) |
| 2 | Nucleotide sequence of FREE2 (B) |
| 3 | Nucleotide sequence of FREE2 (C) |
| 4 | Nucleotide sequence of FREE2 (D) |
| 5 | Nucleotide sequence of FREE2 (E) |
| 6 | Nucleotide sequence of FREE3 |
| 7 | Nucleotide sequence of forward FREE2 (A) primer for MS-QMA |
| 8 | Nucleotide sequence of reverse FREE2 (A) primer for MS-QMA |
| 9 | Nucleotide sequence of forward FREE2 (B) primer for MS-QMA |
| 10 | Nucleotide sequence of reverse FREE2 (B) primer for MS-QMA |
| 11 | Nucleotide sequence of forward FREE2 (C) primer for MS-QMA |
| 12 | Nucleotide sequence of reverse FREE2 (C) primer for MS-QMA |
| 13 | Nucleotide sequence of forward FREE3 primer for MS-QMA |
| 14 | Nucleotide sequence of reverse FREE3 primer for MS-QMA |
| 15 | Nucleotide sequence of SNRPN-M forward primer for PWS/AS PCR |
| 16 | Nucleotide sequence of SNRPN-M reverse for PWS/AS PCR |
| 17 | Nucleotide sequence of SNRPN-P forward primer for PWS/AS PCR |
| 18 | Nucleotide sequence of SNRPN-P reverse primer for PWS/AS PCR |
| 19 | Nucleotide sequence of SNRPN-M target sequence to detect AS |
| 20 | Nucleotide sequence of SNRPN-P target sequence to detect PWS |

Abbreviations used herein are defined in Table 2.

**TABLE 2**

| ***Abbreviations*** | |
|---|---|
| **Abbreviation** | **Definition** |
| AD | Autism disorder |
| AFU | Aligned fluorescence unit |
| AS | Angelman syndrome |
| ASD | Autism spectrum disorder |
| FM | Full mutation |
| FREE | Fragile X epigenetic element (e.g. FREE2, FREE3) |
| FREE2 | FREE2 (A), FREE2 (B), FREE2 (C), FREE2 (D) and FREE2 (E) |
| FSIQ | Full scale IQ |
| FXPOI | Fragile X-associated primary ovary insufficiency |
| FXS | Fragile X syndrome |
| FXTAS | Fragile X-associated tremor ataxia syndrome |
| GZ | Grey zone |
| HRM | High resolution melt |
| MR | Methylation ratio |
| MS-QMA | Methylation specific-quantitative methylation assay |
| MS-QMA MR | Methylation specific-quantitative methylation assay methylation ratio |
| PCR | Polymerase chain reaction |
| PIQ | Performance IQ |
| PM | Permutation |
| POI | Performance organization index |
| PWS | Prader-Willi syndrome |
| RT-PCT | Real time-polymerase chain reaction |
| SCA | X-chromosome aneuploidy |
| UFM | Unmethylated FM |
| VIQ | Verbal IQ |

### BRIEF DESCRIPTION OF THE FIGURES

Some figures contain color representations or entities. Color photographs are available from the Patentee upon request or from an appropriate Patent Office. A fee may be imposed if obtained from a Patent Office.

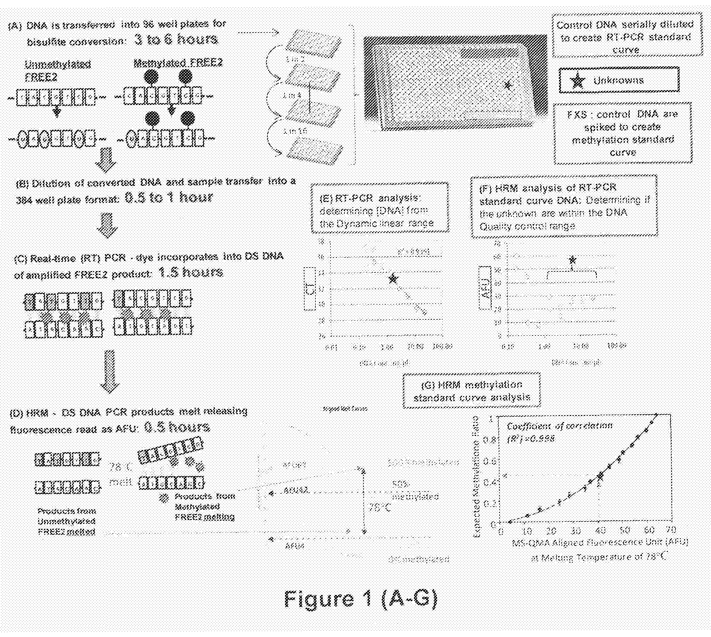

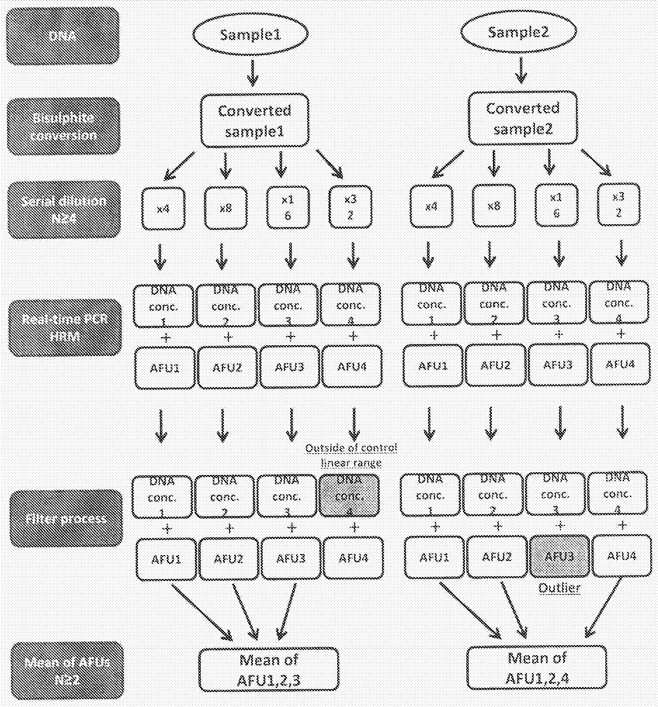

**Figure 1** is a schematic representation of an overview of Methylation Specific Quantitative Melt Analysis (MS-QMA) of the Fragile X Related Epigenetic Element 2 (FREE2). The protocol is based on (A) a single bisulfite converted DNA plate (1 conversion per sample) being **(B)** serially diluted 4 times, with these dilutions analyzed using **(C)** real-time PCR standard curve method and **(D)** High Resolution Melt (HRM) analysis using FREE2 specific primers. This system utilizes a custom designed computer algorithm to simultaneously perform steps **(E), (F)** and **(G)** that determines DNA concentration and quality post conversion for all dilutions from the unknown samples from real-time PCR standard curve (pink diamonds). The unknown sample dilutions (blue star in the pink box) within the concentration and quality control ranges (depicted by pink diamonds in the pink box), are automatically plotted against the HRM methylation standard curve (green box). The sample dilutions outside the quality control range (depicted by yellow diamonds in the pink box) are not utilized in HRM analysis, and are discarded. **(H)** The HRM methylation standard curve is also automatically obtained from 100% methylated and completely unmethylated control samples spiked at different ratios. The AFU for the methylation standard curve and unknowns is analyzed at the melting temperature where there is the greatest difference in fluorescence between 100% methylated and completely unmethylated controls which corresponds to the lowest temperature point where all double stranded DNA (DS DNA) from the unmethylated strands has completely melted. AFU for the unknowns is then converted to methylation ratio (MR) from the HRM standard curve (in the green box). The percentage of methylation can be obtained by multiplying the MR by 100.

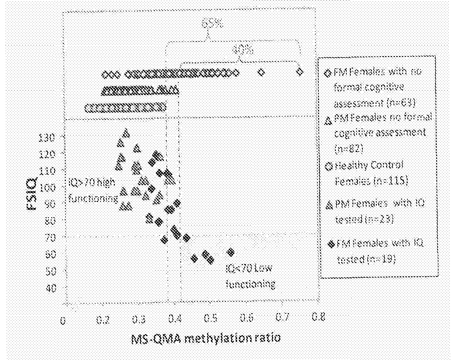

**Figure 2(i)** is a graphical representation showing the relationship between Full Scale IQ (FIQ) and FREE2 methylation assessed using MS-QMA in blood of 19 FM females and 23 PM females; and MS-QMA methylation reference ranges in 63 FM, 82 PM, and 115 control (CGG<40) females, where formal cognitive testing was not performed. Note: blue dot-line at 0.41 methylation ratio represents the cut off methylation threshold for low functioning FM females (IQ<70) and the upper limit for the PM group. Values above 0.41 methylation ratio threshold (red broken line) identify 40% of all FM females. The green broken line at 0.37 methylation ratio represents the cut off methylation threshold above the maximum value of the control group. Values above 0.37 methylation ratio threshold identify 65% of all FM females.

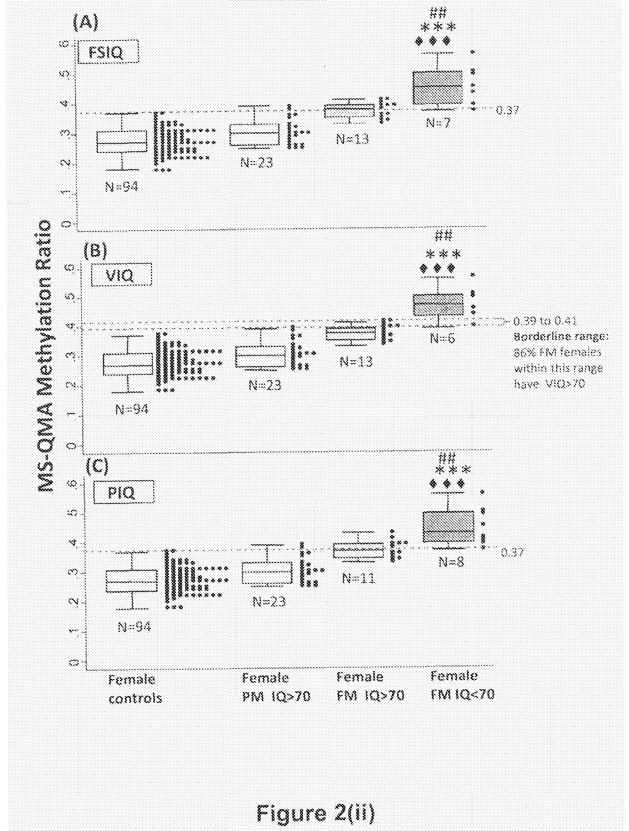

**Figure 2(ii)** is a graphical representation of a comparison between groups of FREE2 MS-QMA methylation ratio (MR) in venous blood based on Wechsler IQ scores and CGG expansion size in 138 females. **(A)** The median MS-QMA MR was significantly increased in FM females with scores < 70 for full scale IQ (FSIQ) compared to FM females with scores >70, PM females and controls. This was also the case for verbal IQ (VIQ) **(B)** and performance IQ (PIQ) **(C).** The broken line represents the optimal threshold value (cut-off point) for each IQ measure determined using area under the ROC curve (AUC) and Youden Index as summary measures of diagnostic accuracy described in Table 5.
   Red broken line represents threshold determined to provide optimal separation for FM females based on verbal IQ (VIQ) of 70. The purple broken line represents the upper limit of the borderline range where for VIQ there is overlap between FM females with VIQ > and < 70. Blue broken line represents threshold determined to provide optimal separation for FM females based on full scale IQ (FSIQ) and performance IQ (PIQ) of 70, and is equivalent to the maximum value of the female control sample. Note: FM FSIQ<70 compared to controls ***-*P*<0.001; ** -*P*<0.01; * -*P*<0.05; FM IQ<70 compared to PM IQ >70 ◆◆◆ - P<0.001; ◆◆ -*P*<0.01; ◆ -*P*<0.05; and FM IQ<70 compared to FM IQ>70 ### - *P*<0.001; ## -*P*<0.01; # -P<0.05.

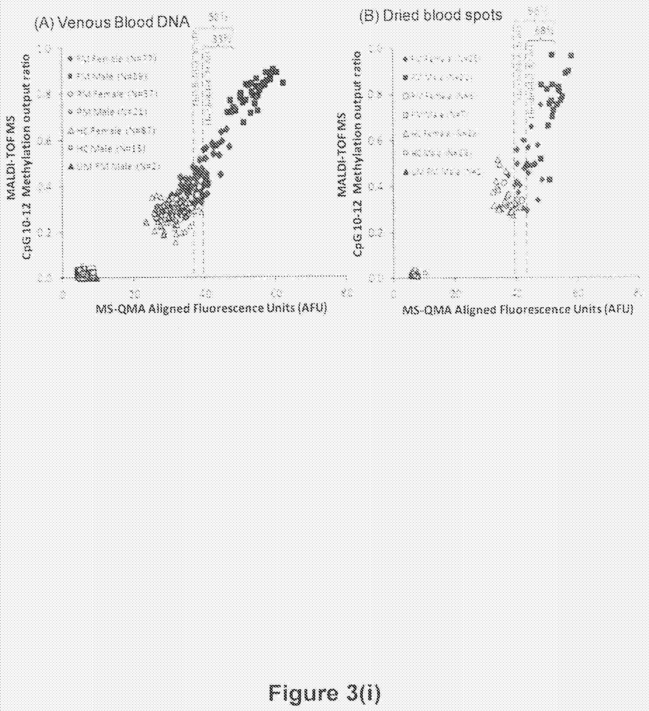

**Figure 3(i)** is a graphical representation of FREE2 (A) methylation assessed using MALDI-TOF MS and MS-QMA in: (A) DNA from venous blood; and (B) newborn and adult dried blood spots (DBS) of FXS affected FM, PM, rare non-methylated FM (UFM) not affected with FXS (IQ>70) and healthy control (HC - CGG<40) males and females. (A) DNA from venous blood values identify 33% of all FM females with 70% of these with IQ<70 and 86 of these with IQ<80 (as indicated in Figure 2(i)). 36 AFU represents the cut off methylation threshold with maximum specificity and sensitivity for FM genotype, independent of the cognitive status. (B) DBS values at 42 AFU identify 68% of all FM females with no overlap with PM and healthy control groups in females. 39 AFU represents the cut off methylation threshold with maximum specificity and sensitivity for FM genotype, independent of the cognitive status. In DBS values identify 96% of all FM females with only 4% control and 28% PM false positives. Note that in both venous blood DNA and DBS values thresholds identify 100% of FM males with FXS, with no overlap with PM, healthy control and UFM males (IQ>70) not affected with FXS.

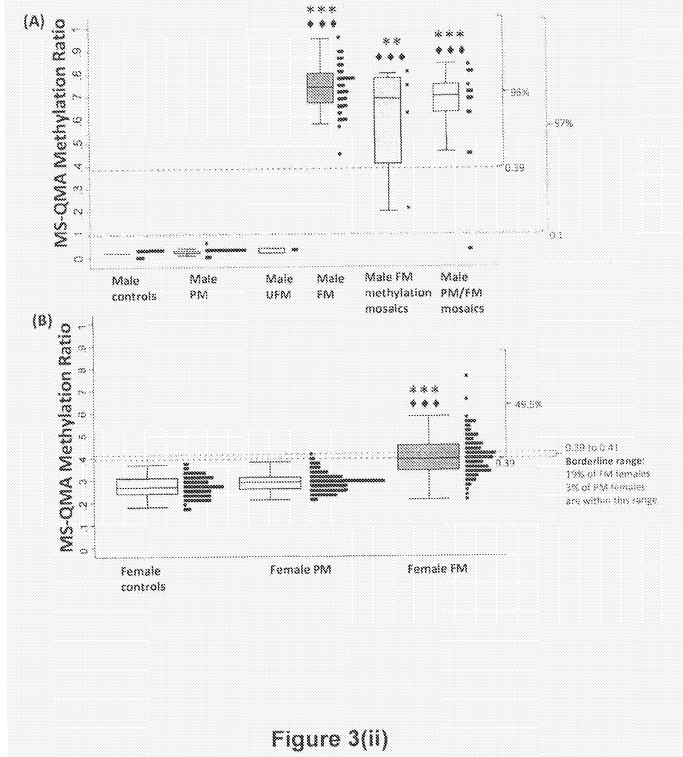

**Figure 3(ii)** is a graphical representation of the distribution of MS-QMA methylation ratio (MR) in a larger cohort of controls and *FMR1* expansion males and females. **(A)** MR in venous blood DNA from 20 male controls, 28 PM, 3 'high functioning' UFM males (identified through cascade testing with FSIQ, VIQ and PIQ >70 and determined to be unmethylated on Southern blot analysis), 52 FM males (identified through investigation of developmental delay/ASD), 4 FM methylation mosaics and 17 PM/FM size mosaics. **(B)** MR in venous blood DNA from 88 female controls, 105 PM and 95 FM females (identified through investigation of developmental delay/ASD). Note: Formal IQ testing was performed only on the UFM group. The red broken line is the threshold identified in Figure 2(ii) which separates FM females with VIQ > or <70. The purple broken line represents the upper limit of the borderline range where for VIQ there is overlap between FM females with VIQ > and< 70 in Figure 2. The green broken line is the threshold that separates FM males from UFMs, PM and control males. Each brace connected to the broken line indicates the range of methylation values and the proportion of FM identified above this threshold. Compared to controls *** -*P*<0.001; ** -*P*<0.01; * - P<0.05; compared to PM IQ >70 ◆◆◆ - P<0.001; ◆◆ -P<0.01; ◆ -*P*<0.05.

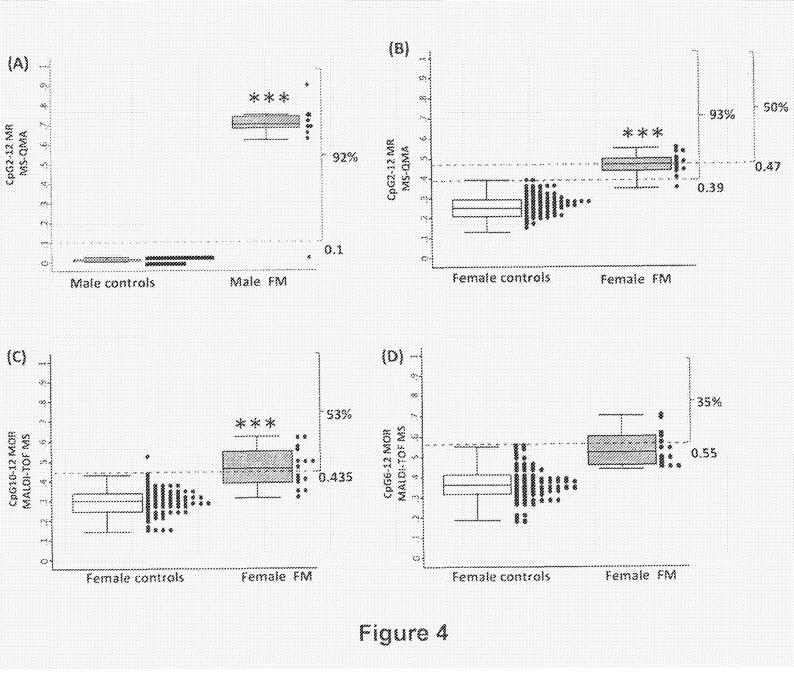

**Figure 4** is a graphical representation of MS-QMA and MALDI-TOF MS analysis of different FMR1 intron 1 FREE2 units in the same newborn blood spots (NBS): comparisons of control and FM groups. (A) MS-QMA CpG2-12 methylation ratio (MR) in spots from 87 male controls (CGG<40) and 13 FM males; (B) MS-QMA CpG2-12 MR in spots from 95 female controls (CGG<40) and 15 FM females. (C) MALDI-TOF MS CpG 10-12 methylation output ratio (MOR) and (D) MALDI-TOF MS CpG 6-12 MR in spots from 95 female controls (CGG<40) and 15 FM females. Note: Only spots stored at room temperature for 10 years or less were included, since storage within this period was found to have no significant impact on the MS-QMA and MALDI-TOF MS outputs. Compared to controls ***-*P*<0.001. The green broken line is the threshold from figure 3 that separated FM males from UFMs, PM and control males. The red broken line is the threshold from Figure 2(ii) which separated FM females of VIQ > or < 70. The purple broken line is the threshold which separated FM female newborn blood spot cohort into two equal portions. The blue broken line is the CpG6-12 threshold above maximum value of blood spot female controls. Each brace connected to the broken line gives the range of values above the specific threshold and the proportion of FM identified above this threshold.

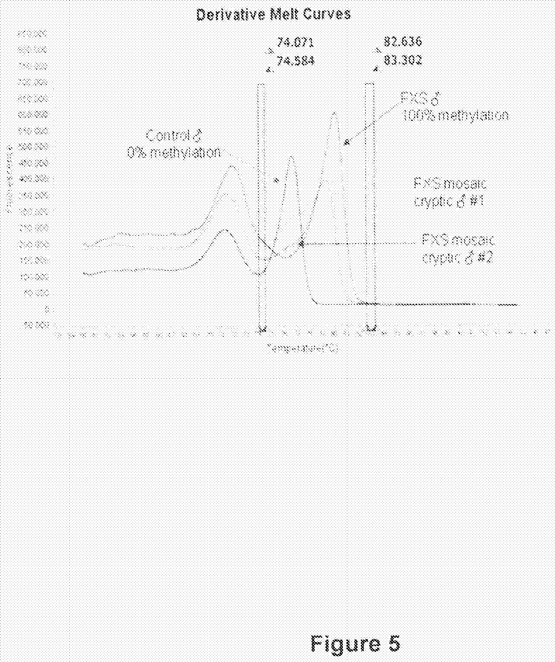

**Figure 5** is a graphical representation of MS-QMA melt profiles from venous blood DNA of FXS cryptic male #1 - IQ 30/ASD - 41 and 450 CGG repeat alleles and cryptic male #2 - IQ 30/ASD - 25 and 1300 CGG repeat alleles, both 70% methylated by MALDI-TOF MS; one control male - 0% methylated by MALDI-TOF MS and one FXS male - 100% methylated by MALDI-TOF MS.

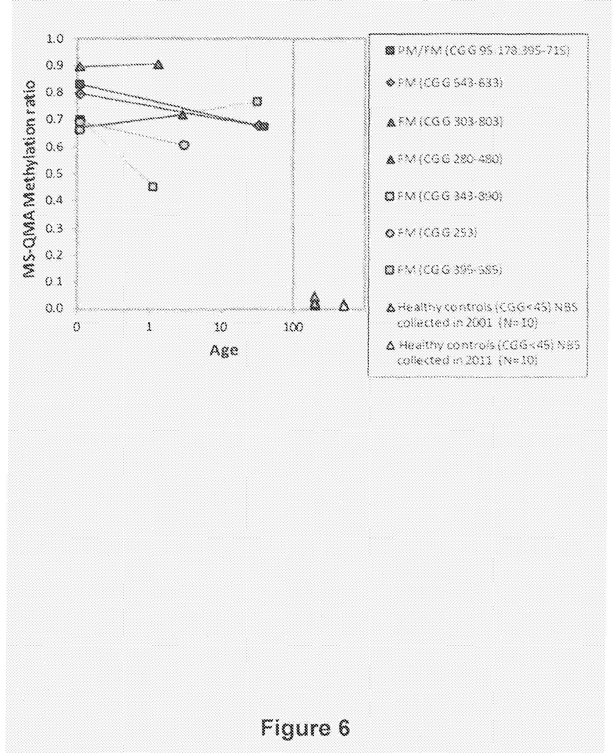

**Figure 6** is a graphical representation of FREE2 MS-QMA repeated methylation measures for 7 FXS males in blood spots taken at birth and in dried blood spots made at time of consent. MS-QMA methylation reference range in newborn blood spots of male controls (CGG<40). Open boxes/triangles represent newborn blood spots kept at room temperature for less than 2 years. Grey boxes/triangles represent newborn blood spots kept at room temperature for 11 years.

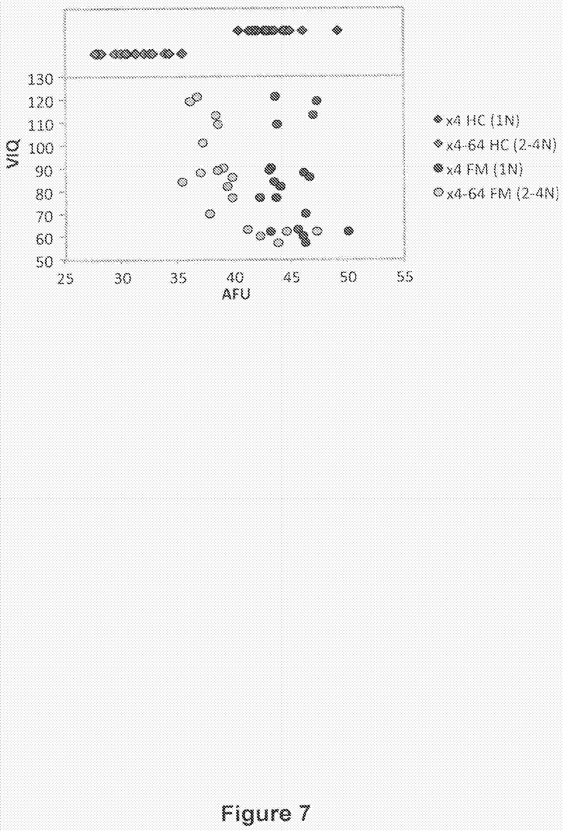

**Figure 7** is a graphical representation showing a comparison of *FMR1* MS-QMA Aligned Fluorescent Unit (AFU) with a serial dilution (2-4N) and with only a single dilution (IN) of the DNA samples with the relationship between Verbal IQ (VIQ). One AFU (red point) was obtained from 4 times diluted DNA samples (IN). Rectangular boxes: 21 control (CGG<40) females without formal cognitive testing scores. Circles: 18 FM females with VIQ.

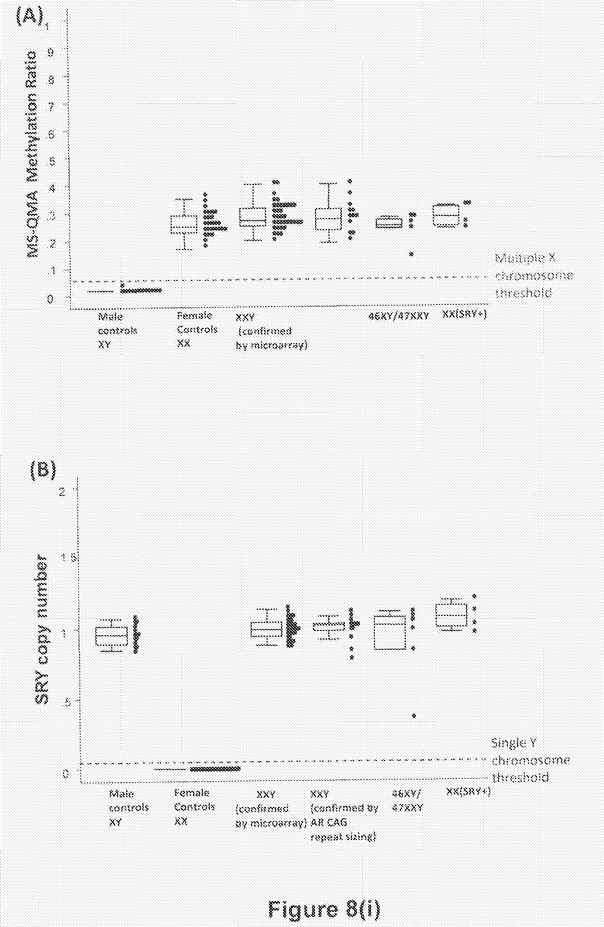

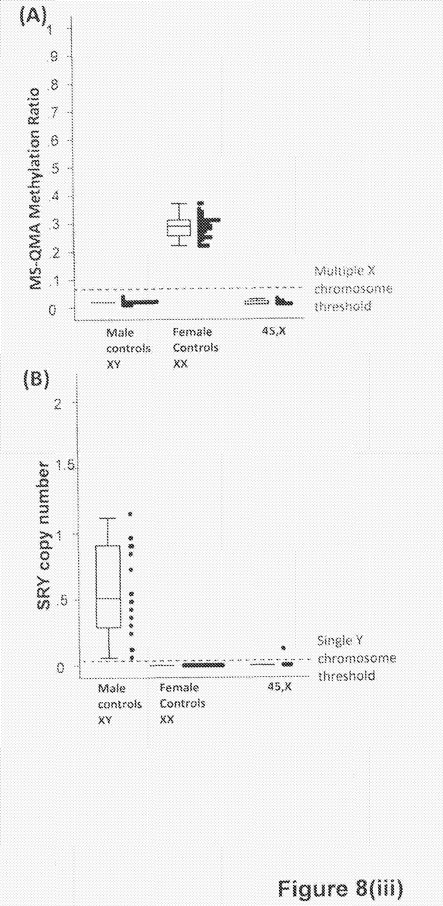

**Figure 8(i)** is a graphical representation of MS-QMA methylation ratio (MR) and SRY copy number in 100 saliva DNA samples from control males and females and individuals with clinical features of Klinefelter syndrome. The sample consisted of 13 46,XY control males (<40 CGGs); 27 46,XX control females (<40 CGGs); 36 47,XXY Klinefelter syndrome males (confirmed by microarray analysis); 12 47,XXY treated as Klinefelter syndrome males (confirmed by androgen receptor methylation analysis and androgen receptor CAG repeat length heterozygocity; no karyotype available); 5 mosaics for 46,XY/47,XXY (confirmed by microarray analysis); and 4 46,XX males with an SRY translocation (confirmed by microarray analysis); (A) The MR values determined using MS-QMA; (B) The SRY / Beta-globin copy number ratios were determined using real-time PCR relative standard curve method.

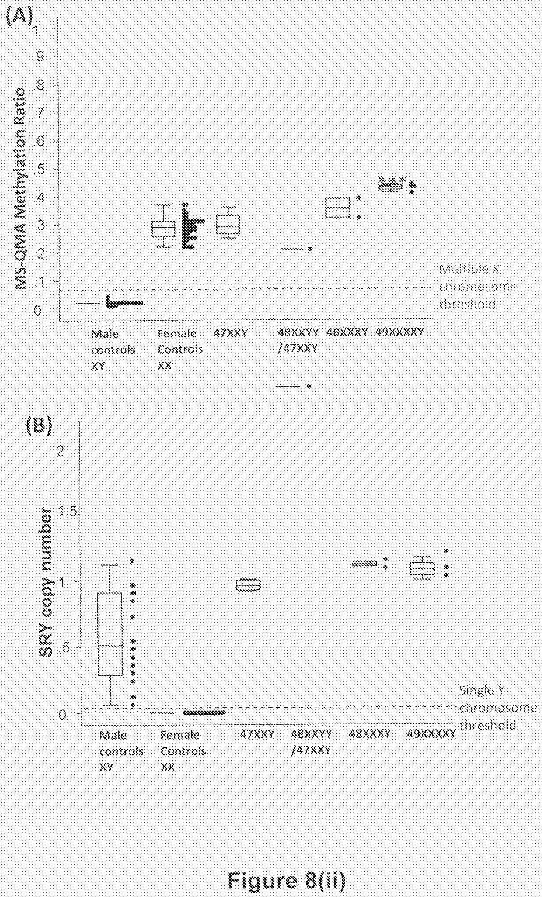

**Figure 8(ii)** is a graphical representation of MS-QMA methylation ratio (MR) and SRY copy number in blood DNA samples from control males and females and individuals with clinical features of Klinefelter syndrome. The sample consisted of 19 46,XY control males (<40 CGGs); 48 46,XX control females (<40 CGGs); 4 47,XXY Klinefelter syndrome males (confirmed by microarray analysis); 1 48,XXYY/47,XXY mosaic male (confirmed by microarray analysis); 2 48,XXXY males (confirmed by microarray analysis); and 4 49,XXXXY males (confirmed by microarray analysis). (A) The MR values determined using MS-QMA; (B) The SRY / Beta-globin copy number ratios determined using real-time PCR relative standard curve method. Note: Comparison to 46,XX controls ** - P<0.01, *** P<0.001.

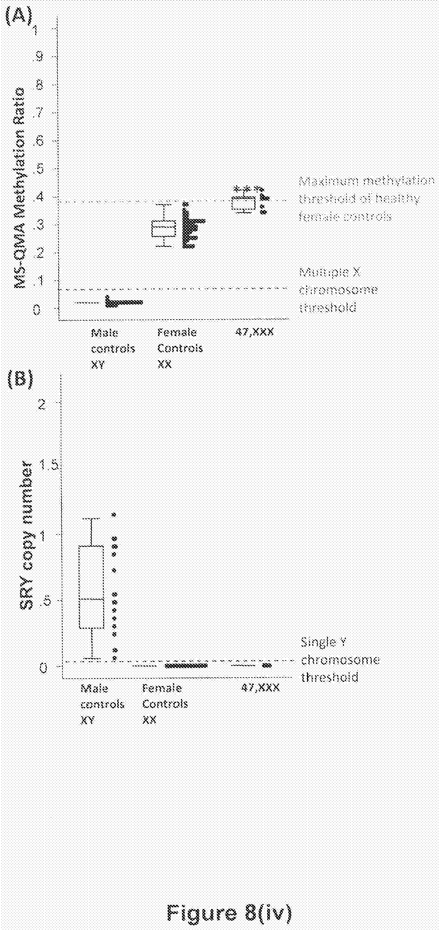

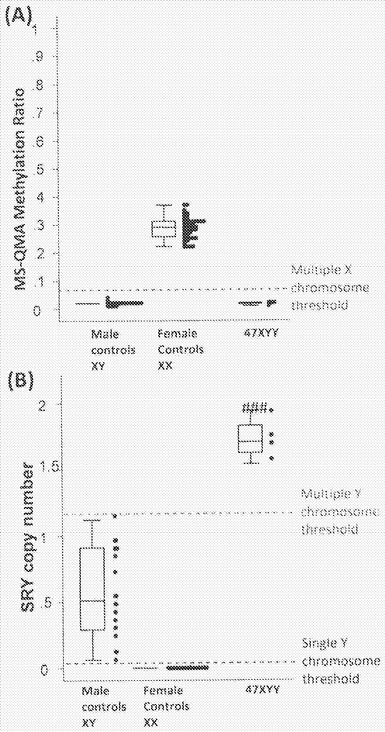

**Figure 8(iii)** in a graphical representation of MS-QMA methylation ratio (MR) and SRY copy number in blood DNA samples from control males and females and individuals with Turners Syndrome. The sample consisted of 19 46,XY control males (<40 CGGs); 48 46,XX control females (<40 CGGs); 10 45,X Turners syndrome females (confirmed by microarray analysis).
**Figure 8(iv)** is a graphical representation of MS-QMA methylation ratio (MR) and SRY copy number in blood DNA samples from control males and females and individuals with Triple X Syndrome. The sample consisted of 19 46,XY control males (<40 CGGs); 48 46,XX control females (<40 CGGs); 8 47,XXX females (confirmed by microarray analysis). Note: Comparison to 46,XX controls ** - P<0.01, *** P<0.001.
**Figure 8(v)** is a graphical representation of MS-QMA methylation ratio (MR) and *SRY* copy number in blood DNA samples from control males and females and individuals with Jacob's Syndrome. The sample consisted of 19 46,XY control males (<40 CGGs); 48 46,XX control females (<40 CGGs); 4 47,XYY males (confirmed by microarray analysis). Note: Comparison to 46,XY controls ### P<0.001.

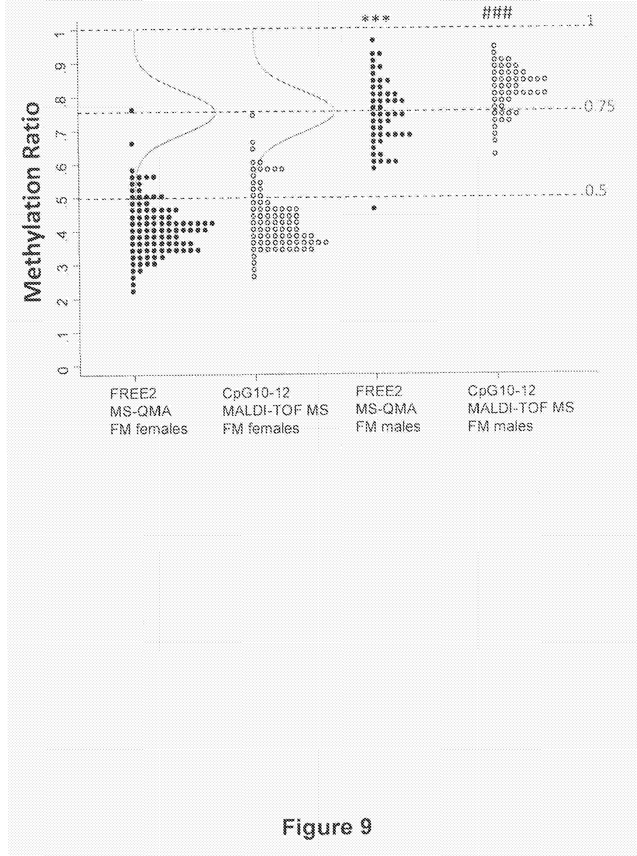

**Figure 9** is a graphical representation of FREE2 methylation comparisons between 50 FM males and 95 females with MS-QMA and MALDI-TOF MS CpG-10/12 methylation output ratio. MS-QMA targets seven CpG sites within *FMR1* intron 1 including CpG10-12; while MALDI-TOF MS CpG10-12 MOR is specific only for methylation of CpG10-12. Note: all comparisons between FM males and FM females showed P<0.001; *** comparisons for MS-QMA values; ### comparison for MALDI-TOF MS between FM groups. The bell shaped curve represents the expected normal distribution for the FM females methylation values if the X-inactivation at the locus were random, with mean methylation ratio of 0.75, the higher tail of distribution at 1 and the lower tail of distribution at 0.5.

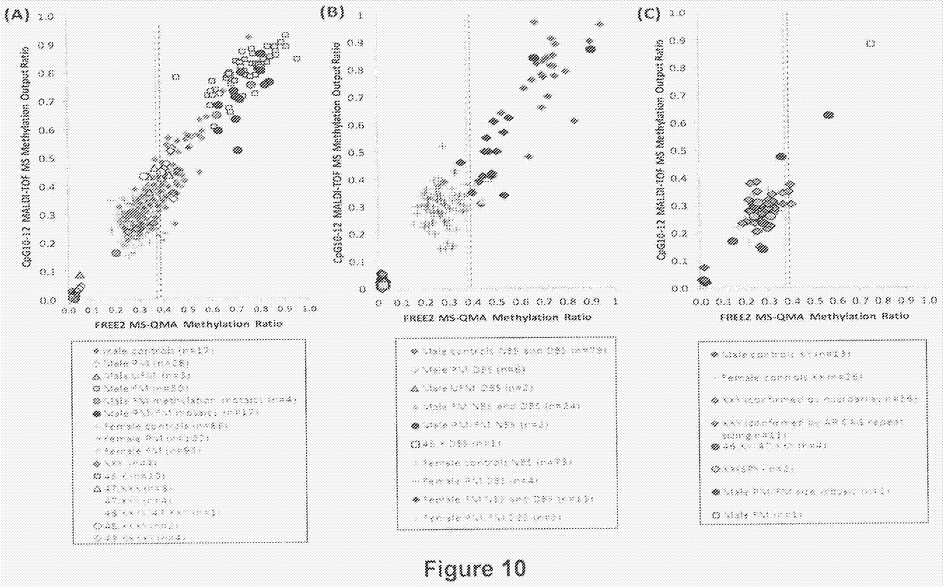

**Figure 10** is a graphical representation of FREE2 methylation assessed using MALDI-TOF MS CpG10-12 and MS-QMA in *FMR1* expansion carriers, sex chromosome aneuploidies and healthy control (CGG<40) males and females. **(A)** 433 DNA from venous blood **(B)** 239 newborn and adult dried blood spots (DBS) and **(C)** 97 Saliva DNA. Note: Linear regression analysis results corresponding to these plots are presented in Table 8. Red broken line represents threshold determined to provide optimal separation for FM females based on VIQ of 70. Blue broken line represents threshold determined to provide optimal separation for FM females based on FSIQ and PIQ of 70, and is equivalent to the maximum value of the female control sample. Dried blood spots stored for < 10 years were not included.

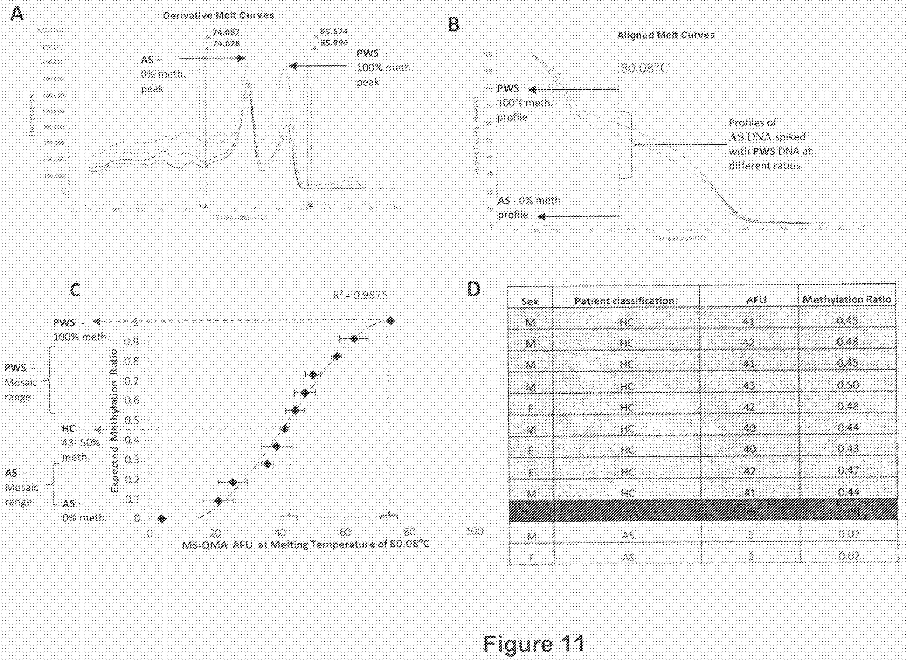

**Figure 11** shows the use of the SNRRP promoter in MS-QMA High Resolution Melt (HRM) Profiles and the HRM standard curve of 12 separate DNA spiking experiments of DNA from Prader-Willi (PWS) and Angelman (AS) syndromes. (A) representative derivative melt curves and (B) Aligned Melt Curves of AS DNA 0% methylated, where both (A) and (B) were spiked at different ratios with 100% methylated PWS DNA; (C) methylation specific standard curve representing the relationship between the mean aligned fluorescence at 80.08°C of 12 separate DNA spiking experiments determined using MS-QMA (X axis) and the expected methylation ratio in AS DNA spiked with methylated PWS DNA; the error bars represent two standard deviations between runs for each point. (D) AFU for the unknowns was converted to methylation ratio (MR) from the HRM standard curve (C); with the healthy control methylation range between 0.43 and 0.5 MR; PWS at ∼0.9 -1 MR; and AS between 0 and 0.15 MR. Based on these ranges the expected AS mosaic range was between 0.16 and 0.42 MR and the expected PWS mosaic range was between 0.51 and 0.98 MR. Note: The AFU for the methylation standard curve and unknown samples (HC, PWS and AS) was analyzed at the melting temperature of 80.08°C.

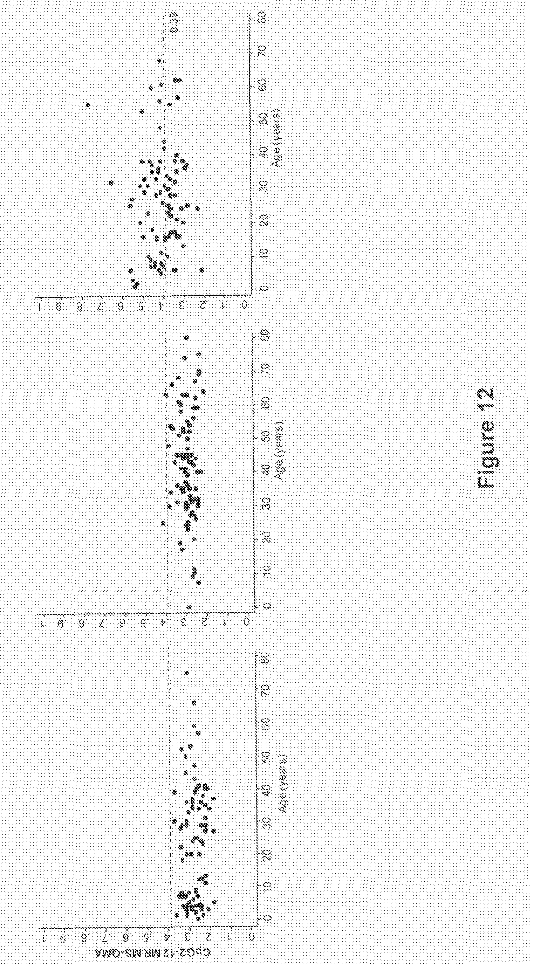

**Figure 12** is a graphical representation of the relationship between FREE2 CpG2-12 methylation ratio analysed using MS-QMA and age in female venous blood DNA. There was no significant relationship found using linear regression analysis between methylation ratio (y axis) and age (x axis) in (A) control, (B) PM and (C) FM females. We also used Davies test (Davies, 1987 Biometrika 74, 33-43) to test if there was a change in the slope in the relationship between MS-QMA-MR and age for three difference females groups. The break points were identified at 35.8 and 54.4 year of age respectively for controls and PM females, however they were both not significant (p-value = 0.36 and 0.14 respectively). For FM females the break-point occurred at age 5.98 (95% CI = 0.06-11.9), but the slopes before and after this breakpoint were not significant (p-value = 0.23 and 0.62 respectively).

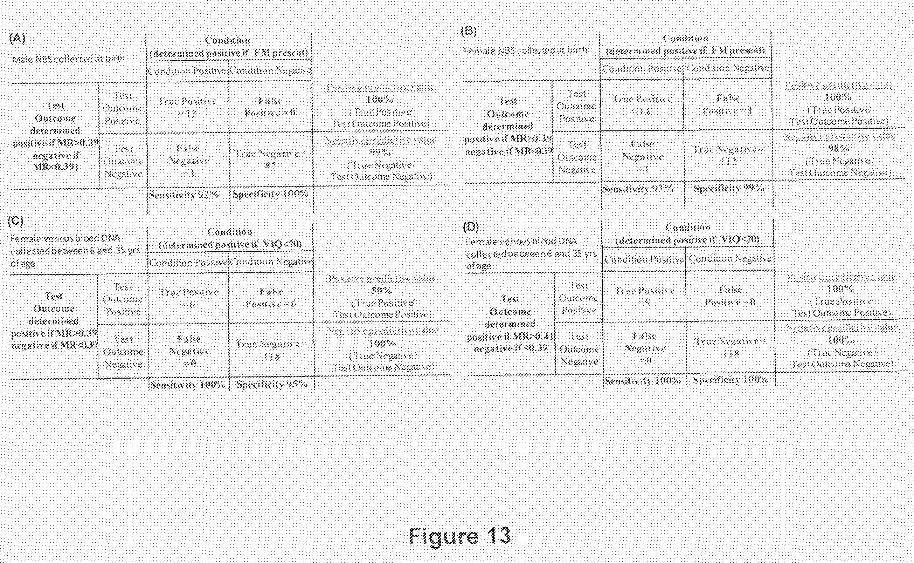

**Figure 13** Matrixes for sensitivity, specificity, positive and negative predictive values for MS-QMA MR in male and female dried blood spots collected at birth and female venous blood collected between 6 and 35 years of age. (A) NBS males and (B) NBS that had a FM allele were considered positive. In venous blood of FM females age between 6 and 35 years (C) and (D) VIQ < 70 was condition positive and VIQ > 70 was condition negative. For (C) test positive was MR>0.39 and test negative was MR < 0.39; while for (D) positive was MR>0.41 and test negative was MR < 0.39. For (D) all MR within the 2% borderline range of 0.39 to 0.41, were not included in the analyses. Note: In venous blood of FM females sensitivity and specificity were calculated based on the Youden index optimal cutoff value of 0.39 for VIQ.

### DETAILED DESCRIPTION

Throughout this specification, unless the context requires otherwise, the word "comprise" or variations such as "comprises" or "comprising", will be understood to imply the inclusion of a stated element or integer or method step or group of elements or integers or method steps but not the exclusion of any element or integer or method step or group of elements or integers or method steps.

As used in the subject specification, the singular forms "a", "an" and "the" include singular and plural aspects unless the context clearly dictates otherwise. Thus, for example, reference to "a methylation marker" includes a single methylation marker, as well as two or more different methylation markers; reference to "an algorithm" includes a single algorithm, as well as two or more algorithms.

The present disclosure teaches a methylation specific-quantitative melt analysis (MS-QMA) protocol to quantitate the extent of methylation at a target site on DNA. The MS-QMA comprises bisulfite conversion of non-methylated cytosine residues to uracil nucleotides followed by generation of a real-time amplification standard curve method using forward and reverse primers to amplify the target site and then melt analysis

The amplification and melting steps involve the incorporation of a label in real-time which emits an identifiable signal when part of double stranded DNA and wherein the signal decreases during the melt process as single stranded DNA is generated. DNA samples to be tested are analyzed based on the temperature which provides a useful separation between methylated and non-methylated standards. In an embodiment, an algorithm is used to determine DNA concentration and quality post conversion of DNA from the DNA samples to be tested from the real-time amplification standard curve. The DNA concentrations and quality control ranges are then plotted against a melt standard curve. The signal strength of the label in the unknown samples is then converted to percentage methylation from the melt standard curve.

Accordingly, enabled herein is a method for quantitating the extent of methylation of a target site within a DNA sample, the method comprising:
(i) subjecting the DNA sample to bisulfite treatment to convert non-methylated cytosine nucleotides in the DNA to uracil nucleotides;
(ii) amplifying and melting a portion of the DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA; and then
(iii) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(iv) determining the signal strength in the DNA sample to be tested which falls within the dynamic linear range of amplification and the stable amplified product melt range;
(v) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vi) determining the percentage of methylation for the DNA sample by reading the percentage of methylation from the level of signal strength using the standard curve resolved from the second control DNA sample set.

Reference to Roman numerical paragraphs is not to imply that two or more steps may precede each other or be performed simultaneously. It will be understood that the terms "percentage of methylation" and "methylation ratio" or "MR" are used herein interchangeably. The percentage methylation is calculated by multiplying the methylation ratio by 100, i.e. MR x 100 = % methylation.

Accordingly, enabled herein is a method for quantitating the extent of methylation of a target site within a DNA sample, the method comprising:
(i) subjecting the DNA sample to bisulfite treatment to convert non-methylated cytosine nucleotides in the DNA to uracil nucleotides;
(ii) amplifying and melting a portion of the DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA; and then
(iii) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(iv) determining the signal strength in the DNA sample to be tested which falls within the dynamic linear range of amplification and the stable amplified product melt range;
(v) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vi) determining the methylation ratio for the DNA from the level of signal strength using the standard curve resolved from the second control DNA sample set.

Generally, the amplification reaction is a real-time polymerase chain reaction (RT-PCR) and the melting reaction is a high resolution melt (HRM). Amplification data and thermal melt data may be monitored by any of a number of means but is conveniently monitored by an increase in fluorescence or emitted light during amplification or a decrease in fluorescence or emitted light during denaturation. The degree of, or change in, fluorescence is correlational or proportional to the degree of change in the physical conformation of the DNA.

Conveniently, the output data from the amplification and melt reactions are processed *via* an algorithm that determines DNA concentration and quality post-conversions from the real-time PCR amplification curve and which plots a sample to be tested within the concentration and quality control range against the HRM standard curve.

A method of measuring the degree of denaturation/unfolding of the target DNA is through monitoring of the fluorescence of dyes or molecules added to the reaction along with control and test DNA samples. A fluorescence dye or molecule refers to any fluorescent molecule or compound (i.e. a fluorophore) which can bind to a target DNA either once the target DNA is unfolded or denatured or before the target DNA undergoes conformational change by, e.g. denaturing and which emits fluorescent energy or light after it is excited by, e.g. light of a specified wavelength.

One dye type suitable for use herein is one that intercalates within strands of nucleic acids. The example of such a dye is ethidium bromide. An example of use of ethidium bromide for binding assays includes, e.g. monitoring for a decrease in fluorescence emission from ethidium bromide due to binding of test DNA. See, e.g. Lee et al. (1993) J Med Chem 36(7):863-870. The use of nucleic acid intercalating gents in measurement of denaturation is well known to those in the art. See, e.g. Haughland (1996) Handbook of Fluorescent Probes and Research Chemicals, Molecular Probes, Inc. Eugene, Oreg.

Dyes that bind to nucleic acids by mechanisms other than intercalation can also be employed in the subject assay. For example, dyes that bind the minor groove of double stranded DNA can be used to monitor the molecular unfolding/denaturation of the target molecule due to temperature. Examples of suitable minor groove binding dyes are the SYBR Green family of dyes sold by Molecular Probes Inc. (Eugene, Oreg., USA). See, e.g. Haughland (1996) *supra.* SYBR Green dyes will bind to any double stranded DNA molecule. When a SYBR Green dye binds to double stranded DNA, the intensity of the fluorescent emissions increases. As more double stranded DNA are denatured due to increasing temperature, the SYBR Green dye signal will decrease. Another suitable dye is LCGreen Plus sold by Idaho Technology, Inc. (Salt Lake City, Utah, USA).

In an embodiment, the stable amplified product melting range is selected such that the product has reached the maximum PCR amplification. Furthermore, in an embodiment the melting temperature is selected to provide the greatest difference between non-methylated and control samples.

Hence, taught herein is a method for quantitating the extent of methylation of a target site within a DNA sample, the method comprising:
(i) subjecting the DNA sample to bisulfite treatment to convert non-methylated cytosine nucleotides in the DNA to uracil nucleotides;
(ii) amplifying and melting a portion of the DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA; and then
(iii) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(iv) determining the signal strength in the DNA sample to be tested which falls within the dynamic linear range of amplification and the stable amplified product melt range;
(v) deriving a standard curve of signal strength released as part of melt *versus* the known percentage of methylation from the second control DNA set sample, the melting temperature for the second control DNA set selected at the signal providing a maximum difference between the non-methylated and methylated control samples; and
(vi) determining the percentage of methylation for the DNA sample by reading the percentage of methylation from the level of signal strength using the standard curve resolved from the second control DNA sample set.

In an embodiment, the DNA sample to be tested is serially diluted after bisulfite conversion. If multiple dilutions are analyzed, then the mean of the determined percentage methylation is calculated. In one embodiment, the DNA sample is serially diluted four times after bisulfite treatment. It will be understood that the terms "percentage of methylation" and "methylation ratio" or "MR" are used herein interchangeably. The percentage methylation is calculated by multiplying the methylation ratio by 100, i.e. MR x 100 = % methylation.

Taught herein is a method for quantitating the extent of methylation of a target site within a DNA sample, the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA; and then
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference such as the maximum difference between the non-methylated and methylated control samples; and then
(vii) determining the percentage of methylation for the DNA sample by reducing the percentage of methylation from the level of signal strength using the standard curve resolved from the second control DNA sample set, wherein if multiple dilutions of the same DNA sample set are selected, the mean of the determined percentage methylation is calculated;
wherein an algorithm is used to determine whether a sample set of DNA falls within the dynamic linear range and the stable amplified product melt range and to correlate signal strength with percentage of methylation.

In an embodiment, an algorithm is used to interface with the method. Hence enabled herein is a method for quantitating the extent of methylation of a target site within a DNA sample, the method comprising:
(i) subjecting the DNA sample to bisulfite treatment to convert non-methylated cytosine nucleotides in the DNA to uracil nucleotides;
(ii) optionally serially diluting the DNA sample after bisulfite conversion;
(iii) amplifying and melting a portion of the DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA; and then
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and then
(vii) determining the percentage of methylation for the DNA sample by determining the percentage of methylation from the level of signal strength using the standard curve resolved from the second control DNA sample set, wherein if multiple dilutions of the same DNA sample set are selected, the mean of the determined percentage methylation is calculated;
wherein an algorithm is used to determine whether a sample set of DNA falls within the dynamic linear range and the stable amplified product melt range and to correlate signal strength with percentage of methylation.

In an embodiment, the extent of methylation of a target DNA site on a genome is correlated to a clinical phenotype.

Hence, a method for quantitating the extent of methylation of a target site within a DNA sample, the level of which is associated with a clinical phenotype, the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) subjecting the bisulfite treated DNA sample comprising the target site to real-time PCR and high resolution in melt with selected forwarded and reverse primers incorporating a fluorescence label in real-time and releasing the fluorescence label from double stranded DNA as determined by the AFU as part of the melting, the PCR and melt occurring in the presence of a first control DNA of known concentration providing an identifiable fluorescence signal in double stranded DNA as determined by and a second control DNA of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) deriving a standard curve of signal strength measured in AFU released as part of melt *versus* the known percentage of methylation from the second control DNA sample set, the melting temperature for the second control DNA set selected at the signal providing the greatest difference between the non-methylated and methylated control samples;
(vi) determining the signal strength in AFU in the DNA sample to be tested which falls within the dynamic linear range of amplification and the stable amplified product melt range;
(vii) determining the percentage of methylation for the DNA sample by reading the percentage of methylation from the level of signal strength in AFU using the standard curve resolved from the second control DNA sample set, wherein the percentage of methylation correlates with a particular clinical phenotype.

Examples of clinical phenotype include neurological disorders, imprinting disorders, modified X-chromosome disorders, cancers, transplant rejection and pregnancies.

Examples of neurological disorders includes fragile X syndrome (FXS), fragile X-associated tremor ataxia syndrome (FXTAS), autism, mental retardation, cognitive impairment, Klinefelter's syndrome, Turner's syndrome, Prada-Willi syndrome syndrome/Angelman syndrome and fragile X-associated primary ovarian insufficiency (FXPOI) and other disorders associated with aneuploidy and/or modified X-chromosome, including Triple X Syndrome and Jacob's Syndrome. In an embodiment, the condition is associated with skewed X-chromosome inactivation.

In an embodiment, the neurological condition is cognitive and behavioral impairment, a neurodevelopmental and/or neurodegenerative disorder, attention deficit disorder, mood disorder, schizophrenia, bipolar disorder, memory lapse, and/or poor memory retention.

Conditions contemplated herein include pathoneurological conditions such as pathoneurodevelopmental and pathoneurodegenerative conditions as well as non-neurological conditions. Conditions and disorders contemplated herein include polyglutamine (polyQ) diseases such as Huntington's disease (HD), dentatorubropallidoluysiantrophy (DRPLA), spinobulbar muscular atrophy or Kennedy disease (SBMA), spinocerebella ataxia Type 1 (SCA1), spinocerebella ataxia Type 2 (SCA2), spinocerebella ataxia Type 3 or Machado-Joseph disease (SCA3), spinocerebella ataxia Type 6 (SCA6), spinocerebella ataxia Type 7 (SCA7), spinocerebella ataxia Type 17 (SCA17) and non-polyQ diseases such as Fragile X syndrome (FXS), Fragile X-associated tremor or ataxia (FXTAS), Fragile XE mental retardation (FRAXE), myotonic dystrophy (DM), spinocerebella ataxia (SCA8) and spinocerebella ataxias Type 12 (SCA12). Other conditions contemplated herein include trinucleotide expansion related disorders including but not limited to Fragile X-associated primary ovary insufficiency (FXPOI) and Friedrich's ataxia (FRDA). Fragile type, folic acid type, rare 12 (FRA12A), autism (including co-morbid autism), mental retardation (MR), Klinefelter's syndrome, RNA toxicity disease, Turner's syndrome, a modified X-chromosome disorder including skewed X-chromosome inactivation, and cognitive impairment and also contemplated. Further contemplated herein are learning and behavioral problems.

A methylation map of a target DNA site can thus be constructed in accordance with the MS-QMA assay in the genome of various cells. Any cell type may be assayed. These cells include cultured or uncultured chorionic villi sample (CVS) cells, lymphoblasts, blood cells including whole blood, blood fraction, venous blood, arterial blood and dried blood, buccal cells, an amniocyte and EBV transformed lymphoblast cell lines from male and female subjects with either no clinical phenotype or from a spectrum clinical phenotypes.

In an embodiment, the DNA target site is selected from fragile X-related epigenetic elements FREE1, FREE2 and FREE3. Reference herein to "FREE2" includes FREE2 (A), FREE2 (B), FREE2 (C), FREE2 (D) and/or FREE2 (E). Reference can be made to the contents of International Patent Publication No. WO 2012/174610. It is proposed that these regions are responsible neurological phenotypes including autism, cognitive impairment, FXS and mental retardation phenotype including fragile X mental retardation-like conditions and FMR conditions.

In another embodiment, the DNA target site is selected from the SNRPN genetic locus including its promoter region. It is proposed that this region is responsible for neurological phenotypes including Prada-Willi syndrome (PWS)/Angelman syndrome (AS).

Hence, in an embodiment, the instant disclosure correlates a change in extent of methylation of a genetic locus or region with a clinical manifestation. The quantitated extent of methylation provides an indicator as to the presence or absence of a condition severity or stage of progression of the condition and/or its amelioration during treatment.

The present assay also demonstrates that the quantitative methylation pattern of the DNA target site is significantly associated with symptoms of a pathological condition compared to healthy controls with normal size alleles. Thus, assessment of the methylation pattern of the contemplated DNA target site is proposed to be a useful biomarker for the rapid and wide spread screening of infants, neonates and other age groups for neurodegenerative, neurodevelopmental or other disorders characterized by changes in methylation pattern.

A "normal" or "control" in the present assay may be a control genome from a healthy individual performed at the same time or the methylation pattern may be compared to a statistically validated standard. A healthy individual includes a subject with no symptoms of a pathological condition. A healthy individual also includes a subject with a (CGG)ₙ where ₙ is <40, with no clinically apparent neurological phenotype.

As used herein, the terms "subject", "patient", "individual", "target" and the like refer to any organism or cell of the organism on which the MS-QMA assay of the present disclosure is performed whether for experimental, diagnostic, prophylactic, and/or therapeutic purposes. Typical subjects include both male and female humans but the present assay extends to experimental animals such as non-human primates, (e.g., mammals, mice, rats, rabbits, pigs and guinea pigs/hamsters). The "subject" may also be referred to as a population since the present assay is useful in population studies including epidemiological prevalence studies or assays of ethnic population. In an embodiment, the subject is a human. The test may be tailored to human females or human males or pre-natal humans or a DNA from a cell of a human zygote.

The terms "fragile X mental retardation-like condition" and "FMR condition" refer to a neurological disease, disorder and/or condition characterized by one or more of the following symptoms: (1) behavioral symptoms, including but not limited to hyperactivity, stereotypy, anxiety, seizure, impaired social behavior, and/or cognitive delay; (2) defective synaptic morphology, such as an abnormal number, length, and/or width of dendritic spines; and/or (3) defective synaptic function, such as enhanced long-term depression (LTD); and/or reduced long-term potentiation (LTP); and/or impaired cognitive ability. The pathological condition is a disease, disorder, and/or condition caused by and/or associated with one or more of the following: (1) a mutation in FMR1 or FMR4 or ASFMR1; (2) defective FMR1/FMR4/ASFMR1 expression; (3) increased and/or decreased levels of FMRP; (4) defective FMRP function; (5) increased and/or decreased expression of genes or genetic functions regulated by FMR1, FMRP, FMR4 transcript or ASFMR1 transcript; (6) the increased methylation of FMR locus at CpG or CpNpG sites in the region upstream of FMR1 promoter and/or the region downstream of the (CGG)ₙ portion of the FMR1 promoter but not including the (CGG)ₙ portion; (7) an increased and/or decreased function of the FMR locus *via* miRNAs and/or members of the miRNA pathway; (8) an increased and/or decreased ability of FMRP to interact with its known target RNAs, such as RNAs encoding Racl, microtubule-associated protein IB, activity-regulated cytoskeleton-associated protein, and/or alpha-calcium/calmodulin-dependent protein kinase II; and/or (9) symptoms of FXS, FXTAS, POF, mental retardation, autism and/or autism spectrum disorders. Those of ordinary skill in the art will appreciate that the teachings of the present disclosure are applicable to any neurodevelopmental or neurodegenerative disorders linked, associated or otherwise influenced by the function of the FMR genetic locus or genes therein such as FMR1, FMR4 and ASFMR1. Non-neurological disorders are also contemplated herein including FXPOI.

The term "genomic DNA" includes all DNA in a cell, group of cells, or in an organelle of a cell and includes exogenous DNA such a transgenes introduced into a cell.

The present disclosure further contemplates a method for identifying in a genome of a mammalian cell including a human cell, a pathological condition associated with methylation within the FMR locus, the method comprising extracting genomic DNA from the cell and subjecting the DNA to a method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set; the amplification reaction using primers selective of a region of the FMR genetic locus comprising CpG and/or CpNpG sites, the CpG and CpNpG sites located in a region selected from:
   (i) fragile X-related epigenetic element 2 (A) [FREE2 (A)] comprising the nucleotide sequence set forth in SEQ ID NO:1 or a homolog thereof or portion or part thereof defined by having at least 80% nucleotide sequence identity to SEQ ID NO:1 or which hybridizes to SEQ ID NO:1 or its complementary form under medium stringency conditions;
   (ii) fragile X-related epigenetic element 2 (B) [FREE2 (B)] comprising the nucleotide sequence set forth in SEQ ID NO:2 or a homolog thereof or portion or part thereof defined by having at least 80% nucleotide sequence identity to SEQ ID NO:2 or which hybridizes to SEQ ID NO:2 or its complementary form under medium stringency conditions;
   (iii) fragile X-related epigenetic element 2 (C) [FREE2 (C)] comprising the nucleotide sequence set forth in SEQ ID NO:3 or a homolog thereof or portion or part thereof defined by having at least 80% nucleotide sequence identity to SEQ ID NO:3 or which hybridizes to SEQ ID NO:3 or its complementary form under medium stringency conditions;
   (iv) fragile X-related epigenetic element 2 (D) [FREE2 (D)] comprising the nucleotide sequence set forth in SEQ ID NO:4 or a homolog thereof or portion or part thereof defined by having at least 80% nucleotide sequence identity to SEQ ID NO:4 or which hybridizes to SEQ ID NO:4 or its complementary form under medium stringency conditions;
   (v) fragile X-related epigenetic element 2 (E) [FREE2 (E)] comprising the nucleotide sequence set forth in SEQ ID NO:5 or a homolog thereof or portion or part thereof defined by having at least 80% nucleotide sequence identity to SEQ ID NO:5 or which hybridizes to SEQ ID NO:5 or its complementary form under medium stringency conditions; and
   (vi) fragile X-related epigenetic element 3 (FREE3) comprising the nucleotide sequence set forth in SEQ ID NO:6 or a homolog thereof or portion or part thereof defined by having at least 80% nucleotide sequence identity to SEQ ID NO:6 or which hybridizes to SEQ ID NO:6 or its complementary form under medium stringency conditions.

Insofar as the melt reaction relates to FREE2 or FREE3 or other FMR locus regions, the temperature melting giving maximum difference between methylated and non-methylated DNA is 78°C ± 10°C. This includes 68, 69, 70, 71, 72, 73, 74, 75, 76, 77, 78, 79, 80, 81, 82, 83, 84, 85, 86, 87 and 88°C or a fraction in between.

The present disclosure further contemplates a method for identifying in a genome of a mammalian cell including a human cell, a pathological condition associated with methylation within the SNRPN locus, the method comprising extracting genomic DNA from the cell and subjecting the DNA to a method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set; the amplification reaction using primers selective of a region of the SNRPN genetic locus comprising CpG and/or CpNpG sites, the CpG and CpNpG sites located in a region selected from:
   (i) SNRPN genetic locus (SNRPN-M) comprising the nucleotide sequence set forth in SEQ ID NO:19 or a homolog thereof or portion or part thereof defined by having at least 80% nucleotide sequence identity to SEQ ID NO:19 or which hybridizes to SEQ ID NO:19 or its complementary form under medium stringency conditions; and
   (ii) SNRPN genetic locus (SNRPN-P) comprising the nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions.

Insofar as the melt reaction relates to SNRPN, the temperature melting giving maximum difference between methylated and non-methylated DNA is 80°C ± 10°C. This includes 70, 71, 72, 73, 74, 75, 76, 77, 78, 79, 80, 81, 82, 83, 84, 85, 86, 87, 88, 89 and 90°C or a fraction in between. In one embodiment the temperature melting giving maximum difference between methylated and non-methylated DNA is 80.08°C.

Any real-time amplification, methodology may be employed. Amplification methodologies contemplated herein include the polymerase chain reaction (PCR) such as disclosed in U.S. Patent Nos. 4,683,202 and 4,683,195; the ligase chain reaction (LCR) such as disclosed in European Patent Application No. EP-A-320 308 and gap filling LCR (GLCR) or variations thereof such as disclosed in International Patent Publication No. WO 90/01069, European Patent Application EP-A-439 182, British Patent No. GB 2,225,112A and International Patent Publication No. WO 93/00447. Other amplification techniques include Qβ replicase such as described in the literature; Stand Displacement Amplification (SDA) such as described in European Patent Application Nos. EP-A-497 272 and EP-A-500 224; Self-Sustained Sequence Replication (3SR) such as described in Fahy et al. (1991) PCR Methods Appl. 1(1):25-33) and Nucleic Acid Sequence-Based Amplification (NASBA) such as described in the literature.

A PCR amplification process is useful in the practice of the present assay.

A "nucleic acid" as used herein, is a covalently linked sequence of nucleotides in which the 3' position of the phosphorylated pentose of one nucleotide is joined by a phosphodiester group to the 5' position of the pentose of the next nucleotide and in which the nucleotide residues are linked in specific sequence; i.e. a linear order of nucleotides. A "polynucleotide" as used herein, is a nucleic acid containing a sequence that is greater than about 100 nucleotides in length. An "oligonucleotide" as used herein, is a short polynucleotide or a portion of a polynucleotide. An oligonucleotide typically contains a sequence of about two to about one hundred bases. The word "oligo" is sometimes used in place of the word "oligonucleotide". The term "oligo" also includes a particularly useful primer length in the practice of the present disclosure of up to about 10 nucleotides.

As used herein, the term "primer" refers to an oligonucleotide or polynucleotide that is capable of hybridizing to another nucleic acid of interest under particular stringency conditions. A primer may occur naturally as in a purified restriction digest or be produced synthetically, by recombinant means or by PCR amplification. The terms "probe" and "primers" may be used interchangeably, although to the extent that an oligonucleotide is used in a PCR or other amplification reaction, the term is generally "primer". The ability to hybridize is dependent in part on the degree of complementarity between the nucleotide sequence of the primer and complementary sequence on the target DNA.

The terms "complementary" or "complementarity" are used in reference to nucleic acids (i.e. a sequence of nucleotides) related by the well-known base-pairing rules that A pairs with T or U and C pairs with G. For example, the sequence 5'-A-G-T-3' is complementary to the sequence 3'-T-C-A-5' in DNA and 3'-U-C-A-5' in RNA. Complementarity can be "partial" in which only some of the nucleotide bases are matched according to the base pairing rules. On the other hand, there may be "complete" or "total" complementarity between the nucleic acid strands when all of the bases are matched according to base-pairing rules. The degree of complementarity between nucleic acid strands has significant effects on the efficiency and strength of hybridization between nucleic acid strands as known well in the art. This is of particular importance in detection methods that depend upon binding between nucleic acids, such as those of the disclosure. The term "substantially complementary" is used to describe any primer that can hybridize to either or both strands of the target nucleic acid sequence under conditions of low stringency as described below or, preferably, in polymerase reaction buffer heated to 95°C and then cooled to room temperature. As used herein, when the primer is referred to as partially or totally complementary to the target nucleic acid, that refers to the 3'-terminal region of the probe (i.e. within about 10 nucleotides of the 3'-terminal nucleotide position).

Reference herein to a stringency in relation to hybridization includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C) % (Marmur and Doty, (1962) J. Mol. Biol. 5:109). However, the Tₘ of a duplex DNA decreases by 1°C with every increase of 1% in the number of mismatch base pairs (Bonner and Laskey (1974) Eur. J. Biochem. 46:83). Formamide is optional in these hybridization conditions. Accordingly, particularly preferred levels of stringency are defined as follows: low stringency is 6 x SSC buffer, 0.1% w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C. Reference to at least "80% identity" includes 80, 81, 82, 83, 84, 85, 86, 87, 88, 89, 90, 91, 92, 93, 94, 95, 96, 97, 98, 99 and 100%.

As indicated above, the cells may be a lymphoblast, a CVS cell, a blood cell, an amniocyte or an EBV transformed lymphoblast cell line. In addition, the methylation profile may be determined or one or both alleles of a genetic locus such as the FMR or the SNRPN genetic locus and in selected cells where mosaicism has occurred. In particular, the extent of methylation can determine homozygosity, heterozygosity and mosaicism in male and female subjects. Reference to "mosaicism" includes the situation wherein two alleles of the same locus have different methylation profiles.

The present disclosure also contemplates kits for determining the methylation or at one or more sites within the genome of a eukaryotic cell or group of cells. The kits may comprise many different forms but in one embodiment, the kits comprise reagents for the bisulfite methylation assay, an amplification reaction and a melt reaction. The kits may further comprise a DNA reference/standard, software algorithms to determine the level of DNA methylation after bisulfite treatment.

A further embodiment enabled herein is a kit for the use in the above methods comprising primers to amplify the FREE2 and/or FREE3, the kit used in the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set.

By "FREE2" means any or all of FREE2 (A), FREE2 (B), FREE2 (C), FREE2 (D) and/or FREE2 (E). In an embodiment, the FREE2 (A) region is screened for extent of methylation.

In an embodiment, the present disclosure provides a use of primers which amplify a DNA sample selected from:
(i) fragile X-related epigenetic element 2 (A) [FREE2 (A)] comprising the nucleotide sequence set forth in SEQ ID NO:1 or a homolog thereof or portion or part thereof defined by having at least 80% nucleotide sequence identity to SEQ ID NO:1 or which hybridizes to SEQ ID NO: 1 or its complementary form under medium stringency conditions;
(ii) fragile X-related epigenetic element 2 (B) [FREE2 (B)] comprising the nucleotide sequence set forth in SEQ ID NO:2 or a homolog thereof or portion or part thereof defined by having at least 80% nucleotide sequence identity to SEQ ID NO:2 or which hybridizes to SEQ ID NO:2 or its complementary form under medium stringency conditions;
(iii) fragile X-related epigenetic element 2 (C) [FREE2 (C)] comprising the nucleotide sequence set forth in SEQ ID NO:3 or a homolog thereof or portion or part thereof defined by having at least 80% nucleotide sequence identity to SEQ ID NO:3 or which hybridizes to SEQ ID NO:3 or its complementary form under medium stringency conditions;
(iv) fragile X-related epigenetic element 2 (D) [FREE2 (D)] comprising the nucleotide sequence set forth in SEQ ID NO:4 or a homolog thereof or portion or part thereof defined by having at least 80% nucleotide sequence identity to SEQ ID NO:4 or which hybridizes to SEQ ID NO:4 or its complementary form under medium stringency conditions;
(v) fragile X-related epigenetic element 2 (E) [FREE2 (E)] comprising the nucleotide sequence set forth in SEQ ID NO:5 or a homolog thereof or portion or part thereof defined by having at least 80% nucleotide sequence identity to SEQ ID NO:5 or which hybridizes to SEQ ID NO:5 or its complementary form under medium stringency conditions; and
(vi) fragile X-related epigenetic element 3 (FREE3) comprising the nucleotide sequence set forth in SEQ ID NO:6 or a homolog thereof or portion or part thereof defined by having at least 80% nucleotide sequence identity to SEQ ID NO:6 or which hybridizes to SEQ ID NO:6 or its complementary form under medium stringency conditions,
in the manufacture of a diagnostic kit or device to detect methylation of the FMR locus-associated with a pathological condition wherein the extent of methylation is determined by the method comprising:
subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set;
and then correlating the extent of methylation with the pathological condition.

In a related embodiment, taught herein is a set of primers which amplify a DNA sample selected from:
(i) fragile X-related epigenetic element 2 (A) [FREE2 (A)] comprising the nucleotide sequence set forth in SEQ ID NO:1 or a homolog thereof or portion or part thereof defined by having at least 80% nucleotide sequence identity to SEQ ID NO:1 or which hybridizes to SEQ ID NO:1 or its complementary form under medium stringency conditions;
(ii) fragile X-related epigenetic element 2 (B) [FREE2 (B)] comprising the nucleotide sequence set forth in SEQ ID NO:2 or a homolog thereof or portion or part thereof defined by having at least 80% nucleotide sequence identity to SEQ ID NO:2 or which hybridizes to SEQ ID NO:2 or its complementary form under medium stringency conditions;
(iii) fragile X-related epigenetic element 2 (C) [FREE2 (C)] comprising the nucleotide sequence set forth in SEQ ID NO:3 or a homolog thereof or portion or part thereof defined by having at least 80% nucleotide sequence identity to SEQ ID NO:3 or which hybridizes to SEQ ID NO:3 or its complementary form under medium stringency conditions;
(iv) fragile X-related epigenetic element 2 (D) [FREE2 (D)] comprising the nucleotide sequence set forth in SEQ ID NO:4 or a homolog thereof or portion or part thereof defined by having at least 80% nucleotide sequence identity to SEQ ID NO:4 or which hybridizes to SEQ ID NO:4 or its complementary form under medium stringency conditions;
(v) fragile X-related epigenetic element 2 (E) [FREE2 (E)] comprising the nucleotide sequence set forth in SEQ ID NO:5 or a homolog thereof or portion or part thereof defined by having at least 80% nucleotide sequence identity to SEQ ID NO:5 or which hybridizes to SEQ ID NO:5 or its complementary form under medium stringency conditions; and
(vi) fragile X-related epigenetic element 3 (FREE3) comprising the nucleotide sequence set forth in SEQ ID NO:6 or a homolog thereof or portion or part thereof defined by having at least 80% nucleotide sequence identity to SEQ ID NO:6 or which hybridizes to SEQ ID NO:6 or its complementary form under medium stringency conditions.

In relation to the targeting FREE2 (A), forward and reverse primers are those defined by SEQ ID NOs:7 and 8, respectively. FREE2 (B) can be targeted by forward and reverse primers defined by SEQ ID NOs:9 and 10, respectively. FREE2 (C) can be targeted by forward and reverse primers defined by SQ ID NOs:11 and 12, respectively. FREE3 can be targeted by forward and reverse primers defined by SEQ ID NOs:13 and 14, respectively.

In relation to an embodiment of the present disclosure a kit is provided for the use in the above methods comprising primers identified by SEQ ID NOs:7 and 8 (FREE2 (A)), SEQ ID NOs:9 and 10 (FREE2 (B)), SEQ ID :NOs:11 and 12 (FREE2 (C)) and SEQ ID NOs:13 and 14 (FREE2 (E)).

A further embodiment enabled herein is a kit for the use in the above methods comprising primers to amplify SNRPN, the kit used in the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set.

In an embodiment, the present disclosure provides a use of primers which amplify a DNA sample comprising the nucleotide sequence selected from:
(i) SNRPN genetic locus (SNRPN-M) comprising the nucleotide sequence set forth in SEQ ID NO:19 or a homolog thereof or portion or part thereof defined by having at least 80% nucleotide sequence identity to SEQ ID NO:19 or which hybridizes to SEQ ID NO:19 or its complementary form under medium stringency conditions; and
(ii) SNRPN genetic locus (SNRPN-P) comprising the nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions,
in the manufacture of a diagnostic kit or device to detect methylation of the SNRPN locus-associated with a pathological condition wherein the extent of methylation is determined by the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set;
and then correlating the extent of methylation with the pathological condition.

In a related embodiment, taught herein is a set of primers which amplify a DNA sample comprising the nucleotide sequence set forth comprising the nucleotide sequence selected from:
(i) SNRPN genetic locus (SNRPN-M) comprising the nucleotide sequence set forth in SEQ ID NO:19 or a homolog thereof or portion or part thereof defined by having at least 80% nucleotide sequence identity to SEQ ID NO:19 or which hybridizes to SEQ ID NO:19 or its complementary form under medium stringency conditions; and
(ii) SNRPN genetic locus (SNRPN-P) comprising the nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions.

In relation to the targeting SNRPN-M, forward and reverse primers are those defined by SEQ ID NOs:15 and 16, respectively. SNRPN-P can be targeted by forward and reverse primers defined by SEQ ID NOs:17 and 18, respectively.

In relation to an embodiment of the present disclosure a kit is provided for the use in the above methods comprising primers identified by SEQ ID NOs:15 and 16 (SNRPN-M) and SEQ ID NOs:17 and 18 (SNRPN-P).

The kit may also comprise instructions for use.

Conveniently, the kit comprises a multi-compartmental microtiter tray. Furthermore, buffers, nucleotides and/or enzymes may be combined into a single compartment or multiple compartment.

As stated above, instructions optionally present in such kits instruct the user on how to use the components of the kit to perform the various methods of the present assay. It is contemplated that these instructions include a description of the use of an algorithm to determine the extent of methylation of a target site.

The present disclosure further contemplates kits which contain a primer for a nucleic acid target of interest with the primer being complementary to a predetermined nucleic acid target. In another embodiment, the kit contains multiple primers or probes, each of which contains a different base at an interrogation position or which is designed to interrogate different target DNA sequences. In a contemplated embodiment, multiple probes are provided for a set of nucleic acid target sequences that give rise to analytical results which are distinguishable for the various probes. The multiple probes may be in microarray format for ease of use. Kits may further comprise vessels containing labels and vessels containing reagents for attaching the labels. Microtiter trays are useful and these may comprise from two to 100,000 wells or from about six to about 10,000 wells or from about six to about 1,000 wells.

Another important application is in the high throughput screening of agents to determine the degree of which demethylation or hypermethylation of whole genomes or specific genomic loci. This may be important, for example, in de-differentiating cells.

The present disclosure further enables a method for screening for an agent which modulates methylation of a target site with a DNA sample, the method comprising screening for a change relative to a control in the extent of methylation modification within the target site in the presences or absence of an agent to be tested, wherein an agent is selected if it induces a change in the extent of methylation, the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set.

By "FREE2" means any or all of FREE2 (A), FREE2 (B), FREE2 (C), FREE2 (D) and/or FREE2 (E). In an embodiment, the FREE2 (A) region is assayed.

In an embodiment, the target site is within the FMR genetic locus in a mammalian cell including a human cell, and includes:
(i) fragile X-related epigenetic element 2 (A) [FREE2 (A)] comprising the nucleotide sequence set forth in SEQ ID NO:1 or a homolog thereof or portion or part thereof defined by having at least 80% nucleotide sequence identity to SEQ ID NO:1 or which hybridizes to SEQ ID NO:1 or its complementary form under medium stringency conditions;
(ii) fragile X-related epigenetic element 2 (B) [FREE2 (B)] comprising the nucleotide sequence set forth in SEQ ID NO:2 or a homolog thereof or portion or part thereof defined by having at least 80% nucleotide sequence identity to SEQ ID NO:2 or which hybridizes to SEQ ID NO:2 or its complementary form under medium stringency conditions;
(iii) fragile X-related epigenetic element 2 (C) [FREE2 (C)] comprising the nucleotide sequence set forth in SEQ ID NO:3 or a homolog thereof or portion or part thereof defined by having at least 80% nucleotide sequence identity to SEQ ID NO:3 or which hybridizes to SEQ ID NO:3 or its complementary form under medium stringency conditions;
(iv) fragile X-related epigenetic element 2 (D) [FREE2 (D)] comprising the nucleotide sequence set forth in SEQ ID NO:4 or a homolog thereof or portion or part thereof defined by having at least 80% nucleotide sequence identity to SEQ ID NO:4 or which hybridizes to SEQ ID NO:4 or its complementary form under medium stringency conditions;
(v) fragile X-related epigenetic element 2 (E) [FREE2 (E)] comprising the nucleotide sequence set forth in SEQ ID NO:5 or a homolog thereof or portion or part thereof defined by having at least 80% nucleotide sequence identity to SEQ ID NO:5 or which hybridizes to SEQ ID NO:5 or its complementary form under medium stringency conditions; and
(vi) fragile X-related epigenetic element 3 (FREE3) comprising the nucleotide sequence set forth in SEQ ID NO:6 or a homolog thereof or portion or part thereof defined by having at least 80% nucleotide sequence identity to SEQ ID NO:6 or which hybridizes to SEQ ID NO:6 or its complementary form under medium stringency conditions.

In another embodiment, the target site is within the SNRPN genetic locus in a mammalian cell including a human cell, and includes:
(i) SNRPN genetic locus (SNRPN-M) comprising the nucleotide sequence set forth in SEQ ID NO:19 or a homolog thereof or portion or part thereof defined by having at least 80% nucleotide sequence identity to SEQ ID NO:19 or which hybridizes to SEQ ID NO:19 or its complementary form under medium stringency conditions; and
(ii) SNRPN genetic locus (SNRPN-P) comprising the nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions.

In cases where the gene is methylated and silenced in affected individuals or tissues, compounds are screened in high throughput fashion in stable cell lines or individuals to identify drugs that result in demethylation and reactivation of the affected gene. Alternatively, a normal active copy of the affected gene is transfected as a transgene into cells to correct the defect. Such transgenes are introduced with modulating sequences that protect the transgene from methylation and keep it non-methylated and transcriptionally active.

In cases where the gene is non-methylated and transcriptionally active or transcriptionally over-active in affected individuals or tissues, compounds are screened in high throughput fashion in stable cell lines to identify drugs that result in methylation and silencing of the affected gene. Alternatively, a transgene encoding a double stranded RNA homologous to the affected sequences or homologs thereof, are transfected as a transgene into cells to methylate the gene, silence it and thereby correct the defect. Such double stranded RNA-encoding transgenes are introduced with modulating sequences which protect it from methylation, keep it transcriptionally active and producing double stranded RNA.

The present disclosure further enables a method for monitoring the treatment of a clinical condition associated with extent of methylation, the method comprising monitoring for a change relative to a control or a pre- and post-treatment sample in the extent of methylation within a DNA target site, the method comprising:
(i) subjecting the DNA sample to bisulfite treatment to convert non-methylated cytosine nucleotides in the DNA to uracil nucleotides;
(ii) serially diluting the DNA sample after bisulfite conversion;
(iii) amplifying and melting a portion of the bisulfite treated DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
(iv) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
(v) determining the signal strength in the DNA sample to be tested which falls within the dynamic linear range of amplification and the stable amplified product melt range;
(vi) deriving a standard curve of signal strength released as part of melt *versus* the known percentage of methylation from the second control DNA set sample, the melting temperature for the second control DNA set selected at the signal providing a selected difference between the non-methylated and methylated control samples; and
(vii) determining the percentage of methylation for the DNA sample by reading the percentage of methylation from the level of signal strength using the standard curve resolved from the second control DNA sample set.

By monitoring includes diagnosis, prognosis, pharmacoresponsiveness, pharmacosensitivity, level of disease progression or remission, improving or declining health of a subject and the like.

Disease conditions associated with abnormal methylation include but are not limited to FXS, FXTAS, FXPOI, autism, cognitive and behavioral impairment, reduced ovarian function, memory lapse, poor memory retention, a neurodevelopmental and/or neurodegenerative disorder, attention deficit disorder, mood disorder, schizophrenia, bipolar disorder, mental retardation, Klinefelter's syndrome, Turner's syndrome, aneuploidy and a modified X-chromosome. Reference to a "modified" X-chromosome includes skewed X-inactivation, inversions, deletions, duplications, hybrids and any modification leading to X-chromosome inactivation. Imprinting conditions include Prada-Willi syndrome (PWS)/Angelman syndrome (AS).

Conditions contemplated herein include pathoneurological conditions such as pathoneurodevelopmental and pathoneurodegenerative conditions as well as non-neurological conditions. Conditions and disorders contemplated herein include polyglutamine (polyQ) diseases such as Huntington's disease (HD), dentatorubropallid-oluysiantrophy (DRPLA), spinobulbar muscular atrophy or Kennedy disease (SBMA), spinocerebella ataxia Type 1 (SCA1), spinocerebella ataxia Type 2 (SCA2), spinocerebella ataxia Type 3 or Machado-Joseph disease (SCA3), spinocerebella ataxia Type 6 (SCA6), spinocerebella ataxia Type 7 (SCA7), spinocerebella ataxia Type 17 (SCA17) and non-polyQ diseases such as Fragile X syndrome (FXS), Fragile X-associated tremor or ataxia (FXTAS), Fragile XE mental retardation (FRAXE), myotonic dystrophy (DM), spinocerebella ataxia (SCA8) and spinocerebella ataxias Type 12 (SCA12). Other conditions contemplated herein include trinucleotide expansion related disorders including but not limited to Fragile X-associated primary ovary insufficiency (FXPOI) and Friedrich's ataxia (FRDA). Fragile type, folic acid type, rare 12 (FRA12A), autism (including co-morbid autism), mental retardation (MR), Klinefelter's syndrome, RNA toxicity disease, Turner's syndrome, a modified X-chromosome disorder and cognitive impairment are also contemplated. Further contemplated herein are learning and behavioral problems.

The present disclosure further contemplates a computer program and hardware which monitors the changing state, if any, of extent of methylation over time or in response to therapeutic and/or behavioral modification or for high throughput routine screening of populations. Such a computer program has important utility in monitoring disease progression, response to intervention and may guide modification of therapy or treatment. The computer program is also useful in understanding the association between increasing methylation and disease progression. The computer program is useful in routine maternal, paternal, pre-natal and post-natal testing.

The computer program executes an algorithm which determines DNA concentration and quality post-conversion for all dilutions from unknown DNA samples from a real-time amplification curve. Hence, the computer program executes an algorithm which determines the signal strength in the DNA sample to be tested which falls within the dynamic linear range of amplification and the table product melt range and reads the percentage of methylation from the level of signal strength using the standard curve resolved from the control DNA sample site of known percentage methylation and non-methylation. A program may include a learning algorithm where the relationship between the expected methylation levels, the variation and signal from the DNA standard, may be embedded in the software.

Thus, in another aspect, enabled herein is a computer program product for assessing extent of methylation of a target DNA site, the product comprising executes an algorithm which determines the signal strength in the DNA sample to be tested which falls within the dynamic linear range of amplification and the table product melt range and reads the percentage of methylation from the level of signal strength using the standard curve resolved from the control DNA sample site of known percentage methylation and non-methylation.

The computer program further comprising: means to converting index value to a code; and means to store the code in a computer readable medium and compare code to a melt standard curve.

In a related aspect, the disclosure extends to a computer program for assessing an association between extent of methylation and a clinical phenotype, wherein the computer program comprises executes an algorithm which determines the signal strength in the DNA sample to be tested which falls within the dynamic linear range of amplification and the table product melt range and reads the percentage of methylation from the level of signal strength using the standard curve resolved from the control DNA sample site of known percentage methylation and non-methylation and a machine-readable data storage medium comprising a data storage material encoded with machine-readable data, wherein the machine-readable data comprise index values associated with the features of change in methylation, together with one or more of:
(a) general phenotype or clinical manifestations in subjects;
(b) behavioral assessment criteria; and/or
(c) cognitive ability; and
means to store the code in a computer readable medium and compare code to a knowledge database to determine whether the code corresponds to a pathological condition.

The present disclosure further provides a web-based system where data on extent of methylation within a target genomic site (optionally together with clinical phenotype) are provided by a client server to a central processor which analyzes and compares to a control and optionally considers other information such as patient age, sex, weight and other medical conditions and then provides a report, such as, for example, a risk factor for disease severity or progression or status or response to treatment.

Hence, knowledge-based computer software and hardware also form part of the present disclosure.

In an embodiment, the assay of the present disclosure is used in existing or newly developed knowledge-based architecture or platforms associated with pathology services. For example, results from the assays are transmitted *via* a communications network (e.g. the internet) to a processing system in which an algorithm is stored and used to generate a predicted posterior probability value which translates to the index of disease probability which is then forwarded to an end user in the form of a diagnostic or predictive report.

The assay may, therefore, be in the form of a kit or computer-based system which comprises the reagents necessary to detect the extent of methylation modification within a genetic locus and includes computer hardware and/or software to facilitate determination and transmission of reports to a clinician.

Enabled herein is an assay which permits integration into existing or newly developed pathology architecture or platform systems. For example, a method contemplated herein allows a user to determine the status of a subject with respect to a methylation-associated pathology, the method including:
(a) receiving data in the form of extent of methylation at a selected genomic site; wherein the extent of methylation or other epigenetic modification provides a correlation to the presence, state, classification or progression of the pathology; the extent of methylation determined by the method comprising:
   (i) subjecting the DNA sample to bisulfite treatment to convert non-methylated cytosine nucleotides in the DNA to uracil nucleotides;
   (ii) amplifying and melting a portion of the DNA sample comprising the target site with selected forwarded and reverse primers incorporating a label in real-time capable of providing an identifiable signal in double stranded DNA as determined by units of signal strength as part of the amplification and releasing the label in real-time from double stranded DNA as determined by units of signal strength as part of melting, the amplification and melting occurring together with a first control sample set of known DNA concentration and a second control DNA sample set of known percentage of methylated and non-methylation DNA;
   (iii) deriving a standard curve of DNA concentration *versus* cycle threshold (Ct) from the first control DNA sample set to determine the dynamic linear range of the amplification; and determining the stable amplified product melting range on the same set of control DNA samples where the signal strength is independent of the DNA dilution which indicates that the product has reached a defined state of PCR amplification;
   (iv) determining the signal strength in the DNA sample to be tested which falls within the dynamic linear range of amplification and the stable amplified product melt range;
   (v) deriving a standard curve of signal strength released as part of melt *versus* the known percentage of methylation from the second control DNA set sample, the melting temperature for the second control DNA set selected at the signal providing a selected difference between the non-methylated and methylated control samples; and
   (vi) determining the percentage of methylation for the DNA sample by reading the percentage of methylation from the level of signal strength using the standard curve resolved from the second control DNA sample set; and then transferring the data from the user *via* a communications network;
(b) processing the subject data *via* multivariate or univariate analysis to provide a disease index value;
(c) determining the status of the subject in accordance with the results of the disease index value in comparison with predetermined values; and
(d) transferring an indication of the status of the subject to the user *via* the communications network. Reference to the multivariate or univariate analysis includes an algorithm which performs the multivariate or univariate analysis function.

Conveniently, the method generally further includes:
(a) having the user determine the data using a remote end station; and
(b) transferring the data from the end station to the base station *via* the communications network.

The base station can include first and second processing systems, in which case the method can include:
(a) transferring the data to the first processing system;
(b) transferring the data to the second processing system; and
(c) causing the first processing system to perform the multivariate analysis function to generate the disease index value.

The method may also include:
(a) transferring the results of the multivariate or univariate analysis function to the first processing system; and
(b) causing the first processing system to determine the status of the subject.

In this case, the method also includes at least one of:
(a) transferring the data between the communications network and the first processing system through a first firewall; and
(b) transferring the data between the first and the second processing systems through a second firewall.

The second processing system may be coupled to a database adapted to store predetermined data and/or the multivariate analysis and/or univariate analysis function, the method including:
(a) querying the database to obtain at least selected predetermined data or access to the multivariate or univariate analysis function from the database; and
(b) comparing the selected predetermined data to the subject data or generating a predicted probability index.

The second processing system can be coupled to a database, the method including storing the data in the database.

The aspect provides a diagnostic rule based on the application of statistical and machine learning algorithms. Such an algorithm uses the relationships between methylation profile and disease status observed in training data (with known disease status) to infer relationships which are then used to predict the status of patients with unknown status. Practitioners skilled in the art of data analysis recognize that many different forms of inferring relationships in the training data may be used without materially changing the outcome disclosure herein.

The present disclosure contemplates, therefore, the use of a knowledge base of training data comprising extent of methylation within a genetic locus from a subject with a clinical phenotype to generate an algorithm which, upon input of a second knowledge base of data comprising levels of the same biomarkers from a patient with an unknown pathology, provides an index of probability that predicts the nature of unknown pathology or response to treatment.

The term "training data" includes knowledge of the extent of methylation relative to a control. A "control" includes a comparison to levels in a healthy subject devoid of a pathology or is cured of the condition or may be a statistically determined level based on trials.

Whilst the assay enabled herein comprises the various steps, in an embodiment, the assay consists essentially of the steps. In another embodiment, the assays consists of the steps.

### EXAMPLES

Aspects disclosed herein are now further described by the following nonlimiting Examples.

### Materials and Methods

### Data Source

A retrospective cohort study of extent of methylation is conducted on venous blood DNA and newborn blood spots and correlated with clinical assessment.

An additional patient cohort comprised of 258 male and 427 female samples collected from birth to 82 years of age collected as part of previous studies (Godler *et al.* (2012) *supra;* Inaba et al. (2013) Genet Med 15:290-298; Loesch et al. (2012) Glin Genet 82:88-92; Tassone et al. (2000) Am J Med Genet 97:195-203). Of these, formal cognitive assessments were performed on 23 PM female, 21 FM females and 3 'high functioning' unmethylated FM (UFM) males (with Full Scale IQ - FSIQ between 71 and 81) determined using the Wechsler intelligence test appropriate for chronological age as described in previous publications (Godler *et al.* (2012) *supra;* Loesch *et al.* (2012) *supra;* Tassone et al. (2000) American Journal of Medical Genetics 94:232-236).

A further patient cohort comprised of 433 DNA from venous blood, 209 newborn and adult blood spots and 100 saliva DNA samples, with the participants' age range between birth and 82 years. Of these, 100 samples were from individuals with a sex chromosome aneuploidy, 194 were from FM carriers (pure and size/methylation mosaic), 140 were from PM carriers, and 308 were from controls. EpiTYPER system analysis of all of the samples included in this study has been performed as part of previous studies (Godler *et al.* (2013) *supra;* Inaba *et al.* (2013) *supra*). EpiTYPER is a tool for the determination of DNA methylation that uses base specific cleavage and matrix-assisted later desorption/ionization time-of flight mass spectrometry. This cohort was used to analyze the sex chromosome in aneuploidy samples, the ability of MS-QMA to detect skewed X-chromosome inactivation, and the comparison between MS-QMA and the EpiTYPER system in this large cohort which includes sex chromosome aneuploidy

### Neuropsychological assessments

For the individuals whose archival clinical data and DNA samples were available, the phenotype was assessed using the Autism Diagnostic Observation Schedule-Generic (ADOS-G) [Loesch et al. (2007) Neuro Sci Biobeta Rev 31:315-326] and Wechsler intelligence test appropriate for chronological age: WPPSI-III for ages less than 6 (Wechsler (2002) Wechsler Protocol and Primary School of Intelligence, The Psychological Corporation, San Antonio, Texas, USA), WISC-III for ages between 6 and 16 years and WAIS-III for ages greater than 16 years (Wechsler (1997) Orlando, the Psychological Corporation). The cognitive status of these individuals has been described in Wechsler (1991) Wechsler Intelligence School for Children, 3rd Ed. (WISC-III), The Psychological Corporation, San Antonio, Texas, USA elsewhere (Godler *et al.* (2012) *supra;* Chanchaiya et al. (2010) Hum Genet 128:539-548; Loesch *et al.* (2007) *supra;* Dissanayake et al. (2009) J Child Psychol Psychiatry 50:290-299) and this information is used for the specificity and sensitivity assessments, determination of predictive value and correlation analysis between molecular and clinical measures.

### Newborn Guthrie spot samples:

The newborn blood spots were assayed. The methylation results for 50 FM males and females in whole blood (greater than 4 years of age) are compared to the results in blood spots taken at birth. The newborn blood spot MS-QMA results are also compared to neuropsychological assessments in these individuals. FREE2 methylation results determined using MALDI-TOF MS are available (Godler *et al.* (2011) *supra;* Godler (2012) *supra;* Godler et al. (2013) Hum Mol Genet*,* In Press).

The MS-QMA protocol developed in accordance with the present disclosure was based on a combined real-time PCR standard curve method and High Resolution Melt (HRM) analysis performed on bisulfite converted DNA as described in Figure 1. The input was either one 3mm dried blood spot, or DNA extracted from 0.3 to 1 ml of venous blood, with DNA or blood spot lysate extracted and CGG repeat sized as previously described (Inaba *et al.* (2013) *supra;* Loesch et al. (2009) Am J Med Genet A 149A:2306-2310; Khaniani *et al.* (2008) *supra;* Tassone *et al.* (2008) *supra;* Christie et al. (2013) Am J Med Genet A 161A:301-311). For 19 FM females, the *FMR1* activation ratio had been previously determined using methylation sensitive Southern blot as described in (de Vries et al. (1996) Am J Hum Genet 58:1025-1032). FMRP immunoreactivity in venous blood smears was previously assessed in 18 of these FM females and was expressed as the percentage of lymphocytes staining positive for the protein (Loesch et al. (2002) Am J Med Genet 107:136-142; Tassone et al. (1999) Am J Med Genet 84:250-261). FREE2 methylation was also assessed in the same samples using the Sequenom EpiTYPER system, as previously described (Godler *et al.* (2010) *supra*).

For the detection of skewed X-chromosome inactivation, 3 to 10 ml venous blood were collected in EDTA-treated tubes went through a DNA extracted using NucleoSpin (Registered Trade Mark) Tissue genomic DNA extraction kit, as per manufacturer's instructions (MACHEREY-NAGEL GmbH & Co. KG, Düren, Germany); 2 ml saliva were collected using the Oragene (Registered Trade mark) DNA Self-Collection Kit (DNA Genotek Inc., Ottawa, Canada) and isolated as per manufacturer's instructions. Wallac dried blood spot puncher (Perkin Elmer, MA, USA) was used to obtain one or two three-millimeter punches from each spot disk into 96-well plates. These were then incubated in 55 µl of salt lysis buffer as previously described (Inaba *et al.* (2012) *supra*) for cell lysis, degradation of proteins and release of the DNA into the solution, and stored at -20°C until analysis.

### MS-QMA conditions:

Table 3 provides the real-time PCR/HRM conditions and primer sequences for the FREE2 (A) region.

**TABLE 3**

| ***MS-QMA conditions*** | | | | |
|---|---|---|---|---|
| Real-time PCR/High resolution melting condition | Size (b) | Distance 3' of CGG (b): | Primer sequence | SEQ ID NO: |
| (I) 95 °C for 10 min. (II) 40 cycles of: 95°C for 30s, 65°C for 1 min. | 161 | 117 | Fw: 5'-AAGGTATTTGGTTTTAGGGTAGGTTTT -3' | 7 |
| (III) 95 °C for 30 sec, 65°C for 1 min, 95 °C for 30 sec, 65°C for 15 sec. | | | Rv: 5'- AAATCCAATCCTTCCCTCCCA -3' | 8 |

### Methylation Specific Quantitative Melt Analysis (MS-QMA)

### Bisulfite conversion and sample preparation

For newborn blood spots, one or two three-millimeter punches from each spot disk were collected into 96-well plates using the Wallac DBS Puncher (Perkin Elmer, MA, USA) and stored at room temperature until analysis. Each punch or set of punches was incubated in 55 µl of salt lysis buffer as previously described (Inaba *et al.* (2013) *supra*). The supernatant was then transferred to a fresh 96-well plate. The extracts were then treated with sodium bisulfite using EZ-96 DNA Methylation-Gold (Trade Mark) (Zymo Research, Irvine, CA).

### Real-time PCR standard curve method and High Resolution Melt Analysis (HRM)

The 96 converted samples (with 3 controls and 93 unknown samples per plate) were serially diluted 4 times post-conversion (Figure 1). The four 96 well plates were then transferred into a 384 well format for real-time PCR analysis utilizing MeltDoctor (Trade Mark) high-resolution melt reagents in 10ul reactions as per manufacturer's instructions (Life technologies, Foster City, CA). For Real-time PCR, a unique primer set was used that targets specific CpG sites within the FREE2 region that were previously shown to be most significantly associated with cognitive impairment in FM females (Godler *et al.* (2012) *supra*). The annealing temperature for the thermal cycling protocol was 65°C for 40 cycles. The ViiA (Trade Mark) 7 Real-Time PCR System (Life technologies, Foster City, CA) was then used to initially measure the rate of dye incorporation into double stranded DNA in order to quantify DNA concentration of the unknown samples using the relative standard curve method (Figure 1). The dynamic linear range (usually between 0.05-10 ng/µl) was determined from the standard curve using a series of doubling dilutions of a converted DNA standard from a control lymphoblast cell line during each run. To progress to the next stage of the analysis the unknown samples had to be within this dynamic linear range (Figure 1 in wells on the 384 plate in pink). The high resolution melt step followed the real-time PCR without additional sample handling/ sample transfer. In the temperature range of 74 °C and 82°C the products from methylated FREE2 sequence separated into single strands at higher temperatures than those from unmethylated FREE2. The HRM Software Module for ViiA (Trade Mark) 7 System was then used to plot the rate of PCR product separation to single strands at different temperatures with the difference in fluorescence first converted to Aligned Fluorescence Units (AFU) at the temperature that provided the greatest separation between methylated and unmethylated sequences, and then converted to the methylation ratio (MR) from the methylation curve, as described in Figure 1. It will be understood that the terms "percentage of methylation" and "methylation ratio" or "MR" are used herein interchangeably. The percentage methylation is calculated by multiplying the methylation ratio by 100, i.e. MR x 100 = % methylation.

### Data analysis

Testing for normality distribution of the methylation ratio was conducted using Shaprico-Wilk test at significant level p=0.05. Depending on results of this test for the inter-group comparisons, either two-sample t-test for the means was used, if the data were normally distributed, or nonparametric Mann-Whitney test for median was used, if the data were not normally distributed. Individuals were also classified with FSIQ, VIQ and PIQ>70 as negative and FSIQ, VIQ and PIQ<70 as positive for FM females. Males were classified individuals with FM alleles recruited through the developmental delay/ASD referrals for FXS testing as positive, and all other male samples as negative. The receiver operating characteristic (ROC) curve was then used to evaluate ability of MS-QMA MR to classify the positive and negative classes. Area under the ROC curve (AUC) computed using predicted probabilities from logistic regression was used as the summary measure of diagnostic accuracy and Youden Index (Youden (1950) Cancer 3:32-35) was used to determine the optimal threshold (cut-off point) for MS-QMA analysis.

The relationship between MS-QMA MR and each outcome variable including cognitive scores and other molecular measures were assessed using simple linear regression analysis. All analyses were conducted using RMS, DiagnosisMed and the publicly available R statistical computing package (Godler *et al.* (2012) *supra;* R Foundation for Statistical Computing (2007) http:/www.r-project.org/(Accessed February 2009)).

### CGG sizing, methylation using Southern blot and SRY real-time PCR

For venous blood and saliva DNA, the processing of DNA samples and assessment of CGG repeat size (with precision of +/- one repeat) was conducted using a fully validated PCR amplification assay (Loesch *et al.* (2009) *supra;* Khaniani *et al.* (2008) *supra*). CGG repeat sizing and methylation of the *FMR1* CpG island restriction sites of all samples greater than 55 repeats was also performed using a methylation sensitive Southern Blot procedure with appropriate normal and abnormal controls, as previously described (Tassone *et al.* (2008) *supra*). FREE2 methylation analysis using the Sequenom EpiTYPER system for each sample was performed in quadruplicate, giving four separate methylation output ratios (MOR), which were averaged to take account for technical variation resulting from bisulfite conversion, PCR and mass cleave reactions, as previously described (Godler *et al.* (2010) *supra;* Khaniani *et al.* (2008) *supra;* Tassone *et al.* (2008) *supra*). For newborn and dried blood spots, the CGG sizing was performed using the standard PCR amplification assay (Loesch *et al.* (2009) *supra;* Khaniani *et al.* (2008) *supra*) and triple primed PCR, as previously described. Of the 209 newborn and adult dried blood spots, 160 had CGG sizing performed as part of the previous study (Christie *et al.* (2013) *supra*). For sex chromosome aneuploidy samples and control males and females, the SRY copy number was determined using the real-time PCR relative standard curve method, normalized to β-globin, as described (Inaba *et al.* (2012) *supra*).

### EXAMPLE 1

### Methylation Specific-Quantitative Melt Analysis (MS-QMA) protocol

### Bisulfite conversion and sample preparation:

The protocol is based on combined real-time PCR standard curve method and High Resolution Melt (HRM) Analysis of bisulfite converted DNA and is referred to as methylation specific-quantitative melt analysis (MS-QMA). The required input is either a 3mm dried blood spot or DNA extracted from 0.3 to 1 ml of venous blood.

For newborn blood spots, one or two three-millimeter punches from each spot disk are collected into 96-well plates using the Wallac DBS Puncher (Perkin Elmer, MA, USA) and stored at room temperature until analysis. Each punch or set of punches is incubated in 55 µl of salt hybridization buffer for 15 minutes at 98°C in a heat block for cell lysis, degradation of proteins and release of the DNA into the solution. After boiling, the samples are centrifuged and the supernatant transferred to a fresh 96-well plate. This extract is then treated with sodium bisulfite as previously described for venous blood DNA (Inaba *et al.* (2012) *supra*).

Bisulfite conversion is performed as per manufacturer's instructions (Zymo Research, Irvine, CA) using 96 well format Z-96 DNA Methylation-Gold (Trade Mark) MagPrep kit (less than 3 hours). The Z-96 DNA Methylation-Gold (Trade Mark) MagPrep kit integrates DNA denaturation and bisulfite conversion processes into one-step coupled to a magnetic bead based clean-up for high-throughput methylation analysis using liquid handling robotics. EZ-96 DNA Methylation-Gold (Trade Mark) [Zymo Research, Irvine, CA] is an alternative conversion kit that does not utilize magnetic beads, but may be also used.

### Real-time PCR standard curve method:

The 96 converted samples (which include 3 controls and 93 unknown samples per plate) are serially diluted 4 times post conversion (Figure 1). The four 96 well plates are then transferred into a 384 well format for real-time PCR analysis utilizing MeltDoctor (Trade Mark) high-resolution melt reagents in 10 µl reactions as per manufacturer's instructions (Life technologies, Foster City, CA). For real-time PCR, unique primer sets which target specific CpG sites within the FREE2 (A) region are used (SEQ ID NOs:7 and 8) which have been shown to be significantly associated with cognitive impairment in FM females (Godler (2012) *supra*). The annealing temperature for the thermal cycling protocol is 65°C for 40 cycles. The ViiA (Trade Mark) 7 real-time PCR System (Life technologies, Foster City, CA) is used to initially measure the rate of dye incorporation into double stranded DNA to quantify DNA concentration of the unknown samples. This is done using a standard curve of serially diluted converted DNA of known concentrations (Figure 1). For the data not to be discarded from the methylation analysis steps following real-time PCR, Ct values for the unknown sample dilutions have to be within the dynamic linear range (Figure 1) in wells on the 384 plate), usually between 0.05-10 ng/µl. The dynamic linear range is generally performed on a series of doubling dilutions of a converted DNA standard from a control lymphoblast cell line.

### High Resolution Melt Analysis (HRM):

Products from methylated FREE2 (A) sequence separate into single strands at higher temperatures than those from non-methylated FREE2 (A), between 74°C and 82°C. As strands separate the MeltDoctor (Trade Mark), dye is released and is detected by the system. The HRM Software Module for ViiA (Trade Mark) 7 System is then used to plot the rate of PCR product separation to single strands at different temperatures. HRM step follows real-time PCR in a closed well format (no additional sample handling/sample transfer). This difference in fluorescence is quantified using a computer algorithm. Aligned Fluorescence Units (AFU) are extracted by the algorithm at the temperature that provides the greatest separation between methylated and non-methylated sequences of control methylated and non-methylated standards (the HRM methylation standard curve in Figure 1(i)). This point equates to the lowest temperature at which all double stranded DNA that originated from the non-methylated strands has completely melted emitting no new fluorescence, while the double stranded DNA from the methylated strands is actively emitting fluorescence due to strand separation and dye release. AFU's of the real-time PCR standard curve are then used to determine the quality control range (Figure 1). The AFU values for the unknown samples with high DNA quality post conversion need to be within the range of dilutions of the dynamic linear range that provide AFU output that does not change between dilutions.

The data analysis algorithm extrapolates methylation % for bisulfite dilutions from the HRM methylation standard curve for the unknown samples when: (i) the DNA concentration post conversion is within the real-time PCR dynamic linear range; and (ii) they are within the AFU quality control range. The HRM methylation standard curve is essential for conversion of AFU to methylation %, and is co-run on each 384 well plate with unknowns. This curve is plotted from AFU values over expected methylation of spiked lymphoblast DNA samples from a control male with completely non-methylated FREE2 (A) and a FXS male with 100% methylated FREE2 (A). The AFU values for the unknown samples are plotted against an HRM methylation standard curve (Figure 1), where AFU is converted to methylation percentage.

For an example of data analysis, the following filter conditions are used: (1) apply DNA concentration threshold (e.g. if DNA conc1 is <0.5 ng/µl, then remove AF1 from the data set; (2) remove outlier (up to 2 AFs) [e.g. if absolute value (mean of AF1-4)-AF2) is >6.2] (this value is obtained from two time standard deviation of the linear dynamic range controls), then remove AF2 from the data set; and (3) N ≥ 2 (e.g. if 2N:AF3 and AF4 [AF1 and AF2 are removed from the data sets]). See Figure 1.

### EXAMPLE 2

### Development and Technical Validation of the FREE2 MS-QMA Assay

To assess the intra run variation and the ability of the assay to predict the expected methylation ratio (MR) 16 different spiking experiments were performed. A temperature of 78°C was identified as the lowest temperature at which all unmethylated alleles are completely melted, at which point no further fluorescence is emitted. At this temperature, the 100% methylated alleles are actively melting, and emitting fluorescence. A reliable method with the lowest inter and intra run variation (two standard deviations) and the lowest detection limit (LOD of 0.02 MR) used the AFUs from the aligned fluorescence curves at 78°C. This produced a correlation coefficient of 0.998 for the High Resolution Melt (HRM) standard curve representing the relationship between AFU at 78°C and the expected methylation ratio in the spiked samples.

MS-QMA analysis was performed in Figure 2(ii) in 138 females previously examined for the relationship between FREE2 methylation and cognitive impairment using the MALDI-TOF MS based EpiTYPER system (Godler *et al.* (2012) *supra*) [Figure 3(i) and Table 4) and 288 females (Figure 3(ii)) for the majority of whom no formal cognitive assessment performed (Figure 3(ii)). It was found that assay sensitivity and specificity for FM females with IQ<70 in the sub-group of 138 participants varied depending on the type of IQ measure. The optimal threshold of 0.39 MR that provided sensitivity of ∼95% and specificity 100% was for detection of FM females with VIQ<70 (Table 5A). The threshold of 0.37 MR provided the highest sensitivity and specificity of ∼92% and 100%, respectively for both the FSIQ and PIQ assessments in FM females.

Notably, there was also some overlap between controls and PM and low functioning FM females at the lower threshold of 0.37 for FSIQ and PIQ (Figure 3(ii)) suggesting that the VIQ threshold of 0.39 should be used instead. At 0.39 MR there were no female controls (CGG<40) with MR values above this threshold. When applied to a group 288 females (Figure 3(ii)), approximately 50% of all FM females had an MR above 0.39 MR. In contrast, only one PM female had MR above the 0.39 threshold, and only two PM females had MR of 0.39. This equates to 0% of controls and ∼3% of PM females at or slightly above the VIQ<70 threshold. These 3% of PM females and 19% of FM females had MS-QMA output within a borderline range of 0.39 to 0.41 MR (Figures 2(ii) and 3(ii)) suggesting that, CGG sizing should be used on all samples in this range to resolve any potential overlap between allele classes.

It is also important to note that in this borderline range (0.39 to 0.41 MR) there was overlap between VIQ<70 and >70 for a proportion of FM females. However, for all PM and FM samples above and below this borderline range MS-QMA VIQ<70 sensitivity, specificity, positive and negative predictive values were 100% (Figure 2(ii) and Figure 13). Each FM female within the borderline range (0.39 to 0.41 MR) had 86% probability for VIQ>70 (high functioning). Furthermore all PM females within this range had 100% probability for VIQ>70, suggesting that in relation to verbal cognitive impairment the MS-QMA results should be reported as risk or probabilities depending where the MR values fall.

Intergroup comparison of MS-QMA in venous blood DNA from 124 males showed that the median MR was significantly higher for FM males (identified through investigation of developmental delay/ASD), FM methylation mosaics and PM/FM size mosaics than for male controls, PM males and 'high functioning' UFM males with FSIQ, VIQ and PIQ >70 (Figure 3(ii)). A threshold of 0.1 MR provided sensitivity and specificity approaching 100% for FM males and FM methylation mosaics, while for PM/FM size mosaics the sensitivity was 88% and specificity was 100% at this threshold (Table 5).

### EXAMPLE 3

### Follow-up testing of identified cryptic FM individuals using reference methods

To confirm the cryptic FM status, the samples identified as positive using MS-QMA are re-tested using CGG sizing PCR, methylation-sensitive Southern blot and MALDI-TOF MS FREE2 analysis as described in earlier publications (Godler *et al.* (2010) *supra;* Godler *et al.* (2012) *supra*). Briefly, processing of 'positive' DNA samples and the assessment of the size of CGG repeat from the extracted DNA (with precision of +/- one repeat) are conducted using a fully validated PCR amplification assay (Khaniani et al. (2008) Mol Cytogenet 1:5). CGG repeat sizing and methylation of the FMR1 CpG island restriction sites of all positive samples are also performed using a methylation sensitive Southern Blot procedure with appropriate normal and abnormal controls, as previously described (Godler *et al.* (2010) *supra;* Tassone et al. (2008) J Mol Diagn 10:43-49). Briefly, EcoRI and NruI digestion is performed on 7 to 9 µg of DNA. The *FMR1* alleles are detected using the StB12.3 probe, labeled with Dig-11-dUTP by PCR (PCR Dig Synthesis kit; Roche Diagnostics). Southern blot methylation for the expanded *FMR1* alleles are determined as previously described (Godler *et al.* (2010) *supra*) with alleles classified as either non-methylated, partially methylated or fully methylated. Alleles at CGG sizes greater than 150 repeats that are methylated by Southern blot are classified as FM; alleles between 55 and 200 repeats that are non-methylated by Southern blot are classified as PM. FREE2 (A) methylation analysis using MALDI-TOF MS are assessed in the same samples using the Sequenom EpiTYPER system, as previously described (Godler *et al.* (2010) *supra*). FREE2 (A) methylation analysis for each sample are performed in duplicate, giving two separate methylation output ratios (MOR), averaged to take account of technical variation resulting from bisulfite conversion, PCR and mass cleave reactions.

### EXAMPLE 4

### Advantages of FREE2 (A) MS-QMA in FXS diagnostics

Standard laboratory testing for FXS falls into the following categories. The first category applies to diagnostic testing of individuals with intellectual disability or ASD of unknown etiology, where a positive result leads to testing of other family members, in whom the risk of carrier status is high. The second category applies to prenatal testing in known carrier pregnancies. The third category is the population screening of newborns. Earlier treatment intervention, identification of probands pointing to high risk relatives and provision of reproductive counseling are strong arguments in favor of newborn screening.

One major impediment is the limited suitability of current test methods which are a combination sizing of small and large CGG repeat expansions by PCR and Southern blot testing, respectively (Tassone *et al.* (2008) *supra*). Several PCR based approaches have been developed to amplify PM and small FM alleles, however, they do not provide information on the gene's methylation status (Tassone *et al.* (2008) *supra;* Filipovic-Sadic et al. Clin Chem 56(3):399-408; Hantash et al. Genet Med 12(3):162-173; Dodds et al. (2009) Anal Chem). These short-comings necessitate further testing using methylation sensitive Southern blot. However, the use of Southern blot for any type of large scale testing is primarily restricted by low throughput, cost and DNA quality and quantity limitations.

A PCR based test has been developed that determines CGG length up to FM size and examines methylation of two *HpaII* sites on either side of the CGG expansion (Chen et al. (2011) Genet Med 13:528-538). Whilst it has been suggested that use of this test avoids reflexing to methylation sensitive Southern blot because it provides methylation analysis as well as CGG size, the CpG sites examined by this method are different from those examined by methylation sensitive Southern blot and provide different results particularly in PM females compared to the reference method. Importantly, methylation of these *HpaII* sites has not been as yet related to any clinical phenotype in *FMR1* expansion carrier females, thus reflexing to methylation sensitive Southern blot is still required for carrier females.

Furthermore, all of the above PCR tests detect GZ and PM carriers who are highly prevalent in the general population (for males 1 in ∼30 for GZ and 1 in ∼700 for PM carriers; for females 1 in ∼15 GZ and 1 in ∼250 for PM carriers) [Sherman (2000) Am J Med Genet 97:189-194; Bretherick et al. (2005) Hum Genet 117:376-382]. These small expansions do not cause FXS but PM carriers have a high risk of transmitting them in an expanded form. Furthermore, both PM and GZ alleles have been associated with elevated FMR1 mRNA (Kenneson et al. (2001) Hum Mol Genet 10:1449-1454; Loesch et al. (2007) J Med Genet 44:200-204) and related to increased risk of developing FXPOI, while PM alleles have been also linked to FXTAS (Bretherick *et al.* (2005) *supra;* Hagerman *et al.* (2001) *supra;* Greco et al. (2002) Brain 125:1760-1772; Allingham-Hawkins et al. (1999) Am J Med Genet 83:322-325; Sullivan et al. (2005) Hum Reprod 20:402-412; Bodega et al. (2006) Hum Reprod 21:952-957). Thus, a test that detects GZ and PM alleles may inadvertently turn a screen for FXS into a predictive assay for a late onset disorder.

This is addressed by the MALDI-TOF mass spectrometry test for FREE2 (A) methylated markers. The method is advantageous over most other MS-PCR based assays (Boyd et al. ((2006) Anal Biochem 354:266-273; Zhou et al. (2006) Clin Chem 52:1492-1500) and enzyme based MS-MLPA methods (Nygren et al. (2008) J Mol Diagn 10:496-501) developed to examine methylation of the 'classical' FMR1 CpG island, as MALDI-TOF MS can be used to rapidly examine large stretches of DNA for methylation. In contrast, most PCR, or MS-MLPA and enzyme based MS-MLPA methods are restricted to a few sites that are less biologically significant and/or more heavily affected by skewed X-inactivation (Boyd *et al.* ((2006) *supra;* Nygren *et al.* (2008) *supra*). This point is clearly evident from another high-throughput MS-PCR assay recently developed by another group, with proposed applications in population screening (Coffee *et al.* (2009) *supra*), where although this assay has 100% specificity and 100% sensitivity for detecting FMR1 methylation in males, in females it cannot not reliably detect excess FMR1 methylation, and its levels do not correlate with intellectual disability (Coffee *et al.* (2009) *supra*). In contrast, FREE2 (A) analysis using MALD-TOF MS is suitable for both males and female samples, and most importantly, as demonstrated (Godler *et al.* (2010) *supra;* Godler *et al.* (2010) *supra;* Inaba *et al.* (2012) *supra*), it reflects the level of neurodevelopmental changes in carriers of expanded FMR1 alleles. Whilst FREE2 (A) MALD-TOF MS analysis showed high sensitivity and specificity for FXS, it could not differentiate between GZ and PM alleles and controls - a technical drawback that could also be a major advantage of the MALDI-TOF MS over the existing methodologies particularly in newborn screening applications.

The main limitation of MALDI-TOF MS method is that it requires expensive, specialized equipment, which is not commonly used in diagnostic laboratories and uses a multi-step process with relatively high reagent costs. The MS-QMA methodology described herein, however, is more suitable for widespread and high throughput use since it does not require expensive equipment or specialized training.

The MS-QMA protocol requires virtually no initial sample processing such as measurement of DNA concentration and purity using spectrophotometry or capillary electrophoresis for fragmentation/quality. Either one 3mm dried blood spot or DNA extracted from 0.3 to 1 ml of venous blood is the required input. Other advantages of MS-QMA is that it has higher inter-run reproducibility (<5% variation) with the lower limit of detection of 5% methylation than MALDI-TOF MS. This is evidenced from Figure 1 HRM methylation standard curve (inside the green box) where male control DNA has been spiked at different ratios with 100% methylated FXS male DNA. The HRM methylation standard curve represents the relationship between the mean aligned fluorescence (AFU) at 78°C of six separate DNA spiking experiments determined using MS-QMA (X axis) and the expected methylation ratio in male control DNA spiked with methylated FXS male DNA (Y axis). The error bars represent one standard deviation between runs for each point, with the coefficient of correlation approaching 1 demonstrating that AFU is highly predictive of expected % methylation of the FREE2 (A) region.

Furthermore, MS-QMA requires ∼1000 fold less DNA quantity than Southern blot, ∼100 fold less DNA than the MS-PCR established for FXS testing in males (Zhou *et al.* (2006) *supra*), and ∼10 fold less DNA than MALDI-TOF MS. It is less sensitive to DNA quality issues than Southern blot (as it was used to identify FM/FXS samples that failed when analyzed *via* Southern blot). The MS-QMA analysis is far more rapid than Southern blot (<2 days vs. 1 to 2 weeks for Southern). To maximize productivity the process is further automated by coupling the real-time PCR machine to the Applied Biosystems (Registered Trade Mark) Twister (Registered Trade Mark) II Robot, which can automatically load up to six 384 plates in 12 hours. This increases MS-QMA throughput to ∼500 samples per 24 hours.

In addition, FREE2 (A) analysis using both MS-QMA and MALDI-TOF MS identify cryptic FXS individuals (mosaics with normal and FM size expansions) missed using standard FXS testing. This could become a major advantage of FREE2 (A) analysis over the current FXS testing protocol, once the prevalence estimates are determined for these individuals in the ASD and cognitive impairment populations and provides a strong argument for inclusion of FREE2 (A) methylation analysis as a first line test for all ASD and developmental delay cases of unknown cause referred for molecular testing worldwide.

Furthermore, FREE2 (A) methylation analysis has been conducted using both MALDI-TOF MS (Inaba *et al.* (2012) *supra*) and MS-QMA (Figure 3(i)) and are both robust for analysis of methylation from both freshly prepared and archival newborn dried blood spots. MS-QMA test has the potential to become the most suitable method for widespread testing due to its specificity for the individuals that are affected with FXS, and thus it would identify the individuals that would benefit from early intervention.

### EXAMPLE 5

### Sensitivity and specificity of the MS-QMA test for the type and severity of specific cognitive impairment using DNA from blood

Sensitivity and specificity are determined for MS-QMA (Figure 2(i)) and MALDI-TOF MS (Figure 3(i)) FREE2 (A) methylation analysis in *FMR1* expansion carriers and controls. The sensitivity is considered to be a measure of the probability of correctly identifying the presence of specific cognitive and behavioral deficits as determined using neuropsychological assessments, and the specificity - a measure of the probability of correctly identifying a person as not being affected by cognitive and behavioral deficits. The individuals are considered as affected with: (i) ASD if the ADOS-G score is >17; (ii) cognitive impairment if IQ test score is <70; or cognitive indexes or cognitive sub-scores are <7.5. Comparisons between the median methylation for blood DNA between PM and FM groups and healthy controls are also conducted using the nonparametric Mann-Whitney two-sample test. ANOVA or nonparametric Kruskal-Wallis rank test are also used to compare the differences in the levels of methylation determined using MALDI-TOF MS and MS-QMA, with the neuropsychological measures between the FM (n=111), PM (n=186) and age matched control (n=90) groups.

**TABLE 4**

| *Comparison of FREE2 MS-QMA median methylation ratios for FM females re-classified as negative for FSIQ, VIQ and PIQ values >70 and as positive for FSIQ, VIQ and PIQ values <70, with the comparisons conducted using the non-parametric Mann-Whitney two- sample test.* | | | | | | | |
|---|---|---|---|---|---|---|---|
| | **n₁** | **n₂** | **med₁** | **med₂** | **IQR₁** | **IQR₂** | **P-value** |
| **FSIQ** | 7 | 13 | 0.45 | 0.38 | 0.11 | 0.04 | **0.0068**** |
| **PIQ** | 8 | 11 | 0.43 | 0.38 | 0.1 | 0.04 | **0.0101*** |
| **VIQ** | 6 | 13 | 0.47 | 0.38 | 0.07 | 0.03 | **0.0024**** |

| | | | | | | | |
|---|---|---|---|---|---|---|---|
| Note: **P* values<0.05 are highlighted in bold; ***P* values<0.01 are highlighted in bold; sample size (n); median (med); interquartile range (IQR);. **n₁** represents numbers of positive (IQ<70), and **n₂** represents number of negative (IQ>70). | | | | | | | |

### EXAMPLE 6

### Relationship between FREE2 (A) MS-QMA results and the severity of cognitive and behavioral impairments

Nonparametric regression is used to determine whether there is a non-linear relationship between each predictor of methylation levels (determined using MALDI-TOF MS and MS-QMA) in PM and FM carriers, and cognitive/behavioral outcome measures in the subgroup of 111 FM and the combined sample of 297 carriers. Parametric regression and correlation analysis is then used to conduct analysis between a pair of these variables. In the multivariate analysis stepwise regression using Bayesian information criteria and penalized regression method is used to select the best methylation analysis approach that best predicts the outcome. All final models are validated internally using cross-validation and bootstrap. Finally, ANOVA or nonparametric Kruskal-Wallis rank test is used to compare the differences in the levels of methylation, determined using MALDI-TOF MS and MS-QMA, and the neuropsychological measures between FM ASD⁺ and ASD⁻ groups.

Furthermore, nonparametric regression is used to determine whether there is a non-linear relationship between each predictor of FREE2 (A) methylation levels in PM and FM groups in blood, and cognitive/behavioural outcome for the FMR1 expansion carriers as detailed in (Godler *et al.* (2012) *supra*).

The level of FREE2 methylation in venous blood DNA determined using MS-QMA correlated significantly with Wechsler Adult Intelligence Scale (Godler et al. (2010) Genet Med 12:595) FSIQ, VIQ and PIQ and most WAIS subtest scores (Table 6). The epigenotype-phenotype correlations were most evident for the relationships of MS-QMA MR with VIQ, and the Arithmetic and Information subtests (p<0.01). However, in PM females these subscale and subtest scores did not show significant correlations. In these samples MS-QMA MR was also significantly correlated with FREE2 CpG sites examined using the EpiTYPER system (Table 7), with p<0.0001 for all of these sites. Of the other molecular parameters available through the previous studies for these samples (Godler *et al.* (2010) *supra;* Godler *et al.* (2011) *supra*), FREE2 MS-QMA also showed significant correlation (p=0.018) with *FMR1* activation ratio determined using methylation sensitive Southern blot, which represents the methylation status of the *FMR1* CpG island on normal size alleles in these females. Correlation with FMRP levels was of borderline significance (p=0.058), with no significant correlation observed with CGG size in the FM range of these females (Table 7).

**TABLE 5**

| *Sensitivity and specificity for MS-QMA MR in venous blood DNA: (A) FM females with FSIQ, VIQ and PIQ<70 determined using the ROC analysis in a sample of 94 controls, 23 PM, and 21 FM females. Sensitivity and specificity were calculated based using the Youden index optimal cut off value which for FSIQ and PIQ was 0.37, and for VIQ was 0.39; (B) 52 FM males indentified through investigation of developmental delay*/*ASD, 4 FM methylation mosaics and 17 PM*/*FM size mosaics in a sample of 124 males including 20 male controls, 28 PM and 3 'high functioning' FM males with FSIQ, VIQ and PIQ > 70; determined to be unmethylated using methylation sensitive Southern blot identified through cascade testing.* | | | | | | |
|---|---|---|---|---|---|---|
| **Variable** | **AUC*** | **SE** | **95% CI** | ****Optimal cut off value (Youden index) for MS-QMA MR** | **Sensitivity+** | **Specificity+** |
| **(A) Females** | | | | | | |
| **FSIQ** | 0.983 | 0.013 | 0.946-0.998 | 0.37 | 1.000 | 0.911 |
| **VIQ** | 0.993 | 0.007 | 0.958-0.999 | 0.39 | 1.000 | 0.943 |
| **PIQ** | 0.983 | 0.011 | 0.946-0.998 | 0.37 | 1.000 | 0.918 |

| **(B) Males** | | | | | | |
|---|---|---|---|---|---|---|
| **FM males with ASD** | 1.000 | 0.000 | 0.965-1.000 | 0.10 | 1.000 | 1.000 |
| **FM methylation mosaics** | 1.000 | 0.000 | 0.935-1.000 | 0.10 | 1.000 | 1.000 |
| **PM/FM size mosaics** | 0.959 | 0.033 | 0.876-0.991 | 0.10 | 0.8824 | 1.000 |

| | | | | | | |
|---|---|---|---|---|---|---|
| *AUC indicates Area under the curve; **Specificity and methylation cut off value were calculated at 100% sensitivity; + Sensitivity and specificity were calculated base on the Youden index optimal cut-off value. | | | | | | |

**TABLE 6**

| *Relationships between FREE2 MS-QMA MR (predictor) with outcome variables WAIS FSIQ, subscales and subtests using simple linear regression* | | | | |
|---|---|---|---|---|
| | N | Estimated coeff. | SE | P |
| **Full Scale IQ (FSIQ)** | 20 | -236.7 | 64.99 | **0.002**** |
| **Verbal IQ (VIQ)** | 19 | -239.0 | 64.64 | **0.002**** |
| **Performance IQ (IQ)** | 19 | -184.5 | 66.59 | **0.013*** |
| **Verbal Comprehension Index (VCI)** | 15 | -197.9 | 72.95 | **0.018*** |
| **Perceptual Organization Index (POI)** | 15 | -161.5 | 80.34 | 0.066 |
| **Working Memory Index (WMI)** | 9 | -201.5 | 72.57 | **0.027*** |
| **Processing Speed Index (PSI)** | 15 | -115.0 | 52.42 | **0.047*** |
| **Picture Completion** | 17 | -40.43 | 14.19 | **0.012*** |
| **Vocabulary** | 16 | -44.63 | 15.75 | **0.013*** |
| **Coding** | 15 | -28.87 | 11.91 | **0.031*** |
| **Similarities** | 17 | -27.76 | 13.72 | 0.061 |
| **Block Design** | 17 | -26.33 | 13.83 | 0.076 |
| **Arithmetic** | 17 | -41.15 | 10.13 | **0.001**** |
| **Matrix Reasoning** | 9 | -22.22 | 18.34 | 0.265 |
| **Digit Span** | 16 | -23.48 | 12.61 | 0.084 |
| **Information** | 17 | -42.85 | 12.19 | **0.003**** |
| **Picture Arrangement** | 15 | -31.67 | 12.11 | **0.021*** |
| **Comprehension** | 17 | -42.24 | 17.54 | **0.029*** |
| **Symbol Search** | 15 | -19.21 | 10.34 | 0.086 |
| **Letter Number Sequencing** | 9 | -41.42 | 14.49 | **0.024*** |
| **Object Assembly** | 17 | -21.27 | 13.90 | 0.147 |

| | | | | |
|---|---|---|---|---|
| Note: **P* values<0.05 are highlighted in bold; ***P* values<0.01 are highlighted in bold; sample size (n). | | | | |

**TABLE 7**

| *Relationships between FREE2 MS-QMA MR (outcome variable) with each molecular variable (predictor) using simple linear regression* | | | | | | |
|---|---|---|---|---|---|---|
| | N | Estimate coeff. | SE | P | #Standardized coeff | #R-square |
| cpg1 | 18 | 0.5488 | 0.1095 | < **0.0001**** | 0.7815 | 0.5865 |
| cpg2 | 18 | 0.6234 | 0.1239 | < **0.0001**** | 0.7828 | 0.5885 |
| cpg67 | 18 | 0.5504 | 0.0801 | < **0.0001**** | 0.8641 | 0.7308 |
| cpg89 | 18 | 0.6399 | 0.0902 | < **0.0001**** | 0.8712 | 0.7439 |
| cpg1012 | 18 | 0.6090 | 0.0671 | < **0.0001**** | 0.9150 | 0.8373 |
| *FMR1* activation ratio | 19 | -0.2216 | 0.0845 | **0.018*** | -0.5364 | 0.2877 |
| FMRP % positive | 18 | -0.0018 | 0.0009 | 0.058 | -0.4546 | 0.2067 |
| CGG | 20 | 0.00002 | 0.0001 | 0.844 | 0.0469 | 0.0022 |

| | | | | | | |
|---|---|---|---|---|---|---|
| # Standardized coefficient and R-square approaching 1 indicate that a variable has a stronger relationship with outcome variable MS-QMA MR. Note: **P* values<0.05 are highlighted in bold; ***P* values<0.01 are highlighted in bold; sample size (n). | | | | | | |

### EXAMPLE 7

### Positive and negative predictive values of this test in newborn DNA samples from FXS affected children

The positive predictive values are determined through a retrospective analysis of 50 FM newborn bloodspots using FREE2 (A) MALDI-TOF MS and MS-QMA systems. The positive predictive values for FREE2 (A) methylation analysis are calculated as the probability of methylation to provide a positive test result as determined using: (i) methylation analysis in venous blood DNA at greater than 4 years of age; (ii) neuropsychological assessments in affected subjects greater than 4 years of age. The positive methylation thresholds for each clinical measure are determined using the receiver operating characteristic curve (ROC) analysis and the ability of the MALDI-TOF MS and MS-QMA methylation value to classify the affected and not affected classes for each clinical measure will be determined as described in (Godler *et al.* (2012) *supra*).

The negative predictive values for FREE2 (A) methylation analysis are calculated, as the probability that methylation analysis in newborn blood spot DNA from 300 de-identified controls, with normal FMR1 gene as determined using standard diagnostic protocols, to provide a negative test result. The negative test result is defined by the negative methylation thresholds in venous blood DNA of the combined sample of *FMR1* expansion carriers and controls determined using ROC analysis.

In newborn blood spots at the threshold of 0.1 for males and 0.39 for females the sensitivity, specificity, positive and negative predictive value for presence of a FM allele approached 100% (Figure 4 and Figure 13). For male newborn blood spots the exception was an FM spot which had an MR value identical to those UFM 'high functioning' individuals. This was not an artifact as it was shown to be unmethylated using EpiTYPER system analysis of FREE2 (Inaba *et al.* (2013) *supra*).

Unexpectedly in newborn blood spots MS-QMA identified more than 90% of all FM females above the 0.39 threshold, while in earlier study (Inaba *et al.* (2013) *supra*) MALDI-TOF MS analysis of CpG10-12 identified only 50% above the affected threshold (which for CpG10-12 was 0.435). In an attempt to explain the blood spot discrepancy was performed MALDI-TOF MS analysis on the same newborn blood spots for FREE2 CpG10-12 and CpG6-12 (Figures 4C and D). Consistent with the previous study (Inaba *et al.* (2013) *supra*), MALDI-TOF MS analysis of CpG10-12 identified only 50% above the affected threshold. However, inclusion of additional CpG sites for MALDI-TOF MS analysis (present in the MS-QMA amplicon) increased the MOR of both control and FM groups, and altered the proportion of FM females above the affected threshold (Figure 4D). This suggests that difference in the number and the way the CpG sites were analysis is the most likely the reason why in NBS MS-QMA identified more than 90% of all FM females above the 0.39 threshold while in this and earlier studies (Inaba *et al.* (2013) *supra)* MALDI-TOF MS analysis of CpG10-12 identified only 50% above the affected threshold. Furthermore, although the EpiTYPER approach targets CpG 3, 4 and 5 within FREE2, these cluster as one fragment which is too big in size (Dalton) to be captured within the mass spectrum. For this reason CpGs 3 to 5 cannot be analyzed by the EpiTYPER approach, but are analyzed by the MS-QMA approach, and in this sense MS-QMA appears to be a superior strategy.

### EXAMPLE 8

### Application of methylation specific-quantitative melt analysis

The epigenetic marker for FXS, FREE2 (A) methylation analysis, is inversely correlated with FMRP expression in males and females with expanded *FMR1* alleles (Godler *et al.* (2010) *supra;* Godler *et al.* (2011) *supra*). Data also show that FREE2 (A) MALDI-TOF MS methylation analysis is superior to methylation-sensitive Southern blot (used in current FXS diagnostics) and FMRP immunostaining in blood as a predictor of cognitive impairment in female carriers of expanded *FMR1* alleles as assessed using Wechsler Adult Intelligence Scale (WAIS) IQ tests, with specificity and sensitivity approaching 100% (Godler *et al.* (2012) *supra*).

The MS-QMA protocol described herein also examines the FREE regions. However its reagent cost is a third of that for the reference method MALDI-TOF MS, and it is also high-throughput and methylation-specific. MS-QMA involves bisulfite conversion followed by a real-time PCR relative standard curve method utilizing unique primer set that targets specific CpG sites within the FREE2 (A) region (Godler *et al.* (2012) *supra*) which is then followed by the high resolution melt (HRM) analysis. The real-time PCR output is used as an internal DNA quality/concentration control. The HRM analysis provides methylation output, defined by AFU's are determined from melt profiles. AFU is generated by a computer algorithm to obtain specific temperature/s that provide the greatest separation between methylated and non-methylated sequences of control methylation standards. This AFU measure shows high inter- and intra-run reproducibility (<5% variation).

Figure 5 data show that MS-QMA is a robust tool for detection of FXS cryptic mosaic individuals missed using standard FXS testing. These cryptic mosaics with ASD have both a normal size allele and a FM methylated allele, where the smaller alleles are generated through somatic instability. The melt profiles of the individuals contain two peaks, one peak at the same temperature as male healthy controls, and the other higher peak at the same temperature as FM only FXS affected individuals.

In Figure 2(i) quantitation using MS-QMA provided the same methylation values as the reference method, MALDI-TOF MS. However, one of the advantages of MS-QMA is that, unlike MALDI-TOF MS, it is both qualitative and quantitative. This contributes to twice as high detection limit for methylated FM alleles (only 5%) in the background of non-methylated control alleles of for MS-QMA. Therefore, MS-QMA has the potential to identify more FXS cryptic mosaic individuals than MALDI-TOF MS. Using MS-QMA the test can accurately differentiate all carriers of harmful methylated FM alleles from healthy controls and PM carriers in a cohort studies. In the group of 17 FM females MS-QMA, AFU correlated most significantly with Full Scale IQ (FSIQ) and Verbal IQ (VIQ) (P<0.01) (Figure 2), but also with Performance IQ (PIQ) (P=0.03). AFU also showed significant correlation with most cognitive sub-score measures, with the strongest magnitude of correlation with Arithmetic (P=0.0098) and Information (P=0.0059) sub-scores. For FM females there were differences in assay sensitivity and specificity assessments and optimal AFU threshold depending on the IQ measure. The optimal threshold of 39 AFU provided highest sensitivity and specificity approaching 100%, for detection of FM with VIQ<70. In an independent cohort of 60 FM females with no formal cognitive assessment, it was found that 33% of all FM females were above 39 AFU (Figure 3(i)). If this threshold was lowered to 36 AFU it provided maximum specificity and sensitivity for FM genotype, independent of the cognitive status. Values above this lower threshold identified 58% of all FM females with only 3% control and PM female false positives in venous blood DNA. If applied in newborn screening, these false positives can be further resolved through by second stage testing follow-up involving CGG sizing.

The output of MS-QMA in DNA from venous blood to the previously published and validated MALDI-TOF MS method (Godler *et al.* (2012) *supra*) in the larger cohort of males and females for venous blood DNA and newborn and adult dried blood spots (DBS) (Figure 3(i)). In both venous blood DNA and DBS, FM males and females showed methylation significantly higher than in their respective PM and control groups as assessed by both MALDI-TOF MS and MS-QMA (P<0.0001). As expected, the only FM group that showed overlap with PM and control groups were FM females. Interestingly, in DBS the threshold of 39 AFU determined to be optimal for identification of the FM group with verbal cognitive impairment (Figure 2(i)) identified 96% of all FM females examined which is ∼3 fold increase to that in venous blood FM subgroup (Figure 3(i)).

In venous blood DNA the combined comparison of all allele classes for males (n=76) and females (n=223) showed correlation coefficient of 0.99 and 0.78, respectively, with P<0.0001. In DBS the combined comparison of all allele classes for males (n=50) and females (n=54) showed correlation coefficient of 0.98 and 0.5, respectively, with P<0.0001. In the FM only males, venous blood DNA subgroup (n=39) showed correlation coefficient of 0.75; p<0.0001; and for DBS (n=22) correlation coefficient of 0.85; p<0.0001. In the FM only females, venous blood DNA subgroup (n=78) showed correlation coefficient of 0.77; p<0.0001; and for DBS (n=25) correlation coefficient of 0.37; p=0.07 (not significant possibly due to small sample size). It is also important to note that in the rare FM male not affected with FXS (IQ>70) and non-methylated as determined by Southern blot (UFM) (red triangles in Figure 3(i)), were also completely non-methylated by MALDI-TOF MS and MS-QMA in both venous blood DNA and DBS. This suggests that MS-QMA FREE2 (A) methylation output in blood reflects that in brain, and is consistent with two reference methods - MALDI-TOF MS and methylation sensitive Southern blot analysis.

The present disclosure provides a novel method (MS-QMA) which is much more efficacious and cost-effective than Sequenom EpiTYPER approach for methylation analysis of the FREE2 region if utilized in FXS diagnostics and population screening. In females the *FMR1* activation ratio determined using the Southern blot was significantly correlated with FREE2 methylation assessed using MS-QMA and the EpiTYPER system (Godler *et al.* (2011) *supra*). In males, two PM/FM mosaics and three 'high functioning' FM males (IQ>70) unmethylated in the *FMR1* CpG island by Southern blot (Godler *et al.* (2010) *supra*), were below the 0.1 MR threshold within FREE2 as determined using MS-QMA and the EpiTYPER system (Figure 4A). Therefore, the ability of MS-QMA to differentiate the 'high functioning' FM males from the typical FXS males, is likely to be of prognostic value when used for early detection of FXS in males.

A somewhat surprising finding was that in newborn blood spots at 0.39 threshold MS-QMA identified not only most FM males, but also almost all FM females, with sensitivity, specificity, positive and negative predictive value for presence of a FM allele between 92 and 100%. For this reason, this indicates that the MS-QMA test has applications for early detection of all FXS FM in females as well as males, particularly if used within the 1^{st} year of life.

However, at the age range from 6 to 35 years at the same threshold, in venous blood MS-QMA identified only 50% of all FM females. This FM group had VIQ < 70, with the test showing sensitivity of 100% and specificity ∼95%. Rather than a technical issue or bias of ascertainment, the likely explanation for this is that age range of the participants was different between venous blood and blood spot cohorts. While in the venous blood for FM, PM and control females there was no significant relationship between age and MS-QMA output (Figure 12). This indicates that the MS-QMA output has prognostic value.

### EXAMPLE 9

### Timing of methylation analysis

Data suggest that methylation assessment of the FREE2 (A) biomarkers using MS-QMA can be done at any time of life and that this produces similar result as at birth in both males and females (Figure 6). Specifically, it was found that for MS-QMA FREE2 (A) analysis: (i) there was no significant relationship between the age and AFU in males (n=76) and females (n=223) in Figure 2(i), comprising controls, PM and FM individuals with age range birth and 80 years (3); (ii) methylation values for both NBS and venous blood for FM male and females (Figures 2(i) and 3(i)) were significantly higher than methylation values for control and PM and healthy controls, and the positive, cognitive impairment threshold of 39 AFU; and the 39 AFU threshold separated FM individuals from PM and control groups as effectively in 500ng of DNA from venous blood as in cell extract from one 3 mm punch per sample from dried blood spots of adults and newborns; (iii) in five FM individuals the methylation values were almost identical for the repeated methylation measures taken at birth (NBS material) and at 15, 32, 35 and and 38 years of age (dried blood spot material), and was significantly higher than that for NBS material of male and female controls (Figure 6). Other FREE2 regions (e.g. FREE2 (B), FREE2 (C), FREE2 (D) and FREE2 (E)) as well as FREE3 may also be assayed.

It is also important to note that one female control NBS kept at room temperature for greater than 11 years, showed AFU greater than 39 threshold by ∼2 units, and two had AFU of 39. However, for NBS kept at room temperature for 3 years or less, all AFU are below this threshold. This suggests that there is small increase in AFU for NBS with age possibly due to increased DNA fragmentation/poor DNA quality. This, however, is not a significant limitation when comparing MS-QMA results between birth and time of consent, as most FM participants are children under 10 years of age. Methylation of FREE2 (A) region is highly conserved between tissues and cell types (Godler *et al.* (2010) *supra*), including post-mortem brains of FXS males with co-morbid autism. Hence, data suggest that the biomarker MS-QMA test has superior prognostic and diagnostic value in *FMR1* expansion carriers too young to undergo formal neuropsychological testing.

### EXAMPLE 10

### MS-QMA analysis of saliva and venous blood DNA for detection of sex chromosome aneuploidies

As shown herein, the threshold of 0.37 MR was the maximum value of the female healthy control range as well as the optimal for detection of FM females with performance IQ (PIQ) and full scale IQ (FSIQ) impairment (<70). Using saliva DNA, 3 out of 57 sex chromosome aneuploidy samples showed MS-QMA MR above the 0.37 and 0.39 MR thresholds. These were XXY samples with apparently skewed X inactivation (Figure 8(i)). It is also of interest that the saliva DNA of the FXS affected males had MS-QMA MR values well above these thresholds, while for the PM/FM mosaic group one male showed an MR of 0.37.

For venous blood DNA, only samples with three or more copies of X-chromosome showed MS-QMA MR above 0.37 and 0.39 MR thresholds (Figure 8(ii)). The 47,XXX and 49,XXXXY groups showed MS-QMA MR significantly above those of female controls. As shown in Figures 8(iii)-8(v), MS-QMA methylation was able to distinguish control males and females from individuals with Turners Syndrome (45,X), Triple X Syndrome and Jacob's Syndrome (47,XYY).

As expected, saliva and venous blood DNA samples with a Y chromosome according to previous laboratory testing also showed ∼1 copy of SRY, while samples with two or more copies of a Y chromosome showed SRY copy number between 1.5 and 2 (Figure 8(i) and 8(ii)). The only exception was one 45,X sample that consistently showed SRY/β-globin ratio above the positive threshold of 0.1. Because the Y chromosome was not detected using microarray analysis in this sample, this result may be either a false positive or a true positive where real-time PCR may be a more sensitive approach for detection of low level mosaicism.

### EXAMPLE 11

### Detection of skewed X-inactivation and the comparison with the EpiTYPER system

The relationship between X-inactivation and FREE2 MS-QMA MR is investigated in a larger sample than was previously analyzed with the EpiTYPER system. Despite the differences in assay design, the distribution of the MR values in venous blood DNA for both assays was almost identical in FM females. It showed clear skewing towards the unmethylated state from the distributions expected if the X-inactivation were random at this locus (Figure 9; bell shaped curve). For both assays, methylation in FM males was significantly elevated compared to that in FM females. The only difference between the two assays was in FM males where for the EpiTYPER system the methylation ratio was skewed towards 1, while the FREE2 MS-QMA MR was normally distributed in the same samples, with the top and bottom tails of distribution at ∼1 and 0.5.

### EXAMPLE 12

### MS-QMA and the EpiTYPER system analysis in venous blood and saliva DNA, and dried blood spots

When examining specific subgroups, in control males there was no significant correlation between methods regardless of the sample type, primarily because all of the male control samples were below the detection limit of the EpiTYPER system MR of 0.1. In female controls the two methods were significantly correlated in venous blood and saliva (p<0.05), but not in dried blood spots (Figure 10 and Table 8). As expected, the highest correlation was observed in venous blood DNA and dried blood spots of FM males and females (P<0.0001) possibly due to the greatest spread in methylation values. This significant relationship was consistent in the venous blood DNA of the PM/FM mosaic group, but was absent in female PM carriers. In saliva DNA of Klinefelter syndrome (47,XXY) individuals, the methylation ratio between the MS-QMA analysis and EpiTYPER reference method were also significantly correlated (p<0.05).

Furthermore, the specific comparisons between MS-QMA and the EpiTYPER system in the control females (Figure 10) using dried blood spots of poor DNA quality, showed that MS-QMA did not identify false positives, and by this outperformed the EpiTYPER system. Specifically there were two outliers above the MALD-TOF MS threshold of 0.435 MOR, including one female control (Tassone *et al.* (2008) *supra*), these same samples had MR below 0.39 for the MS-QMA analysis. This suggests that MS-QMA would be more appropriate to avoid false positives in screening applications associated with low quantity and poor quality DNA.

**TABLE 8**

| *Linear regression analysis between predictor FREE2 methylation assessed using MALDI-TOF MS and outcome MS-QMA) in venous blood DNA, Newborn and Dried Blood Spots (NBS and DBS) and saliva DNA. Note: blood spots stored for more than 10 years were not included in these analyses.* | | | | | | | | | |
|---|---|---|---|---|---|---|---|---|---|
| | **n** | **Venous Blood DNA Coef ± SE** | **R²** | **n** | **NBS/DBS Coef ± SE** | **R²** | **n** | **Saliva DNA Coef ± SE** | **R²** |
| **ALL samples** | 433 | 1.042±0.013*** | 0.94 | 239 | 1.011±0.020*** | 0.92 | 97 | 0.976±0.046*** | 0.82 |
| **Males** | 132 | 1.048±0.014*** | 0.98 | 124 | 1.073±0.016*** | 0.97 | | | |
| **Females** | 301 | 0.982±0.032*** | 0.75 | 115 | 0.650±0.064*** | 0.48 | | | |
| | | | | | | | | | |
| ♂ **controls** | 17 | 0.125±0.340 | 0.01 | 89 | -0.088±0.236 | 0.02 | 13 | -0.500±1.158 | 0.02 |
| ♀ **controls** | 86 | 0.256±0.115* | 0.06 | 83 | 0.299±0.100 | 0.10 | 26 | 0.440±0.208* | 0.16 |
| ♂ **FM** | 49 | 0.559±0.069*** | 0.58 | 24 | 0.941±0.121*** | 0.73 | | ... | |
| ♀ **FM** | 95 | 1.040±0.073*** | 0.69 | 24 | 0.759±0.260** | 0.28 | | ... | |
| | | | | | | | | | |
| ♂ **PM/FM** | 16 | 0.998+0.069*** | 0.94 | ... | ... | ... | | ... | |
| ♀ **PM** | 102 | 0.185±0.097 | 0.04 | ... | ... | ... | | ... | |
| **47,XXY** | ... | ... | ... | ... | ... | ... | 47 | 0.330±0.130* | 0.12 |

| | | | | | | | | | |
|---|---|---|---|---|---|---|---|---|---|
| N represents sample size; Coef. represents regression coefficient; SE represents standard errors ; Note: Regression results are presented for groups with n>10; *P<0.05; **P<0.01; ***P<0.001. | | | | | | | | | |

### EXAMPLE 13

### Application of MS-QMA on sample from imprinting disorders

Mammals inherit two complete sets of chromosomes, one from the father and one from the mother, and most autosomal genes are expressed from both maternal and paternal alleles. Imprinted genes show expression from only one member of the gene pair (allele) and gene expression is determined by the parent. Examples of imprinting disorders includes Beckwith-Wiedemann syndrome, Silver-Russell syndrome, Prader-Willi syndrome and Angelman syndrome. A methylation specific PCR test is available for the detection of Prader-Willi syndrome (PWS) and Angelman syndrome (AS) [Kubota et al. (1997) Nat Genet 16(1):16-17]. It is anticipated that the number of positive confirmed patients with these disorders will increase with the use of the MS-QMA assay, which gives a greater sensitivity and specificity.

Prader-Willi syndrome (PWS) and Angelman syndrome (AS) are two different neurological disorders caused by opposite defects in imprinting of the same chromosomal region 15q11-q13. The estimated frequencies in the general population of both PWS and AS are between 1 in 10,000 and 1 in 20,000. Ninety nine percent of all PWS cases are associated with detectable hypermethylation of the SNRPN gene promoter within the chromosomal region, while approximately 80% of AS cases have detectable hypomethylation of the same SNRPN locus. The hypermethylation in PWS is usually caused by absence of a paternally contributed allele that is unmethylated; while hypomethylation of AS is usually associated with absence of maternally contributed allele that is hypermethylated at the locus. In healthy control individuals not affected with AS or PWS, SNRPN gene promoter methylation is usually ∼50%, as they have one copy of paternally contributed and one copy of maternally contributed allele.

Primers were designed for the MS-QMA for the detection of PWS/AS using a similar region of the SNRPN gene used in the current diagnostic PCR assay (Kubota *et al.* (1997) *supra*). These are SEQ ID NOs:15 through 18 for targets SEQ ID NO:19 (SNRPN-M for AS) [Chr 15:25200039-252000212] and SEQ ID NO:20 (SNRPN-P for PWS) [Chr 15:25200068-252000167].

Figure 11 shows the results of MS-QMA spiking experiments of PWS and AS blood DNA samples mimicking mosaicism; as well as venous blood DNA of PWS, AS and healthy control individuals. From the figure it is evident that MS-QMA provides quantitative methylation assessment of SNRPN gene promoter, and can be used to differentiate pure PWS and AS, as well as PWS and AS artificially spiked mosaics from healthy controls.

### EXAMPLE 14

### Serial dilution after bisulphite conversions for MS-QMA

Serial dilutions after bisulphite conversions are required for maximum efficacy for MS-QMA and the linkage with clinical prognosis. Without serial dilution (Figure 7), not all samples may fall into the dynamic range of DNA control 1. This is demonstrated by the red dots in Figure 7 which correspond to only one dilution and these samples do not correlate to the clinical prognosis. These samples are inconsistent with both the clinical prognosis and the expected level of methylation. A filter process is used to ensure the greatest correlation with clinical prognosis and quantitation of the methylated DNA. An example of this filter is as follows: if the DNA concentration is not in the dynamic range and greater than two standard deviations from the mean, the data generated from this sample (which includes methylation data) are not use for further analysis. The mean is re-calculated for the remaining samples without the outlier and this mean value is used to determine the quantitative methylation result.

The MS-QMA methylation assay has significant potential for use in newborn FXS screening as there has been no test available that is suitably sensitive in males and females, is high throughput and low cost. The benefits of identifying most male and female probands early are improved clinical management, identification of other carriers through cascade testing and the provision of this information for reproductive planning. As part of the 1^{st} NBS screen, the 0.39 threshold should be used followed by second line testing that would involve CGG sizing, and this would confirm that all positives carry a FM alleles. This may present a better alternative to using CGG sizing as a first line test in newborns or very young children, because detection of PM alleles that have been associated with late onset disorders (Godler *et al.* (2010) *supra;* Hagerman and Hagerman, Nat Clin Pract Neurol 3:107-112), would raise the ethical issue of pre-symptomatic testing for currently untreatable and non-preventable disorders with incomplete penetrance (Bailey et al. (2009) J Pediatr Psychol 34:648-661). Furthermore, detection of the relatively common GZ and PM alleles which do not cause FXS at population wide level, would require large scale genetic counselling follow-up, which have significant add-on cost-benefit implications.

In the diagnostic context, MS-QMA can be easily combined with several PCR based approaches recently developed to reliably amplify PM and small FM alleles (Tassone *et al.* (2008) *supra;* Filipovic-Sadic *et al. supra;* Hantash *et al. supra;* . MS-QMA methylation values could be used to accurately separate most high end unmethylated PM from low end methylated FM alleles, and may in the future provide prognostic information from quantitative methylation data in both males and females. This could remove the need for the cumbersome Southern blot and identify all categories of expanded alleles.

### EXAMPLE 15

### Correlation with cognitive sub-scores in FM females

In a sub-group of 20 FM females for whom a set of IQ subscales was available, subtest-scores and indices available, it was found that MS-QMA MR strongly correlated with subtest scores representing different aspects of VIQ. Arithmetic subtest scores (Working Memory Index) which largely rely on working memory and attention, and the Information subtest scores (Verbal Comprehension Index) which examine general knowledge stood out as the subtest scores most strongly correlated with MS-QMA MR (p<0.01).

It is also of interest to note, that most FM and all PM females that were within the MS-QMA borderline range 0.39 to 0.41, were high functioning (VIQ>70). This, however, does not rule out other forms of FXS related impairment that may be identified using more subtle measures of cognitive function, such as the sub-scores and indexes of IQ, or measures of behavioral impairment such as ADOS-G.

Hence, described herein for venous blood DNA, in the PM and FM females for the age range examined in this study, MS-QMA has high sensitivity if the aim is not to identify all FM females, but to identify only those likely to have a low verbal IQ (<70). However, in newborn blood spots, MS-QMA is likely to have another application if used within the 1^{st} year of life, which is identification of more than 90% of all FM males and females. This is superior to the comparator EpiTYPER system that target the same sites as MS-QMA, but cannot analyze some of these because their fragments are too large for MALDI-TOF MS based assessment (Tost and Gut (2012) DNA methylation analysis by maldi mass spectrometry: Wiley-VCH Verlag GmbH & Co. KGaA:3-34). Other advantages of MS-QMA analysis are: (i) flexibility for both quantitative (as in the case of standard HRM) and qualitative (as in the case of the EpiTYPER System) assessment; (ii) automated detection of DNA concentration in the samples post conversion; (iii) incorporation of inbuilt PCR amplification quality control measures and associated automated data cleaning; and (iv) reduced time to obtain the methylation ratio - 6 to 9.5 hours for MS-QMA including the bisulfite conversion compared with 48 to 72 hours for the EpiTYPER system. Furthermore, MS-QMA is an automated method that does not require specialized proprietary equipment and extensive training. It uses standard HRM reagents and standard real-time PCR machines that cost up to one third of that of MALDI-TOF MS and pyrosequencing based chemistries and equipment, and for these reasons the likelihood may be high for its widespread uptake into practice as a screening tool for both research and diagnostic applications.

In this study, it is demonstrated that FREE2 MS-QMA can identify most sex chromosome aneuploidies and detect both locus-specific skewing towards the hypomethylated state, as is apparent in FM females, and skewed X inactivation towards the hypermethylated state for the whole X-chromosome, as with sex chromosome aneuploidies with three of more X-chromosomes. The performance of MS-QMA was assessed using the same sample set for detection of these abnormalities. It was found that similar to the EpiTYPER system, when combined with the SRY analysis at the MR threshold of 0.1, MS-QMA identified sex chromosome aneuploidies with specificity and sensitivity approaching 100%. However, without the SRY analysis and at the VIQ threshold of 0.39 MR, MS-QMA could not detect the vast majority of sex chromosome aneuploidies using either venous blood or saliva DNA.

In the *FMR1* locus specific context, as with EpiTYPER system results, the MS-QMA MR control range was lower than the expected value of 0.5, with ∼0.4 being the maximum control value. The test also showed clear skewing towards the unmethylated state from the distributions expected if the X-inactivation were random in FM females at this locus. Specifically, if the X-inactivation were random, one would expect the higher tail of the MR distribution to be at 1, representing all cells having the normal size allele on the inactive X and the methylated FM allele on the active X. The lower tail of the distribution would then be 0.5 MR with all cells having the FM alleles on the inactive X and normal cell alleles on the active X. In contrast, the upper tail for both the MS-QMA and EpiTYPER system results was at ∼0.7 MR with the lower tail at ∼0. 2MR, demonstrating skewing towards the unmethylated state. Because in FM males more than half of the samples showed MR values between 0.7 and 1 MR using either method , the lack of any FM female results with MR values above 0.7 cannot be considered to be a result of technical bias associated with the MS-QMA assay.

It is also interesting to note that for FM males with 100% methylation at the FMR1 CpG island (shown on Southern blot), both MS-QMA and the EpiTYPER system find methylation of FMR1 intron 1 between 60% and 100%. This suggests that methylation mosaicism between different CpG sites in FM males maybe more common than previously thought, and this may relate to the wide spectrum of clinical phenotype (other than severe cognitive impairment) found in FM males.

There are a number of published HRM based methods developed for quantitative methylation analysis developed for locus specific (Snell et al. (2008) Breast Cancer Res 10:R12; Kristensen et al. (2008) Nucleic Acids Res 36:e42; Candiloro et al. (2011) Epigenetics 6:500-507; Malentacchi et al. (2009) Nucleic Acids Res 37:e86) or genome wide (Newman et al. (2012) Epigenetics 7:92-105) applications, each with advantages and limitations for their specific applications. However, one common feature between all of these is use of methylation standards generated from unmethylated samples spiked at different ratios with artificially methylated DNA. An important distinction between these methods and MS-QMA is that MS-QMA uses a real-time PCR based internal filter process on serially diluted bisulfite conversions to 'clean' the data prior to a methylation value being derived. This filter process is essential for the applications described in this study, particularly for the intermediate methylation range this was not performed in previously described quantitative HRM methods, which were primarily developed for assessments in the low methylation range in cancer diagnostics Snell *et al.* (2008) *supra;* Kristensen *et al.* (2008) *supra;* Candiloro *et al.* (2011) *supra;* Malentacchi *et al.* (2009) *supra*). The lack of a filtering/quality control process post-conversion could also be also the reason why quantitative HRM has had limited utility with poor quality DNA samples (Pichler et al. (2009) JMD 11;140-147). For poor DNA quality samples these methods have been largely restricted to formalin fixed paraffin embedded tissue samples (Balic *et al.* (2009) *JMD 11*:102-108); none have been described for quantitative methylation analysis using crude extracts from adult and newborn blood spots or saliva DNA of poor quality as described here.

Comparison of quantitative HRM to pyrosequencing has been made in assessment of methylation of APC and CDKN2A genes (Migheli et al. (2013) PLoS One 8:e52501). As with the previous application of HRM in FXS testing (Elias *et al.* (2011) *supra*), mixtures of methylated and unmethylated DNA were used as standards. While the derived methylation results for both APC and CDKN2A in the clinical test samples was highly consistent with the pyrosequencing results at low methylation levels (∼0 to 30%), , HRM based quantification overestimated methylation by as much as 20% in the 30 to 60% methylation range, as compared to pyrosequencing.

To overcome this technical limitation of quantitative HRM for detection of FXS females, inter- and intra-run methylation ratio variation has to be ∼5% in the methylation range between 30 and 60%. This is achieved using a quality control filter process involving quantitative real-time PCR analysis of serial dilutions after bisulphite conversion.

Hence, demonstrated herein is the immediate applications of MS-QMA for detection of sex chromosome aneuploidies and X-inactivation at the *FMR1* locus. However, the method has potential for any application where quantitative detection of even small changes in the genomic position and the amount of locus specific methylation that of diagnostic or prognostic significance due to mosaicism in the methylation state within and between different cell types (Godler *et al.* (2013) *supra*). These may include monogenic disorders such as Rett Syndrome (Signorini et al (2013). PLoS One 8:e56599), trinucleotide disorders such as Friedreich ataxia and myotonic dystrophy, imprinting disorders such as Angelman, Prader-Willi and Beckwith-Weidemann Syndromes, disorders related to more general skewed X-inactivation, as well as somatic genetic disorders such as cancer.

### BIBLIOGRAPHY

Allingham-Hawkins et al. (1999) Am J Med Genet 83:322-325
Bailey et al. (2009) J Pediatr Psychol 34:648-661
Balic et al. (2009) JMD 11:102-108
Bodega et al. (2006) Hum Reprod 21:952-957
Bonner and Laskey (1974) Eur. J. Biochem. 46:83
Boyd et al. ((2006) Anal Biochem 354:266-273
Bretherick et al. (2005) Hum Genet 117:376-382
Buiting et al. (2003) Am J Hum Genet 72(3):571-577
Candiloro et al. (2011) Epigenetics 6:500-507
Chanchaiya et al. (2010) Hum Genet 128:539-548
Chen et al. (2011) Genet Med 13:528-538
Christie et al. (2013) Am J Med Genet A 161A:301-311
Coffee et al. (2009) Am. J. Hum. Genewt. 85:503-514
Dahl et al. (2007) Clin Chem. 53(4):790-793
de Vries et al. (1996) Am J Hum Genet 58:1025-1032
Dissanayake et al. (2009) J Child Psychol Psychiatry 50:290-299
Dodds et al. (2009) Anal Chem
Elias et al. (2011) Genet. Testing and Mol. Biomarkers 15(56):387-393
Fahy et al. (1991) PCR Methods Appl. 1(1):25-33
Filipovic-Sadic et al. Clin Chem 56(3):399-408
Godler et al. (2010) Genet Med 12:595
Godler et al. (2010) Hum Mole Genet 19:1618-1632
Godler et al. (2011) J Mol Diag 13:528-536
Godler et al. (2012) Clin Chem 58:590-598
Godler et al. (2013) Hum Mol Genet*,* In Press
Greco et al. (2002) Brain 125:1760-1772
Hagerman et al. (2001) Neurology 57(1):127-130
Hagerman and Hagerman, Nat Clin Pract Neurol 3:107-112
Hantash et al. Genet Med 12(3):162-173
Hamilton et al. (2012) European Human Genetics Conference, June 23-26, Nürnberg, Germany
Haughland (1996) Handbook of Fluorescent Probes and Research Chemicals, Molecular Probes, Inc. Eugene, Oreg.
Inaba et al. (2013) Genet Med 15:290-298
Irwin et al. (2000) Cereb Cortex 10(10):1038-1044
Jacquemont et al. (2005) J Med Genet 42(2):e14
Jin et al. (2003) Neuron 39(5):739-747
Jin and Warren (2000) Hum. Mol. Genet 9(6):901-908
Kenneson et al. (2001) Hum Mol Genet 10:1449-1454
Khaniani et al. (2008) Mol Cytogenet 1:5
Kristensen et al. (2008) Nucleic Acids Res 36:e42
Kubota et al. (1997) Nat Genet 16(1):16-17
Lee et al. (1993) J Med Chem 36(7):863-870
Loesch et al. (2002) Am J Med Genet 107:136-142
Loesch et al. (2005) Clin Genet 67(5):412-417
Loesch et al. (2007) Neuro Sci Biobeta Rev 31:315-326
Loesch et al. (2007) J Med Genet 44:200-204
Loesch et al. (2009) Am J Med Genet A 149A:2306-2310
Loesch et al. (2012) Glin Genet 82:88-92
Malentacchi et al. (2009) Nucleic Acids Res 37:e86
Marmur and Doty, (1962) J. Mol. Biol. 5:109
Migheli et al. (2013) PLoS One 8:e52501
Newman et al. (2012) Epigenetics 7:92-105
Nolin et al. (1994) Am J Med Genet. 51(4):509-512
Nolin et al. (2003) Am J. Hum Genet 72(2):454-464
Nygren et al. (2008) J Mol Diagn 10:496-501
Pichler et al. (2009) JMD 11; 140-147
Pieretti et al. (1991) Cell 66(4):817-822
R Foundation for Statistical Computing (2007) http:/www.r-project.org/(Accessed February 2009
Rein et al. (1998) Nucleic Acids Res. 26:2255
Sherman (2000) Am J Med Genet 97:189-194
Signorini et al (2013). PLoS One 8:e56599
Snell et al. (2008) Breast Cancer Res 10:R12
Sullivan et al. (2005) Hum Reprod 20:402-412
Tassone et al. (1999) Am J Med Genet 84:250-261
Tassone et al. (2000) Am J Med Genet 97:195-203
Tassone et al. (2000) American Journal of Medical Genetics 94:232-236
Tassone et al. (2008) J Mol Diagn 10:43-49
Terracciano et al. (2005) Am J Med Genet C Semin Med Genet 137C(1):32-37
Tost and Gut (2012) DNA methylation analysis by maldi mass spectrometry: Wiley-VCH Verlag GmbH & Co. KGaA:3-34
Verkerk et al. (1991) Cell 65(5):905-914
Wang et al. (2009) J. Mol. Diagn. 11(5):446-449
Wechsler (1991) Wechsler Intelligence School for Children, 3rd Ed. (WISC-III), The Psychological Corporation, San Antonio, Texas, USA
Wechsler (1997) Orlando, the Psychological Corporation
Wechsler (2002) Wechsler Protocol and Primary School of Intelligence, The Psychological Corporation, San Antonio, Texas, USA
Wey et al. (2005) Eur J Hum Genet 13(3):273-277
White et al. (2007) Clin Chem 53(11):1960-1962
Youden (1950) Cancer 3:32-35
Zhou et al. (2006) Clin Chem 52:1492-1500

## Claims

1. An *in-vitro* method for quantitating the extent of methylation of a target site within a DNA sample, the method comprising:
(i) subjecting a DNA sample to bisulfite treatment to convert non-methylated cytosine nucleotides in the DNA to uracil nucleotides;
(ii) amplifying and melting in real-time a portion of the DNA sample comprising the target site with selected forward and reverse primers incorporating a double-strand intercalating label providing an identifiable signal in double stranded DNA, the amplification and melting occurring together with a first control sample set of known DNA concentration and a second control DNA sample set of known percentage of methylated and non-methylated DNA;
(iii) generating two standard curves: (a) a first standard curve derived from the real-time amplification of the first control sample, wherein the first standard curve is expressed as DNA concentration *versus* cycle threshold (Ct) which allows for the determination of the dynamic linear range of amplification and for the determination of the stable amplified product melting range of the first control sample where the signal strength is independent of DNA dilution; and (b) a second standard curve derived from the real-time melting of the second control DNA sample, wherein the second standard curve is expressed as signal strength due to melt release *versus* the known percentage of methylation from the second control DNA sample;
(iv) determining whether the signal strength from the amplification and melting of the DNA sample comprising the target site falls within the dynamic linear range of amplification and the stable amplified product melt range by using the standard curves generated in (iii); and
(v) using the signal strength falling within the dynamic linear range of amplification and the stable amplified product melt range to determine the percentage of methylation for the target site within the DNA sample by reading the percentage of methylation from the level of signal strength using the second standard curve derived from the second control DNA sample; and optionally
(vi) correlating extent of methylation with a clinical phenotype.

2. The method of Claim 1, wherein the DNA sample is serially diluted after bisulfite conversion, the method optionally further comprising averaging the Aligned Fluorescence Units (AFU) to determine the percentage methylation if multiple dilutions of the same DNA sample are within the dynamic linear range and stable amplified product melting range.

3. The method of Claim 1 or Claim 2, wherein the amplification in step (ii) is a real-time polymerase chain reaction (RT-PCR) and/or the melting in step (ii) is a high resolution melt (HRM) reaction conducted at the greatest difference between the non-methylated and methylated control samples.

4. The method of Claim 3, wherein the label incorporated into or released from the amplified DNA in real-time is an intercalating fluorescent dye which incorporates into double stranded DNA and fluorescences at a signal strength measuring in AFU and which does not provide a fluorescence signal in single stranded DNA.

5. The method of Claim 4, further comprising employing an algorithm to determine whether a DNA sample is within the dynamic linear range of amplification and is within the stable amplified product range and to correlate an AFU with percentage of methylation of the DNA sample.

6. The method of any one of Claims 1 to 5, wherein the clinical phenotype is any one or more of the phenotypes selected from the group consisting of cognitive impairment, memory loss, poor cognitive retention, poor cognitive function with a specific emphasis of poor verbal/executive function, behavioral deficit, level or impairment of neurological development, tumor growth development, sex determination, aneuploidy, immune response progression, a trinucleotide disorder, an imprinting disorders, a modified X-chromosome disorder, an X-linked disorder, X-chromosome inactivation, tissue rejection or identifying fetal cells in a maternal subject, autism, mental retardation, Prader-Willi syndrome syndrome/Angelman syndrome, Alzheimer's disease, bipolar disorder, diabetes, male sexual orientation, obesity, schizophrenia, Parkinsonsim, depression, attention deficit disorder, mood disorder and a cancer selected from bladder, breast, cervical, colorectal, esophageal, hepatocellular, lung, mesothelioma, ovarian, prostate, testicular cancer, leukemia, pathoneurodevelopmental or pathoneurodegenerative condition, Huntington's disease (HD), dentatorubropallidoluysiantrophy (DRPLA), spinobulbar muscular atrophy or Kennedy disease (SBMA), spinocerebella ataxia Type 1 (SCA1), spinocerebella ataxia Type 2 (SCA2), spinocerebella ataxia Type 3 or Machado-Joseph disease (SCA3), spinocerebella ataxia Type 6 (SCA6), spinocerebella ataxia Type 7 (SCAT), spinocerebella ataxia Type 17 (SCA17) and non-polyQ diseases such as Fragile X syndrome (FXS), Fragile X-associated tremor or ataxia (FXTAS), Fragile XE mental retardation (FRAXE), myotonic dystrophy (DM), spinocerebella ataxia (SCAB), spinocerebella ataxias Type 12 (SCA12), Fragile X-associated primary ovary insuficiency (FXPOI), Friedrich's ataxia (FRDA), Fragile type, folic acid type, rare 12 (FRA12A), autism (including co-morbid autism), mental retardation (MR), Klinefelter's syndrome, RNA toxicity disease, Turner's syndrome, a modified X-chromosome including Triple X syndrome and Jacob's syndrome and cognitive impairment.

7. The method of any one of Claims 1 to 6, wherein the DNA target site is selected from a site within a genetic locus, an intron, exon, intron-exon boundary, promoter region or a region 5' of the promoter region, a CpG island or island shores or group of CpG islands or island shores, a site downstream of the 3' end region of a genetic locus or within a genomic region selected from the group consisting of the FMR1 genetic locus, FREE2 (A) having the nucleotide sequence as set forth in SEQ ID NO:1 or a nucleotide sequence with at least 80% identity to SEQ ID NO:1 or a nucleotide sequence which hybridizes under medium stringency conditions to the complement of SEQ ID NO: 1; FREE2 (B) comprising the nucleotide sequence set forth in SEQ ID NO:2 or a homolog thereof or portion or part thereof defined by having at least 80% nucleotide sequence identity to SEQ ID NO:2 or which hybridizes to SEQ ID NO:2 or its complementary form under medium stringency conditions; FREE2 (C) comprising the nucleotide sequence set forth in SEQ ID NO:3 or a homolog thereof or portion or part thereof defined by having at least 80% nucleotide sequence identity to SEQ ID NO:3 or which hybridizes to SEQ ID NO:3 or its complementary form under medium stringency conditions; FREE2 (D) comprising the nucleotide sequence set forth in SEQ ID NO:4 or a homolog thereof or portion or part thereof defined by having at least 80% nucleotide sequence identity to SEQ ID NO:4 or which hybridizes to SEQ ID NO:4 or its complementary form under medium stringency conditions; FREE2 (E) comprising the nucleotide sequence set forth in SEQ ID NO:5 or a homolog thereof or portion or part thereof defined by having at least 80% nucleotide sequence identity to SEQ ID NO:5 or which hybridizes to SEQ ID NO:5 or its complementary form under medium stringency conditions; FREE3 having the nucleotide sequence as set forth in SEQ ID NO:6 or a nucleotide sequence with at least 80% identity to SEQ ID NO:6 or a nucleotide sequence which hybridizes under medium stringency conditions to the complement of SEQ ID NO:6, the SNRPN genetic locus, the SNRPN genetic locus comprising the nucleotide sequence set forth in SEQ ID NO:19 or a homolog or portion or part thereof defined by having at least 80% nucleotide identity to SEQ ID NO:19 or which hybridizes to SEQ ID NO:19 or its complementary form under medium stringency conditions; a nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions.

8. The method of Claim 7, wherein a change in extent of methylation within a genomic region selected from the group consisting of the SNRPN genetic locus, the SNRPN genetic locus comprising the nucleotide sequence set forth in SEQ ID NO:19 or a homolog or portion or part thereof defined by having at least 80% nucleotide identity to SEQ ID NO:19 or which hybridizes to SEQ ID NO:19 or its complementary form under medium stringency conditions; a nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions is associated with Prada-Willi syndrome (PWS)/Angelman syndrome (AS).

9. The method of any one of Claims 1 to 8, wherein the DNA sample is derived from cells contained in body fluid or a dried body fluid sample, sputum or other respiratory fluid, blood or a fraction or dried form thereof, tissue fluid, a tissue sample, lymph fluid, semen, urine, fecal material or skin material from a human subject.

10. The method of any one of claims 1 to 9 when used for detecting the presence of a pathological condition associated with methylation within the SNRPN locus or a propensity to develop the same in a human subject, wherein:
(a) the amplification reaction in part (ii) uses primers selective of a region of the SNRPN genetic locus comprising CpG and/or CpNpG sites, the CpG and CpNpG sites located in a region selected from:
(i) SNRPN genetic locus (SNRPN-M) comprising the nucleotide sequence set forth in SEQ ID NO:19 or a homolog thereof or portion or part thereof defined by having at least 80% nucleotide sequence identity to SEQ ID NO:19 or which hybridizes to SEQ ID NO:19 or its complementary form under medium stringency conditions; and
(ii) SNRPN genetic locus (SNRPN-P) comprising the nucleotide sequence set forth in SEQ ID NO:20 or a homolog thereof or portion or part thereof defined by having at least 80% nucleotide sequence identity to SEQ ID NO:20 or which hybridizes to SEQ ID NO:20 or its complementary form under medium stringency conditions.

11. The method of Claim 10 wherein the pathological condition is PLUS/AS.

12. The method of any one of claims 1 to 9 when used for detecting the presence of a pathological condition associated with the presence of a pre-mutation or a full mutation of the Fragile X Mental Retardation (FMR) genetic locus (FMR1) or a propensity to develop the same in a human subject, wherein:
(a) the amplification reaction in part (ii) uses primers selective of a region of the FMR1 genetic locus comprising CpG and/or CpNpG sites, the CpG and CpNpG sites located in a region selected from:
(i) fragile X-related epigenetic element 2 (A) [FREE2 (A)] comprising the nucleotide sequence set forth in SEQ ID NO:1 or a homolog thereof or portion or part thereof defined by having at least 80% nucleotide sequence identity to SEQ ID NO:1 or which hybridizes to SEQ ID NO:1 or its complementary form under medium stringency conditions;
(ii) fragile X-related epigenetic element 2 (B) [FREE2 (B)] comprising the nucleotide sequence set forth in SEQ ID NO:2 or a homolog thereof or portion or part thereof defined by having at least 80% nucleotide sequence identity to SEQ ID NO:2 or which hybridizes to SEQ ID NO:2 or its complementary form under medium stringency conditions;
(iii) fragile X-related epigenetic element 2 (C) [FREE2 (C)] comprising the nucleotide sequence set forth in SEQ ID NO:3 or a homolog thereof or portion or part thereof defined by having at least 80% nucleotide sequence identity to SEQ ID NO:3 or which hybridizes to SEQ ID NO:3 or its complementary form under medium stringency conditions;
(iv) fragile X-related epigenetic element 2 (D) [FREE2 (D)] comprising the nucleotide sequence set forth in SEQ ID NO:4 or a homolog thereof or portion or part thereof defined by having at least 80% nucleotide sequence identity to SEQ ID NO:4 or which hybridizes to SEQ ID NO:4 or its complementary form under medium stringency conditions;
(v) fragile X-related epigenetic element 2 (E) [FREE2 (E)] comprising the nucleotide sequence set forth in SEQ ID NO:5 or a homolog thereof or portion or part thereof defined by having at least 80% nucleotide sequence identity to SEQ ID NO:5 or which hybridizes to SEQ ID NO:5 or its complementary form under medium stringency conditions; and
(vi) fragile X-related epigenetic element 3 (FREE3) comprising the nucleotide sequence set forth in SEQ ID NO:6 or a homolog thereof or portion or part thereof defined by having at least 80% nucleotide sequence identity to SEQ ID NO:6 or which hybridizes to SEQ ID NO:6 or its complementary form under medium stringency conditions; and
(b) determining a difference in the extent of methylation in the selected region of isolated genomic DNA from the human subject relative to the extent of methylation in a control genomic DNA sample from a control subject with (CGG)ₙ, wherein n is < 40 thereby determining that the human subject has a pathological condition associated with pre-mutation or full mutation of FMR1 or a propensity to develop the same.

## Patentansprüche

1. *In vitro*-Verfahren zum Quantifizieren des Ausmaßes der Methylierung einer Zielstelle innerhalb einer DNA-Probe, wobei das Verfahren umfasst:
(i) Unterwerfen einer DNA-Probe einer Bisulfitbehandlung, um nicht methylierte Cytosin-Nukleotide in der DNA in Uracil-Nukleotide umzuwandeln,
(ii) Amplifikation und Schmelzen in Echtzeit eines Teils der DNA-Probe, die die Zielstelle umfasst, mit ausgewählten Forwärts- und Rückwärtsprimern, wobei eine Doppelstranginterkalierende Markierung eingebaut wird, die ein identifizierbares Signal in doppelsträngiger DNA bereitstellt, wobei die Amplifikation und das Schmelzen zusammen mit einem ersten Kontrollprobensatz einer bekannten DNA-Konzentration und einem zweiten Kontroll-DNA-Probensatz eines bekannten Prozentanteils an methylierter und nicht-methylierter DNA erfolgt,
(iii) Erzeugen zweier Standardkurven: (a) einer ersten Standardkurve, die von der Echtzeitamplifikation der ersten Kontrollprobe abgeleitet ist, wobei die erste Standardkurve als DNA-Konzentration gegenüber Zyklusschwellenwert (Ct) ausgedrückt ist, was die Bestimmung des dynamischen linearen Bereichs der Amplifikation und die Bestimmung des stabilen Schmelzbereichs des amplifizierten Produkts der ersten Kontrollprobe, wo die Signalstärke unabhängig von der DNA-Verdünnung ist, ermöglicht, und (b) einer zweiten Standardkurve, die von dem Echtzeitschmelzen der zweiten Kontroll-DNA-Probe abgeleitet ist, wobei die zweite Standardkurve als Signalstärke aufgrund Schmelzfreisetzung gegenüber dem bekannten Prozentanteil der Methylierung aus der zweiten Kontroll-DNA-Probe ausgedrückt ist,
(iv) Bestimmen, ob die Signalstärke aus der Amplifikation und dem Schmelzen der DNA-Probe, die die Zielstelle umfasst, in den dynamischen linearen Bereich der Amplifikation und den stabilen Schmelzbereich des amplifizierten Produkts fällt, indem die in (iii) erzeugten Standardkurven verwendet werden, und
(v) Verwenden der Signalstärke, die in den dynamischen linearen Bereich der Amplifikation und den stabilen Schmelzbereich des amplifizierten Produkts fällt, um den Prozentanteil der Methylierung für die Zielstelle innerhalb der DNA-Probe durch Auslesen des Prozentanteils der Methylierung von dem Grad der Signalstärke unter Verwendung der zweiten Standardkurve, die von der zweiten Kontroll-DNA-Probe abgeleitet ist, zu bestimmen, und gegebenenfalls
(vi) Korrelieren des Ausmaßes der Methylierung mit einem klinischen Phänotyp.

2. Verfahren nach Anspruch 1, wobei die DNA-Probe nach der Bisulfitumwandlung seriell verdünnt wird, wobei das Verfahren gegebenenfalls ferner eine Mittelwertsbildung der abgestimmten Fluoreszenzeinheiten (Aligned Fluorescence Units; AFU) umfasst, um den Prozentanteil der Methylierung zu bestimmen, wenn mehrfache Verdünnungen der gleichen DNA-Probe in den dynamischen linearen Bereich und den stabilen Schmelzbereich des amplifizierten Produkts fallen.

3. Verfahren nach Anspruch 1 oder Anspruch 2, wobei die Amplifikation in Schritt (ii) eine Echtzeit-Polymerase-Kettenreaktion (RT-PCR) ist und/oder das Schmelzen in Schritt (ii) eine Reaktion eines Schmelzens mit hoher Auflösung (HRM) ist, die bei der größten Differenz zwischen den nicht methylierten und methylierten Kontrollproben durchgeführt wird.

4. Verfahren nach Anspruch 3, wobei die Markierung, die in Echtzeit in die amplifizierte DNA eingebaut oder von ihr freigesetzt wird, ein interkalierender Fluoreszenzfarbstoff ist, der in doppelsträngige DNA eingebaut wird und bei einer Signalstärke fluoresziert, die in AFU gemessen wird, und der kein Fluoreszenzsignal in einzelsträngiger DNA bereitstellt.

5. Verfahren nach Anspruch 4, das ferner das Verwenden eines Algorithmus, um zu bestimmen, ob eine DNA-Probe innerhalb des dynamischen linearen Bereichs der Amplifikation liegt und innerhalb des stabilen Bereichs des amplifizierten Produkts liegt, und um eine AFU mit dem Prozentanteil der Methylierung der DNA-Probe zu korrelieren, umfasst.

6. Verfahren nach einem der Ansprüche 1 bis 5, wobei der klinische Phänotyp einer oder mehrere der Phänotypen ist, die ausgewählt sind aus der Gruppe, bestehend aus kognitiver Beeinträchtigung, Gedächtnisverlust, schwacher kognitiver Retention, schwacher kognitiver Funktion mit einem spezifischen Schwerpunkt von schwacher verbaler/exekutiver Funktion, Verhaltensdefizit, Grad oder Beeinträchtigung der neurologischen Entwicklung, Tumorwachstumsentwicklung, Geschlechtsbestimmung, Aneuploidie, Fortschreiten einer Immunantwort, einer Trinukleotidstörung, einer Prägungsstörung, einer modifiziertes-X-Chromosom-Störung, einer X-chromosomalen Störung, X-Chromosom-Inaktivierung, Gewebeabstoßung oder Identifizierung von fötalen Zellen in einer Mutter, Autismus, mentaler Retardierung, Prader-Willi-Syndrom-Syndrom/Angelman-Syndrom, Alzheimer-Krankheit, bipolarer Störung, Diabetes, männlicher sexueller Orientierung, Fettleibigkeit, Schizophrenie, Parkinsonismus, Depression, Aufmerksamkeitsdefizitsyndrom, Stimmungsstörung und einem Krebs ausgewählt aus Blasen-, Brust-, Zervix-, kolorektalem, Speiseröhren-, hepatozellulärem, Lungen-, Mesotheliom-, Eierstock-, Prostata-, Hodenkrebs, Leukämie, Pathoneuroentwicklungs- oder pathoneurodegenerativem Zustand, Huntington-Krankheit (HD), Dentatorubro-Pallidoluysischer Atrophie (DRPLA), Spinobulbär-Muskelatrophie oder Kennedy-Krankheit (SBMA), Spinozerebellärer Ataxie Typ 1 (SCA1), Spinozerebellärer Ataxie Typ 2 (SCA2), Spinozerebellärer Ataxie Typ 3 oder Machado-Joseph-Krankheit (SCA3), Spinozerebellärer Ataxie Typ 6 (SCA6), Spinozerebellärer Ataxie Typ 7 (SCAT), Spinozerebellärer Ataxie Typ 17 (SCA17) und Nicht-PolyQ-Krankheiten wie Fragiles-X-Syndrom (FXS), Fragiles-X-assoziiertem Tremor oder Ataxie (FXTAS), Fragiles-XEmentaler Retardierung (FRAXE), Myotoner Dystrophie (DM), Spinozerebellärer Ataxie (SCAB), Spinozerebellärer Ataxie Typ 12 (SCA12), Fragiles-X-assoziierter primärer Ovarialinsuffizienz (FXPOI), Friedrich-Ataxie (FRDA), Fragiltyp, Folsäuretyp, selten 12 (FRA12A), Autismus (einschließlich komorbidem Autismus), mentaler Retardierung (MR), Klinefelter-Syndrom, RNA-Toxizitätskrankheit, Turner-Syndrom, einem modifizierten X-Chromosom, einschließlich Triple-X-Syndrom und Jacob-Syndrom, und kognitiver Beeinträchtigung.

7. Verfahren nach einem der Ansprüche 1 bis 6, wobei die DNA-Zielstelle ausgewählt ist aus einer Stelle innerhalb eines genetischen Locus, eines Introns, eines Exons, einer Intron-Exon-Grenze, einer Promotorregion oder einer Region 5' der Promotorregion, einer CpG-Insel oder Inselküsten oder Gruppe von CpG-Inseln oder Inselküsten, einer Stelle stromabwärts der 3'-Endregion eines genetischen Locus oder innerhalb einer genomischen Region, ausgewählt aus der Gruppe, bestehend aus dem FMR1-Genlocus, FREE2 (A) mit der in SEQ ID NO:1 angegebenen Nukleotidsequenz oder einer Nukleotidsequenz mit mindestens 80% Identität zu SEQ ID NO:1 oder eine Nukleotidsequenz, die unter Bedingungen mittlerer Stringenz mit dem Komplement von SEQ ID NO:1 hybridisiert, FREE2 (B), umfassend die in SEQ ID NO:2 angegebene Nukleotidsequenz oder ein Homolog davon oder einen Teil oder Anteil davon, definiert durch mindestens 80% Nukleotidsequenz-Identität zu SEQ ID NO:2 oder das mit SEQ ID NO:2 oder ihrer komplementären Form unter Bedingungen mittlerer Stringenz hybridisiert, FREE2 (C), umfassend die in SEQ ID NO:3 angegebene Nukleotidsequenz oder ein Homolog davon oder einen Teil oder Anteil davon, definiert durch mindestens 80% Nukleotidsequenz-Identität zu SEQ ID NO:3 oder das mit SEQ ID NO:3 oder ihrer komplementären Form unter Bedingungen mittlerer Stringenz hybridisiert, FREE2 (D), umfassend die in SEQ ID NO:4 angegebene Nukleotidsequenz oder ein Homolog davon oder einen Teil oder Anteil davon, definiert durch mindestens 80% Nukleotidsequenz-Identität zu SEQ ID NO:4 oder das mit SEQ ID NO:4 oder ihrer komplementären Form unter Bedingungen mittlerer Stringenz hybridisiert, FREE2 (E), umfassend die in SEQ ID NO:5 angegebene Nukleotidsequenz oder ein Homolog davon oder einen Teil oder Anteil davon, definiert durch mindestens 80% Nukleotidsequenz-Identität zu SEQ ID NO:5 oder das mit SEQ ID NO:5 oder ihrer komplementären Form unter Bedingungen mittlerer Stringenz hybridisiert, FREE3 mit der in SEQ ID NO:6 angegebenen Nukleotidsequenz oder einer Nukleotidsequenz mit mindestens 80% Identität zu SEQ ID NO:6 oder einer Nukleotidsequenz, die unter Bedingungen mittlerer Stringenz mit dem Komplement von SEQ ID NO:6 hybridisiert, dem SNRPN-Genlocus, dem SNRPN-Genlocus, umfassend die in SEQ ID NO:19 angegebene Nukleotidsequenz oder ein Homolog davon oder einen Teil oder Anteil davon, definiert durch mindestens 80% Nukleotidsequenz-Identität zu SEQ ID NO:19 oder das mit SEQ ID NO:19 oder ihrer komplementären Form unter Bedingungen mittlerer Stringenz hybridisiert, einer in SEQ ID NO:20 angegebenen Nukleotidsequenz oder einem Homolog davon oder einem Teil oder Anteil davon, definiert durch mindestens 80% Nukleotidsequenz-Identität zu SEQ ID NO:20 oder das mit SEQ ID NO:20 oder ihrer komplementären Form unter Bedingungen mittlerer Stringenz hybridisiert.

8. Verfahren nach Anspruch 7, wobei eine Veränderung des Ausmaßes der Methylierung innerhalb einer genomischen Region, ausgewählt aus der Gruppe, bestehend aus dem SNRPN-Genlocus, dem SNRPN-Genlocus, umfassend die in SEQ ID NO:19 angegebene Nukleotidsequenz oder ein Homolog oder einen Teil oder Anteil davon, definiert durch mindestens 80% Nukleotidsequenz-Identität zu SEQ ID NO:19 oder das mit SEQ ID NO:19 oder ihrer komplementären Form unter Bedingungen mittlerer Stringenz hybridisiert, einer in SEQ ID NO:20 angegebenen Nukleotidsequenz oder einem Homolog davon oder einem Teil oder Anteil davon, definiert durch mindestens 80% Nukleotidsequenz-Identität zu SEQ ID NO:20 oder das mit SEQ ID NO:20 oder ihrer komplementären Form unter Bedingungen mittlerer Stringenz hybridisiert, mit Prader-Willi-Syndrom (PWS)/Angelman-Syndrom (AS) assoziiert ist.

9. Verfahren nach einem der Ansprüche 1 bis 8, wobei die DNA-Probe von Zellen stammt, die in Körperflüssigkeit oder einer getrockneten Körperflüssigkeitsprobe, Sputum oder einer anderen Atmungsflüssigkeit, Blut oder einer Fraktion oder getrockneten Form davon, Gewebeflüssigkeit, einer Gewebeprobe, Lymphflüssigkeit, Samen, Urin, Fäkalienmaterial oder Hautmaterial von einem Menschen enthalten sind.

10. Verfahren nach einem der Ansprüche 1 bis 9 bei einer Verwendung für einen Nachweis des Vorliegens eines pathologischen Zustands, der mit Methylierung innerhalb des SNRPN-Locus assoziiert ist, oder einer Neigung, denselben zu entwickeln, in einem Menschen, wobei:
(a) die Amplifikationsreaktion in Teil (ii) Primer verwendet, die für eine Region des SNRPN-Genlocus selektiv sind, die CpG- und/oder CpNpG-Stellen umfasst, wobei die CpG- und CpNpG-Stellen in einer Region liegen, die ausgewählt ist aus:
(i) dem SNRPN-Genlocus (SNRPN-M), umfassend die in SEQ ID NO:19 angegebene Nukleotidsequenz oder ein Homolog davon oder einen Teil oder Anteil davon, definiert durch mindestens 80% Nukleotidsequenz-Identität zu SEQ ID NO:19 oder das mit SEQ ID NO:19 oder ihrer komplementären Form unter Bedingungen mittlerer Stringenz hybridisiert, und
(ii) dem SNRPN-Genlocus (SNRPN-P), umfassend die in SEQ ID NO:20 angegebene Nukleotidsequenz oder ein Homolog davon oder einen Teil oder Anteil davon, definiert durch mindestens 80% Nukleotidsequenz-Identität zu SEQ ID NO:20 oder das mit SEQ ID NO:20 oder ihrer komplementären Form unter Bedingungen mittlerer Stringenz hybridisiert.

11. Verfahren nach Anspruch 10, wobei der pathologische Zustand PLUS/AS ist.

12. Verfahren nach einem der Ansprüche 1 bis 9 bei einer Verwendung für einen Nachweis des Vorliegens eines pathologischen Zustands, der mit dem Vorliegen einer Prämutation oder einer vollständigen Mutation des Fragiles-X-Mentalretardation (FMR)-Genlocus (FMR1) assoziiert ist, oder einer Neigung, denselben zu entwickeln, in einem Menschen, wobei:
(a) die Amplifikationsreaktion in Teil (ii) Primer verwendet, die für eine Region des FMR1-Genlocus selektiv sind, die CpG- und/oder CpNpG-Stellen umfasst, wobei die CpG- und CpNpG-Stellen in einer Region liegen, die ausgewählt ist aus:
(i) Fragiles-X-verwandtes epigenetisches Element 2 (A) [FREE2 (A)], umfassend die in SEQ ID NO:1 angegebene Nukleotidsequenz oder ein Homolog davon oder einen Teil oder Anteil davon, definiert durch mindestens 80% Nukleotidsequenz-Identität zu SEQ ID NO:1 oder das mit SEQ ID NO:1 oder ihrer komplementären Form unter Bedingungen mittlerer Stringenz hybridisiert,
(ii) Fragiles-X-verwandtes epigenetisches Element 2 (B) [FREE2 (B)], umfassend die in SEQ ID NO:2 angegebene Nukleotidsequenz oder ein Homolog davon oder einen Teil oder Anteil davon, definiert durch mindestens 80% Nukleotidsequenz-Identität zu SEQ ID NO:2 oder das mit SEQ ID NO:2 oder ihrer komplementären Form unter Bedingungen mittlerer Stringenz hybridisiert,
(iii) Fragiles-X-verwandtes epigenetisches Element 2 (C) [FREE2 (C)], umfassend die in SEQ ID NO:3 angegebene Nukleotidsequenz oder ein Homolog davon oder einen Teil oder Anteil davon, definiert durch mindestens 80% Nukleotidsequenz-Identität zu SEQ ID NO:3 oder das mit SEQ ID NO:3 oder ihrer komplementären Form unter Bedingungen mittlerer Stringenz hybridisiert,
(iv) Fragiles-X-verwandtes epigenetisches Element 2 (D) [FREE2 (D)], umfassend die in SEQ ID NO:4 angegebene Nukleotidsequenz oder ein Homolog davon oder einen Teil oder Anteil davon, definiert durch mindestens 80% Nukleotidsequenz-Identität zu SEQ ID NO:4 oder das mit SEQ ID NO:4 oder ihrer komplementären Form unter Bedingungen mittlerer Stringenz hybridisiert,
(v) Fragiles-X-verwandtes epigenetisches Element 2 (E) [FREE2 (E)], umfassend die in SEQ ID NO:5 angegebene Nukleotidsequenz oder ein Homolog davon oder einen Teil oder Anteil davon, definiert durch mindestens 80% Nukleotidsequenz-Identität zu SEQ ID NO:5 oder das mit SEQ ID NO:5 oder ihrer komplementären Form unter Bedingungen mittlerer Stringenz hybridisiert, und
(vi) Fragiles-X-verwandtes epigenetisches Element 3 (FREE3), umfassend die in SEQ ID NO:6 angegebene Nukleotidsequenz oder ein Homolog davon oder einen Teil oder Anteil davon, definiert durch mindestens 80% Nukleotidsequenz-Identität zu SEQ ID NO:6 oder das mit SEQ ID NO:6 oder ihrer komplementären Form unter Bedingungen mittlerer Stringenz hybridisiert, und
(b) Bestimmen eines Unterschieds im Ausmaß der Methylierung in der ausgewählten Region der isolierten genomischen DNA des Menschen im Verhältnis zu dem Ausmaß der Methylierung in einer genomischen DNA-Kontrollprobe von einem Kontrolllebewesen mit (CGG)ₙ, wobei n < 40 ist, dadurch Bestimmen, dass der Mensch einen pathologischen Zustand, der mit einer Prämutation oder einer vollständigen Mutation von FMR1 assoziiert ist, oder eine Neigung, denselben zu entwickeln, aufweist.

## Revendications

1. Procédé in vitro pour quantifier le degré de méthylation d'un site cible dans un échantillon d'ADN, le procédé comprenant les étapes consistant à :
(i) soumettre un échantillon d'ADN à un traitement au bisulfite pour convertir les nucléotides à cytosine non méthylée dans l'ADN en nucléotides à uracile ;
(ii) amplifier et faire fondre en temps réel une partie de l'échantillon d'ADN comprenant le site cible avec des amorces directe et inverse choisies incorporant un marqueur intercalant en double brin qui produit un signal identifiable dans de l'ADN double brin, l'amplification et la fusion s'effectuant conjointement avec une première série d'échantillons témoins à concentration d'ADN connue et une seconde série d'échantillons d'ADN témoins à pourcentage connu d'ADN méthylé et d'ADN non méthylé ;
(iii) engendrer deux courbes d'étalonnage : (a) une première courbe d'étalonnage tirée de l'amplification en temps réel du premier échantillon témoin, la première courbe d'étalonnage étant exprimée en tant que concentration d'ADN en fonction du seuil de cycle (Ct) permettant la détermination de la plage dynamique linéaire d'amplification et la détermination de la plage de fusion du produit amplifié stable du premier échantillon témoin où la force de signal est indépendante de la dilution de l'ADN ; et (b) une seconde courbe d'étalonnage tirée de la fusion en temps réel du second échantillon d'ADN témoin, la seconde courbe d'étalonnage étant exprimée en tant que force de signal due au déclenchement de la fusion en fonction du pourcentage connu de méthylation provenant du second échantillon d'ADN témoin ;
(iv) déterminer si la force de signal provenant de l'amplification et de la fusion de l'échantillon d'ADN comprenant le site cible rentre dans la plage dynamique linéaire d'amplification et la plage de fusion du produit amplifié stable en utilisant les courbes d'étalonnage engendrées en (iii) ; et
(v) utiliser la force de signal rentrant dans la plage dynamique linéaire d'amplification et la plage de fusion du produit amplifié stable pour déterminer le pourcentage de méthylation pour le site cible dans l'échantillon d'ADN par lecture du pourcentage de méthylation à partir du degré de force de signal en utilisant la seconde courbe d'étalonnage tirée du second échantillon d'ADN témoin ; et en option
(vi) corréler le degré de méthylation avec un phénotype clinique.

2. Procédé selon la revendication 1, dans lequel l'échantillon d'ADN est dilué en série après conversion par le bisulfite, le procédé comprenant en option encore le fait de faire la moyenne des unités de fluorescence alignée (AFU) pour déterminer le pourcentage de méthylation si de multiple dilutions du même échantillon d'ADN rentrent dans la plage dynamique linéaire et la plage de fusion du produit amplifié stable.

3. Procédé selon la revendication 1 ou la revendication 2, dans lequel l'amplification dans l'étape (ii) est une réaction d'amplification en chaîne par polymérase en temps réel (RT-PCR) et/ou la fusion dans l'étape (ii) est une réaction de fusion à haute résolution (HRM) conduite à la plus grande différence entre les échantillons témoins méthylés et les échantillons témoins non méthylés

4. Procédé selon la revendication 3, dans lequel le marqueur incorporé dans ou libéré de l'ADN amplifié en temps réel est un colorant fluorescent intercalant qui s'incorpore dans de l'ADN double brin et émet une fluorescence à une force de signal mesurant en AFU et qui ne produit pas un signal de fluorescence dans de l'ADN simple brin.

5. Procédé selon la revendication 4, comprenant encore le fait d'utiliser un algorithme pour déterminer si un échantillon d'ADN rentre dans la plage dynamique linéaire d'amplification et rentre dans la plage de produit amplifié stable et pour corréler une AFU avec un pourcentage de méthylation du l'échantillon d'ADN.

6. Procédé selon l'une quelconque des revendications 1 à 5, dans lequel le phénotype clinique est un quelconque ou plusieurs des phénotypes choisis dans l'ensemble constitué par un déficit cognitif, une perte de mémoire, une rétention cognitive réduite, une fonction cognitive réduite avec un accent particulier sur une fonction verbale/exécutive réduite, un déficit comportemental, un degré d'altération du développement neurologique, un développement de croissance tumorale, un déterminisme sexuel, une aneuploïdie, une accentuation de réponse immunitaire, un trouble dû à des répétitions trinucléotidiques, un trouble d'imprégnation, un trouble dû à un chromosome X modifié, un trouble lié à X, une inactivation du chromosome X, un rejet de tissu ou une identification de cellules foetales chez un sujet maternel, un autisme, un retard mental, un syndrome de Prader-Willi/syndrome d'Angelman, une maladie d'Alzheimer, un trouble bipolaire, un diabète, une orientation sexuelle masculine, une obésité, une schizophrénie, une maladie de Parkinson, une dépression, un trouble du déficit de l'attention, un trouble de l'humeur et un cancer choisi parmi un cancer de la vessie, du sein, du col utérin, colorectal, de l'oesophage, hépatocellulaire, du poumon, un mésothéliome, un cancer de l'ovaire, de la prostate, du testicule, une leucémie, un état pathologique neurodéveloppemental ou neurodégénératif, une maladie de Huntington (MH), une atrophie dentatorubropallidoluysienne (ADRPL), une atrophie musculaire spinale et bulbaire ou maladie de Kennedy (SBMA), une ataxie spinocérébelleuse de type 1 (SCA1), une ataxie spinocérébelleuse de type 2 (SCA2), une ataxie spinocérébelleuse de type 3 ou maladie de Machado-Joseph (SCA3), une ataxie spinocérébelleuse de type 6 (SCA6), une ataxie spinocérébelleuse de type 7 (SCAT), une ataxie spinocérébelleuse de type 17 (SCA17) et des maladies non-polyQ telles qu'un syndrome de l'X fragile (FXS), une ataxie ou un tremblement lié(e) à l'X fragile (FXTAS), un retard mental lié à XE fragile (FRAXE), une dystrophie myotonique (DM), une ataxie spinocérébelleuse (SCAB), une ataxie spinocérébelleuse de type 12 (SCA12), une insuffisance ovarienne primaire associée à l'X fragile (FXPOI), une ataxie de Friedrich (FRDA), type fragile, type acide folique, type 12 rare (FRA12A), un autisme (incluant un autisme comorbide, un retard mental (RM), un syndrome de Klinefelter, une maladie due à une toxicité d'ARN, un syndrome de Turner, un syndrome dû à un chromosome X modifié, incluant un syndrome du triple X et un syndrome de Jacob et un déficit cognitif.

7. Procédé selon l'une quelconque des revendications 1 à 6, dans lequel le site cible d'ADN est choisi parmi un site à l'intérieur d'un locus génétique, d'un intron, d'un exon, d'une frontière intron-exon, d'une région de promoteur ou d'une région 5' de la région de promoteur, d'un îlot ou de bordures d'îlot CpG ou d'un groupe d'îlots ou de bordures d'îlots CpG, un site en aval de la région terminale 3' d'un locus génétique ou au sein d'une région génomique choisie dans l'ensemble constitué par le locus génétique FMR1, FREE2 (A) ayant la séquence de nucléotides présentée dans la Séquence N° 1 ou une séquence de nucléotides ayant un degré d'identité d'au maximum 80 % avec la Séquence N° 1 ou une séquence de nucléotides qui s'hybride dans des conditions de moyenne stringence avec le complément de la Séquence N° 1 ; FREE2 (B) comprenant la séquence de nucléotides présentée dans la Séquence N° 2 ou un homologue de celle-ci ou un segment ou une partie de celle-ci défini(e) par le fait d'avoir un degré d'identité de séquence de nucléotides d'au moins 80 % avec la Séquence N° 2 ou qui s'hybride dans des conditions de moyenne stringence avec la Séquence N° 2 ou sa forme complémentaire ; FREE2 (C) comprenant la séquence de nucléotides présentée dans la Séquence N° 3 ou un homologue de celle-ci ou un segment ou une partie de celle-ci défini(e) par le fait d'avoir un degré d'identité de séquence de nucléotides d'au moins 80 % avec la Séquence N° 3 ou qui s'hybride dans des conditions de moyenne stringence avec la Séquence N° 3 ou sa forme complémentaire ; FREE2 (D) comprenant la séquence de nucléotides présentée dans la Séquence N° 4 ou un homologue de celle-ci ou un segment ou une partie de celle-ci défini(e) par le fait d'avoir un degré d'identité de séquence de nucléotides d'au moins 80 % avec la Séquence N° 4 ou qui s'hybride dans des conditions de moyenne stringence avec la Séquence N° 4 ou sa forme complémentaire ; FREE2 (E) comprenant la séquence de nucléotides présentée dans la Séquence N° 5 ou un homologue de celle-ci ou un segment ou une partie de celle-ci défini(e) par le fait d'avoir un degré d'identité de séquence de nucléotides d'au moins 80 % avec la Séquence N° 5 ou qui s'hybride dans des conditions de moyenne stringence avec la Séquence N° 5 ou sa forme complémentaire ; FREE3 ayant la séquence de nucléotides présentée dans la Séquence N° 6 ou une séquence de nucléotides ayant un degré d'identité d'au moins 80 % avec la Séquence N° 6 ou une séquence de nucléotides qui s'hybride dans des conditions de moyenne stringence avec le complément de la Séquence N° 6, le locus génétique SNRPN, le locus génétique SNRPN comprenant la séquence de nucléotides présentée dans la Séquence N° 19 ou un homologue ou un segment ou une partie de celle-ci défini(e) par le fait d'avoir un degré d'identité de nucléotides d'au moins 80 % avec la Séquence N° 19 ou qui s'hybride dans des conditions de moyenne stringence avec la Séquence N° 19 ou sa forme complémentaire ; une séquence de nucléotides présentée dans la Séquence N° 20 ou un homologue de celle-ci ou un segment ou une partie de celle-ci défini(e) par le fait d'avoir un degré d'identité de séquence de nucléotides d'au moins 80 % avec la Séquence N° 20 ou qui s'hybride dans des conditions de moyenne stringence avec la Séquence N° 20 ou sa forme complémentaire.

8. Procédé selon la revendication 7, dans lequel un changement du degré de méthylation au sein d'une région génomique choisie dans l'ensemble constitué par le locus génétique SNRPN, le locus génétique SNRPN comprenant la séquence de nucléotides présentée dans la Séquence N° 19 ou un homologue ou un segment ou une partie de celle-ci défini(e) par le fait d'avoir un degré d'identité de nucléotides d'au moins 80 % avec la Séquence N° 19 ou qui s'hybride dans des conditions de moyenne stringence avec la Séquence N° 19 ou sa forme complémentaire ; une séquence de nucléotides présentée dans la Séquence N° 20 ou un homologue de celle-ci ou un segment ou une partie de celle-ci défini(e) par le fait d'avoir un degré d'identité de séquence de nucléotides d'au moins 80 % avec la Séquence N° 20 ou qui s'hybride dans des conditions de moyenne stringence avec la Séquence N° 20 ou sa forme complémentaire, est lié au syndrome de Prada-Willi (PWS)/syndrome d'Angelman (AS).

9. Procédé selon l'une quelconque des revendications 1 à 8, dans lequel l'échantillon d'ADN est issu de cellules contenues dans un liquide corporel ou un échantillon de liquide corporel séché, un crachat ou un autre liquide respiratoire, du sang ou une fraction ou une forme séchée de celui-ci, un liquide tissulaire, un échantillon de tissu, du liquide lymphatique, du sperme, de l'urine, de la matière fécale ou de la matière cutanée provenant d'un sujet humain.

10. Procédé selon l'une quelconque des revendications 1 à 9, lorsqu'il est utilisé pour détecter la présence d'un état pathologique associé à la méthylation à l'intérieur du locus SNRPN ou d'une prédisposition à développer un tel état chez un sujet humain, dans lequel :
(a) la réaction d'amplification dans la partie (ii) utilise des amorces sélectives d'une région du locus génétique SNRPN comprenant des sites CpG et/ou CpNpG, les sites CpG et CpNpG étant localisés dans une région choisie parmi :
(i) un locus génétique SNRPN (SNRPN-M) comprenant la séquence de nucléotides présentée dans la Séquence N° 19 ou un homologue de celle-ci ou un segment ou une partie de celle-ci défini(e) par le fait d'avoir un degré d'identité de séquence de nucléotides d'au moins 80 % avec la Séquence N° 19 ou qui s'hybride dans des conditions de moyenne stringence avec la Séquence N° 19 ou sa forme complémentaire ;
(ii) un locus génétique SNRPN (SNRPN-P) comprenant la séquence de nucléotides présentée dans la Séquence N° 20 ou un homologue de celle-ci ou un segment ou une partie de celle-ci défini(e) par le fait d'avoir un degré d'identité de séquence de nucléotides d'au moins 80 % avec la Séquence N° 20 ou qui s'hybride dans des conditions de moyenne stringence avec la Séquence N° 20 ou sa forme complémentaire.

11. Procédé selon la revendication 10, dans lequel l'état pathologique est PLUS/AS.

12. Procédé selon l'une quelconque des revendications 1 à 9, lorsqu'il est utilisé pour détecter la présence d'un état pathologique associé à la présence d'une pré-mutation ou d'une mutation complète du locus génétique (FMR1) de retard mental lié à X fragile (FMR) ou d'une prédisposition à développer un tel état pathologique chez un sujet humain, dans lequel :
(a) la réaction d'amplification dans la partie (ii) utilise des amorces sélectives d'une région du locus génétique FMR1 comprenant des sites CpG et/ou CpNpG, les sites CpG et CpNpG étant localisés dans une région choisie parmi :
(i) un élément épigénétique 2 (A) en relation avec X fragile [FREE2 (A)] comprenant la séquence de nucléotides présentée dans la Séquence N° 1 ou un homologue de celle-ci ou un segment ou une partie de celle-ci défini(e) par le fait d'avoir un degré d'identité de séquence de nucléotides d'au moins 80 % avec la Séquence N° 1 ou qui s'hybride dans des conditions de moyenne stringence avec la Séquence N° 1 ou sa forme complémentaire ;
(ii) un élément épigénétique 2 (B) en relation avec X fragile [FREE2 (B)] comprenant la séquence de nucléotides présentée dans la Séquence N° 2 ou un homologue de celle-ci ou un segment ou une partie de celle-ci défini(e) par le fait d'avoir un degré d'identité de séquence de nucléotides d'au moins 80 % avec la Séquence N° 2 ou qui s'hybride dans des conditions de moyenne stringence avec la Séquence N° 2 ou sa forme complémentaire ;
(iii) un élément épigénétique 2 (C) en relation avec X fragile [FREE2 (C)] comprenant la séquence de nucléotides présentée dans la Séquence N° 3 ou un homologue de celle-ci ou un segment ou une partie de celle-ci défini(e) par le fait d'avoir un degré d'identité de séquence de nucléotides d'au moins 80 % avec la Séquence N° 3 ou qui s'hybride dans des conditions de moyenne stringence avec la Séquence N° 3 ou sa forme complémentaire ;
(iv) un élément épigénétique 2 (D) en relation avec X fragile [FREE2 (D)] comprenant la séquence de nucléotides présentée dans la Séquence N° 4 ou un homologue de celle-ci ou un segment ou une partie de celle-ci défini(e) par le fait d'avoir un degré d'identité de séquence de nucléotides d'au moins 80 % avec la Séquence N° 4 ou qui s'hybride dans des conditions de moyenne stringence avec la Séquence N° 4 ou sa forme complémentaire ;
(v) un élément épigénétique 2 (E) en relation avec X fragile [FREE2 (E)] comprenant la séquence de nucléotides présentée dans la Séquence N° 5 ou un homologue de celle-ci ou un segment ou une partie de celle-ci défini(e) par le fait d'avoir un degré d'identité de séquence de nucléotides d'au moins 80 % avec la Séquence N° 5 ou qui s'hybride dans des conditions de moyenne stringence avec la Séquence N° 5 ou sa forme complémentaire ;
(vi) un élément épigénétique 3 en relation avec X fragile (FREE3) comprenant la séquence de nucléotides présentée dans la Séquence N° 6 ou un homologue de celle-ci ou un segment ou une partie de celle-ci défini(e) par le fait d'avoir un degré d'identité de séquence de nucléotides d'au moins 80 % avec la Séquence N° 6 ou qui s'hybride dans des conditions de moyenne stringence avec la Séquence N° 6 ou sa forme complémentaire ; et
(b) on détermine une différence du degré de méthylation dans la région choisie d'ADN génomique isolé provenant du sujet humain par rapport au degré de méthylation dans un échantillon d'ADN génomique témoin provenant d'un sujet témoin à (CGG)ₙ, où n est < 40, ce qui permet de déterminer que le sujet humain a un état pathologique associé à une pré-mutation ou une mutation complète de FMR1 ou une prédisposition à développer un tel état.
